(19)

Europäisches
Patentamt
European
Patent Office
Office européen
des brevets

(11) **EP 3 244 912 B1**

(12) **EUROPEAN PATENT SPECIFICATION**

(45) Date of publication and mention
of the grant of the patent:
**18.08.2021 Bulletin 2021/33**

(21) Application number: **16700758.2**

(22) Date of filing: **15.01.2016**

(51) Int Cl.:
**A61K 38/26** (2006.01)   **A61P 3/10** (2006.01)

(86) International application number:
**PCT/EP2016/050804**

(87) International publication number:
**WO 2016/113404 (21.07.2016 Gazette 2016/29)**

(54) **TREATMENT OF PEDIATRIC TYPE 2 DIABETES MELLITUS PATIENTS WITH LIXISENATIDE**

BEHANDLUNG VON PEDIATRISCHEN TYP-2-DIABETES MELLITUS PATIENTEN MIT
LIXISENATIDE

TRAITEMENT AVEC LIXISENATIDE DE PATIENTS PEDIATRIQUES AYANT DIABETES MELLITUS
TYPE 2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.01.2015 EP 15151488**

(43) Date of publication of application:
**22.11.2017 Bulletin 2017/47**

(73) Proprietor: **Sanofi-Aventis Deutschland GmbH
65929 Frankfurt (DE)**

(72) Inventors:
• **BERGMANN, Karin
65926 Frankfurt am Main (DE)**
• **HINCELIN-MÉRY, Agnès
75008 Paris (FR)**
• **JAN, Christelle
75008 Paris (FR)**

(74) Representative: **Weickmann & Weickmann
PartmbB
Postfach 860 820
81635 München (DE)**

(56) References cited:
**WO-A1-2010/138671    WO-A1-2013/060850**

• **SHEHADEH NAIM ET AL: "Can GLP-1
preparations be used in children and adolescents
with diabetes mellitus?", PEDIATRIC
ENDOCRINOLOGY REVIEWS, vol. 11, no. 3,
March 2014 (2014-03), pages 324-327,
XP008176667, ISSN: 1565-4753**
• **RAMAN VANDANA S ET AL: "New potential
adjuncts to treatment of children with type 1
diabetes mellitus", PEDIATRIC RESEARCH, vol.
65, no. 4, 1 March 2010 (2010-03-01), pages
370-374, XP009172354, LIPPINCOTT WILLIAMS &
WILKINS, NEW YORK, US ISSN: 0031-3998, DOI:
10.1203/PDR.0B013E3181975EE4**
• **KAHN R: "Type 2 diabetes in children and
adolescents", DIABETES CARE, vol. 23, no. 3, 1
March 2000 (2000-03-01), pages 381-389,
XP055195532, US ISSN: 0149-5992**
• **Anonymous: "A randomized, double-blind,
placebo controlled trial to assess safety,
tolerability, pharmacokinetics and
pharmacodynamics of lixisenatide in paediatric
(10-17 years old) and adult patients with type 2
diabetes", Sanofi , 13 January 2015 (2015-01-13),
pages 1-12, XP002740957, Retrieved from the
Internet:
URL:http://en.sanofi.com/img/content/study
/PKD11475_summary.pdf [retrieved on
2015-06-16]**

EP 3 244 912 B1

- RACCAH D ET AL: "Meta-analysis of GLP-1 agonist lixisenatide use in patients insufficiently controlled with OADs", DIABETOLOGIA, vol. 56, no. Suppl. 1, September 2013 (2013-09), page S362, XP008176700, & 49TH ANNUAL MEETING OF THE EUROPEAN-ASSOCIATION-FOR-THE-STUDY-OF-DIAB ETES (EASD); BARCELONA, SPAIN; SEPTEMBER 23 -27, 2013
- clinical trial register , XP9510953, Retrieved from the Internet: URL:https://www.clinicaltrialsregister.eu/ ctr-search/trial/2011-004584-67/results [retrieved on 2019]
- MALLOY J ET AL: "Pharmacology and tolerability of a single dose of exenatide in adolescent patients with type 2 diabetes mellitus being treated with metformin: A randomized, placebo-controlled, single-blind, dose-escalation, crossover study", CLINICAL THERAPEUTICS, EXCERPTA MEDICA, PRINCETON, NJ, US, vol. 31, no. 4, 1 April 2009 (2009-04-01), pages 806-815, XP026107570, ISSN: 0149-2918, DOI: 10.1016/J.CLINTHERA.2009.04.005 [retrieved on 2009-05-18]
- AARON S KELLY ET AL: "The Effect of Glucagon-Like Peptide-1 Receptor Agonist Therapy on Body Mass Index in Adolescents With Severe ObesityA Randomized, Placebo-Controlled, Clinical Trial", JAMA PEDIATRICS, AMERICAN MEDICAL ASSOCIATION, USA, vol. 197, no. 4, 1 April 2013 (2013-04-01) , pages 355-360, XP009517730, ISSN: 2168-6203, DOI: 10.1001/JAMAPEDIATRICS.2013.1045 [retrieved on 2013-02-04]

Remarks:
  The file contains technical information submitted after the application was filed and not included in this specification

**Description**

Subject of the present invention is desPro$^{36}$Exendin-4(1-39)-Lys$_6$-NH$_2$ (AVE0010, lixisenatide) or/and a pharmaceutically acceptable salt thereof, for use in a method of treatment of pediatric patients suffering from type 2 diabetes mellitus. Yet another subject of the present invention is a pharmaceutical combination for use in a method of treatment of pediatric patients suffering from type 2 diabetes mellitus, said combination comprising (a) lixisenatide or/and a pharmaceutically acceptable salt thereof, and (b) metformin or/and a pharmaceutically acceptable salt thereof.

Also described herein is a method for treatment of a pediatric patient, said method comprising administering lixisenatide or/and a pharmaceutically acceptable salt thereof, optionally in combination with metformin, to a pediatric patient in need thereof.

In a healthy person the release of insulin by the pancreas is strictly coupled to the concentration of blood glucose. An increased level of blood glucose, as appears after meals, is rapidly counterbalanced by a respective increase in insulin secretion. In fasting condition the plasma insulin level drops to a basal value which is sufficient to ensure the continuous supply of glucose to insulin-sensitive organs and tissues and to keep the hepatic glucose production at a low level at night.

In contrast to type 1 diabetes, there is not generally a lack of insulin in type 2 diabetes mellitus but in many cases, particularly in progressive cases, the treatment with insulin is regarded as the most suitable therapy, if required in combination with orally administered anti-diabetic drugs.

An increased glucose level in the blood over several years without initial symptoms represents a significant health risk. It could clearly be shown by the large-scale DCCT study in the USA (The Diabetes Control and Complications Trial Research Group (1993) N. Engl. J. Med. 329, 977-986) that chronically increased levels of blood glucose are a main reason for the development of diabetes complications. Examples for diabetes complications are micro and macrovascular damages that possibly manifest themselves in retinopathies, nephropathies or neuropathies and lead to blindness, renal failure and the loss of extremities and are accompanied by an increased risk of cardiovascular diseases. It can thus be concluded that an improved therapy of diabetes primarily has to aim keeping blood glucose in the physiological range as closely as possible.

A particular risk exists for overweight patients suffering from type 2 diabetes mellitus, e.g. patients with a body mass index (BMI) $\geq$ 30 kg/m$^2$. In these patients the risks of diabetes overlap with the risks of overweight, leading e.g. to an increase of cardiovascular diseases compared to type 2 diabetes mellitus patients being of a normal weight.

Until recently, type 2 diabetes mellitus (T2DM) was almost exclusively an adult disease. Coinciding with the increasing prevalence of obesity in children, the incidence of T2DM in children and adolescents has markedly increased to the point that it accounts for as many as one third of all the new cases of T2DM diagnosed in adolescent.

Children/adolescents with T2DM are usually diagnosed over the age of 10 years, in middle to late puberty, when due to physiological changes in the GH/IGF-1 axis insulin resistance develops. Like in adults, the incidence of type 2 diabetes in children/adolescents is highest in some ethnic populations (e.g. American Indians, African American, Asian/pacific Islander and Hispanics) (Canadian Diabetes Association Clinical Practice Guidelines Expert Committee, Type 2 Diabetes in Children and Adolescents, 2008 Clinical Practice Guidelines, S162-S167).

Diabetes is a therapeutic area for which the EMA Paediatric Working Party considers that research and development of medicinal products for children should be performed. Type 2 diabetes may have an earlier and more aggressive course in pediatric patients; therefore, they are likely to be at higher risk for developing complications and need the best possible glycemic control in the early stage of their disease.

T2DM in children differs from adults in a number of ways that have an important impact on potential treatment. Puberty appears to play a major role in the development of type 2 diabetes in children. During puberty, there is increased resistance to the action of insulin, resulting in hyperinsulinemia. Growth hormones have been considered as candidates for causing insulin resistance during puberty. The mean growth hormone levels increase transiently during puberty coincidental with the decrease in insulin action. Given this information, it is not surprising that the peak age at presentation of type 2 diabetes in children coincides with the usual age of mid-puberty. In an individual who has a genetic predisposition for insulin resistance, compounded with environmental risk exposure, the additional burden of insulin resistance during puberty may tip the balance from a state of compensated hyperinsulinemia with normal glucose tolerance to inadequate insulin secretion and glucose intolerance that continues beyond puberty (American Diabetes Association, Diabetes Care 2000, 23(3): 381-389).

In the US, about 12% of type 2 pediatric diabetes mellitus patients received metformin monotherapy and about 34 % received insulin monotherapy (Dombrowsky and Barrett, Type II Diabetes Mellitus in Children: Analysis of Prevalence Based on the Pediatric Heath Information System (PHIS) Database, American College of Clinical Pharmacology Annual Meeting September 22nd - 24th 2013, Bethesda, Maryland). In many pediatric type 2 diabetes mellitus patients, progression of the disease is rapid, and control of hyperglycaemia may become insufficient even at maximal tolerated doses of metformin.

However there are no reported studies describing the efficacy of GLP-1 analogs in the pediatric population.

Metformin is a biguanide hypoglycemic agent used in the treatment of non-insulin-dependent diabetes mellitus (type 2 diabetes mellitus) not responding to dietary modification. Metformin improves glycemic control by improving insulin sensitivity and decreasing intestinal absorption of glucose. Metformin is usually administered orally. However, control of type 2 diabetes mellitus in obese patients by metformin may be insufficient. Thus, in these patients, additional measures for controlling type 2 diabetes mellitus may be required.

Metformin is the international nonproprietary name of 1,1-dimethylbiguanide (CAS number 657-24-9).

The compound desPro$^{36}$Exendin-4(1-39)-Lys$_6$-NH$_2$ (AVE0010, lixisenatide) is a derivative of Exendin-4. AVE0010 is disclosed as SEQ ID NO:93 in WO 01/04156:

## SEQ ID NO: 1: lixisenatide (44 amino acids)
H-G-E-G-T-F-T-S-D-L-S-K-Q-M-E-E-E-A-V-R-L-F-I-E-W-L-K-N-G-G-P-S-S-G-
A-P-P-S-K-K-K-K-K-K-NH$_2$

## SEQ ID NO: 2: exendin-4 (39 amino acids)
H-G-E-G-T-F-T-S-D-L-S-K-Q-M-E-E-E-A-V-R-L-F-I-E-W-L-K-N-G-G-P-S-S-G-
A-P-P-P-S-NH$_2$

Exendins are a group of peptides which can lower blood glucose concentration. The Exendin analogue lixisenatide is characterised by C-terminal truncation of the native Exendin-4 sequence. Lixisenatide comprises six C-terminal lysine residues not present in Exendin-4.

Lixisenatide is also termed des-38-proline-exendin-4(*Heloderma suspectum*)-(1-39)-peptidylpenta-L-lysyl-L-lysinamide (CAS number 320367-13-3).

The aim of the present invention can be seen in the improvement of anti-diabetic treatment in children and adolescents suffering from diabetes mellitus, in particular from type 2 diabetes mellitus.

In Examples 1 and 2 of the present invention, after a standardized liquid breakfast in 12 pediatric patients with Type 2 Diabetes mellitus (T2DM) with a mean HbA$_{1c}$ of 8.65% and mean body weight of 84.7 kg, a non-significant decrease in plasma glucose (corrected plasma glucose AUC$_{0:30h-4:30h}$ and plasma glucose AUC$_{0:30h-4:30h}$) was observed with single doses of lixisenatide 5 and 10 $\mu$g compared to placebo. In contrast, single doses of lixisenatide 5 and 10 $\mu$g significantly reduced plasma glucose (corrected plasma glucose AUC$_{0:30h-4:30h}$ and plasma glucose AUC$_{0:30h-4:30h}$) compared to placebo in 12 adult patients with T2DM. Lixisenatide exposure was similar for both dose groups in the evaluable pediatric patients, whereas in adult patients, the lixisenatide exposure dose-proportionally increased. In pediatric patients, the exposure was similar to that in adults for lixisenatide 5 $\mu$g, but lower for lixisenatide 10 $\mu$g. Single doses of lixisenatide 5 and 10 $\mu$g were safe and well tolerated in both, pediatric and adult patients in this study of short duration.

In conclusion, Examples 1 and 2 demonstrated comparable pharmacokinetic (PK) and pharmacodynamic (PD) profiles in pediatric and adults patients at a dose of 5 $\mu$g, as well as no unexpected safety results.

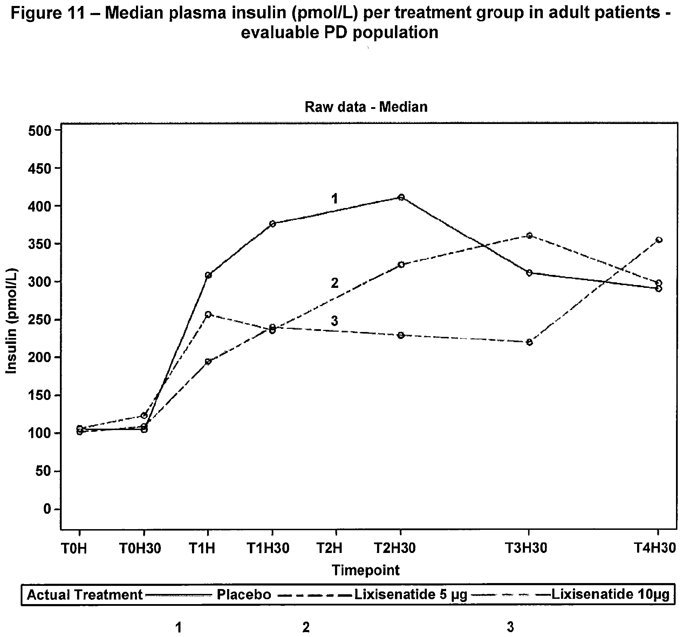

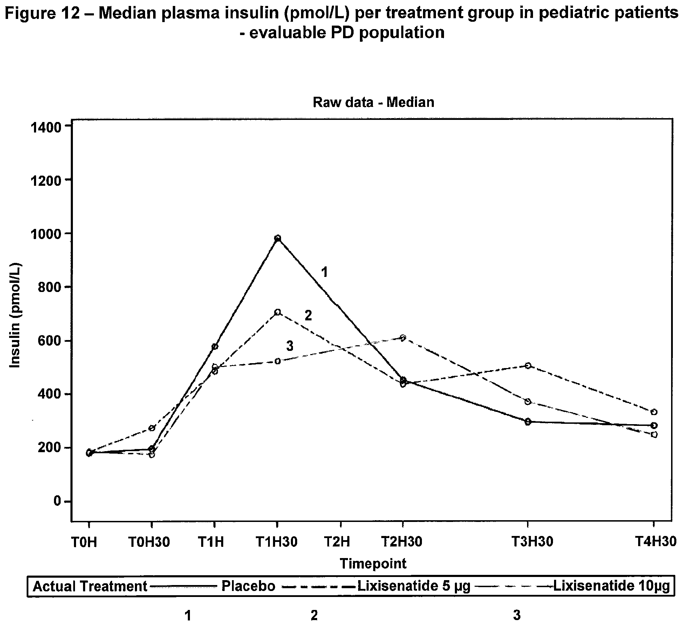

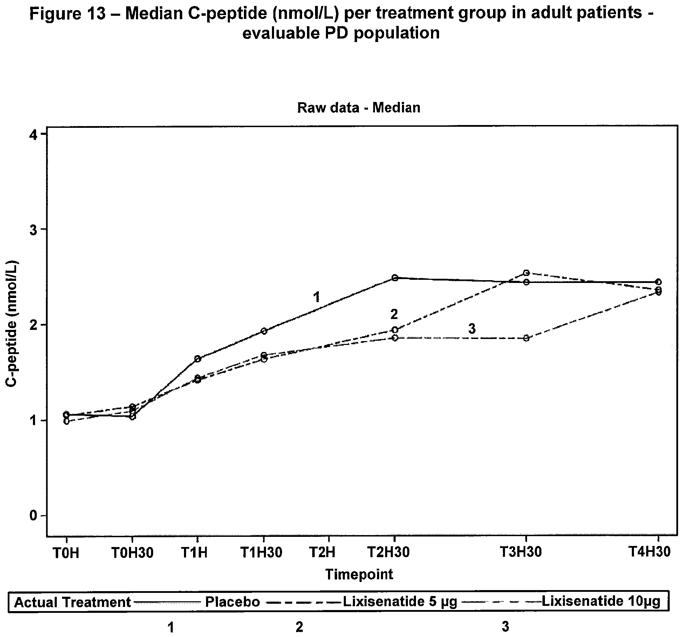

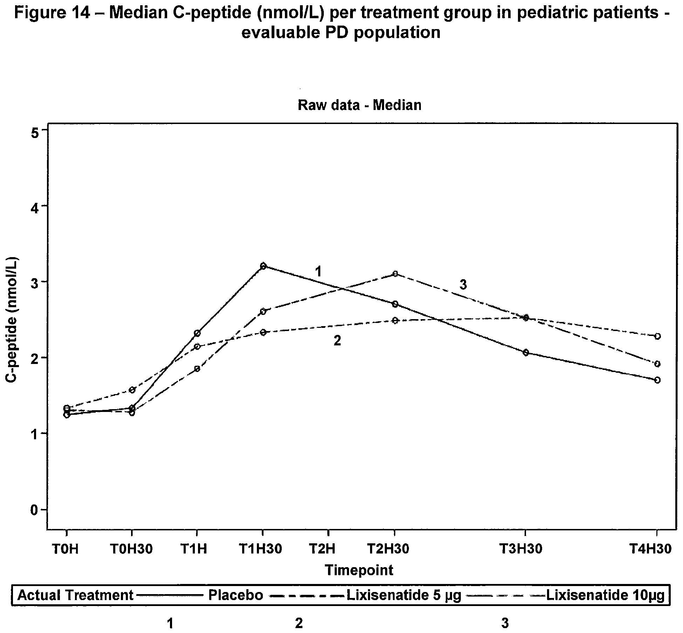

Examples 1 and 2 of the present invention confirm that adult patients have a different pathophysiology compared with children and adolescents. The response to a standardized liquid breakfast differed in pediatric type 2 diabetes mellitus patients from that of adult patients. In the pediatric control population (placebo group), the peak insulin concentration was observed about one hour after the test meal (Figure 12), followed by a rapid decline. In the adult control population (placebo group), the postprandial insulin peak was broader. The peak insulin concentration was observed about 2 hours after the test meal (Figure 11). A similar difference was observed in the postprandial C-peptide concentration (Figures 13 and 14).

In line with these findings, differences were identified in the effects of lixisenatide in the pediatric patients compared with the effects obtained in the adult patient population. The above-described differences in time course of postprandial insulin and C-peptide secretion found in the placebo groups were also observed under lixisenatide.

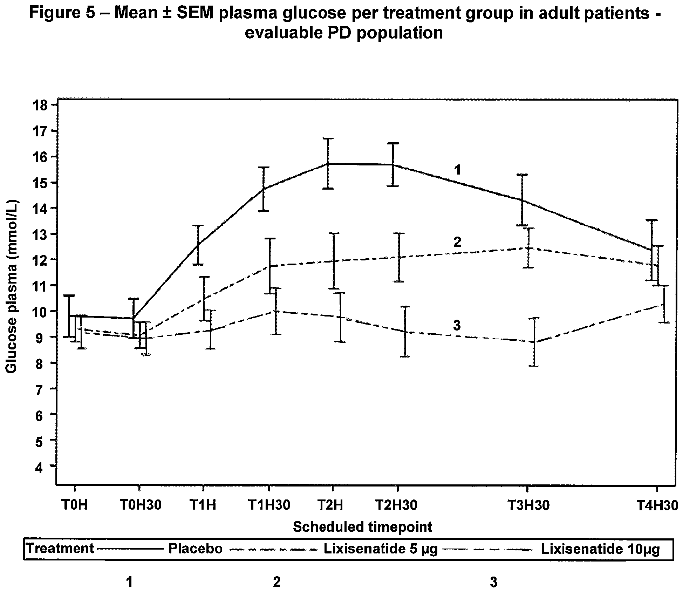

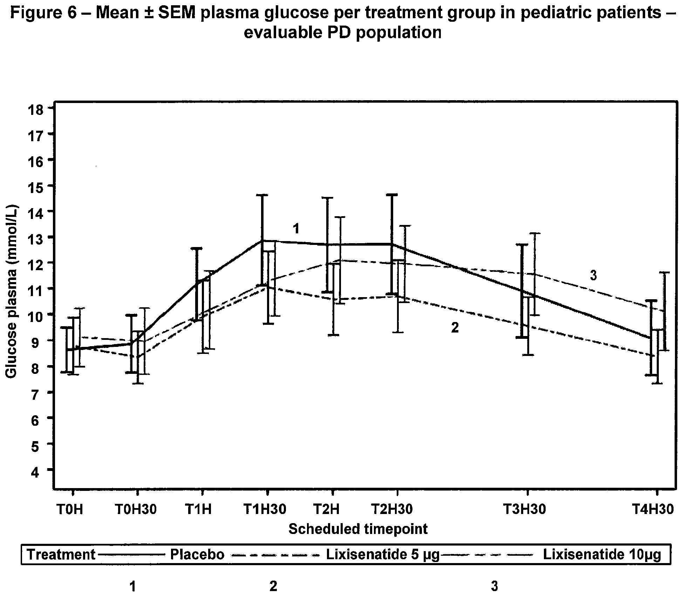

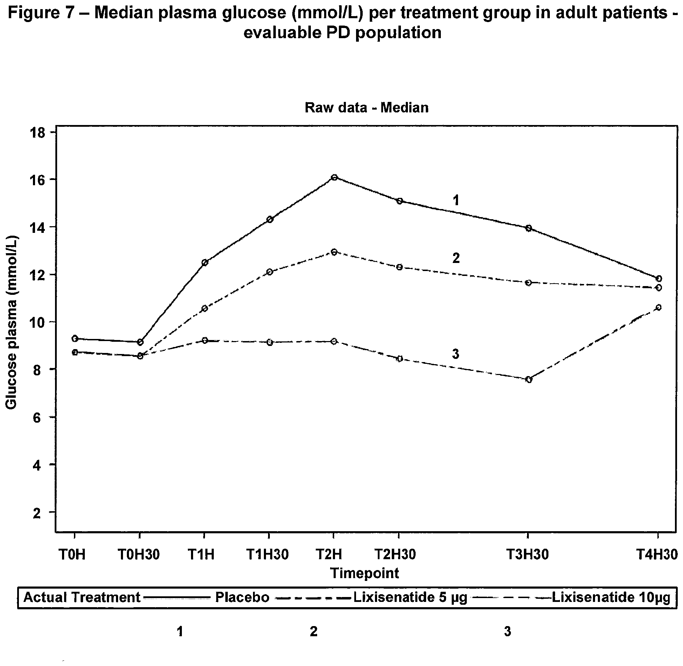

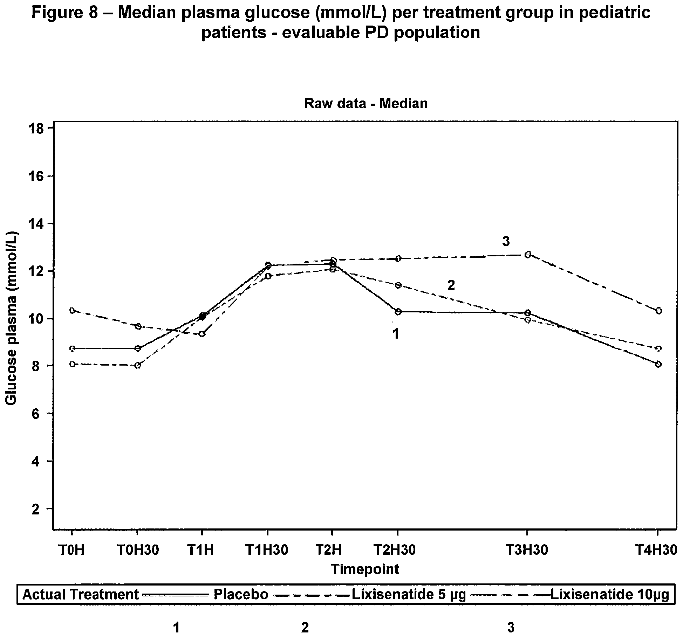

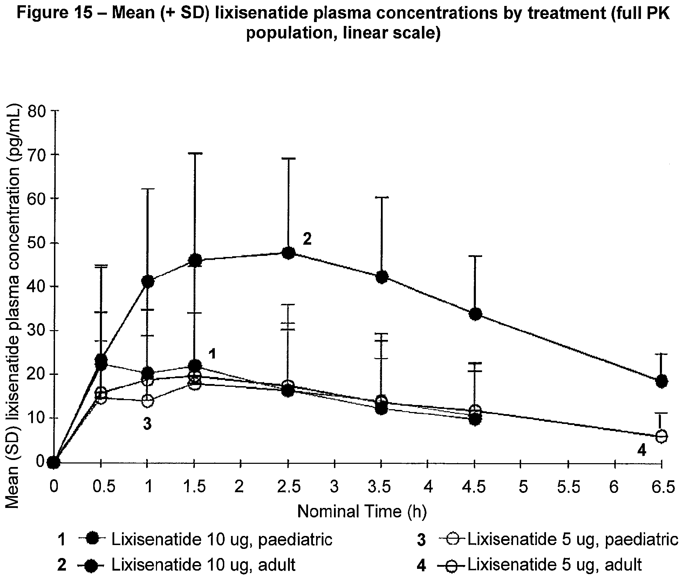

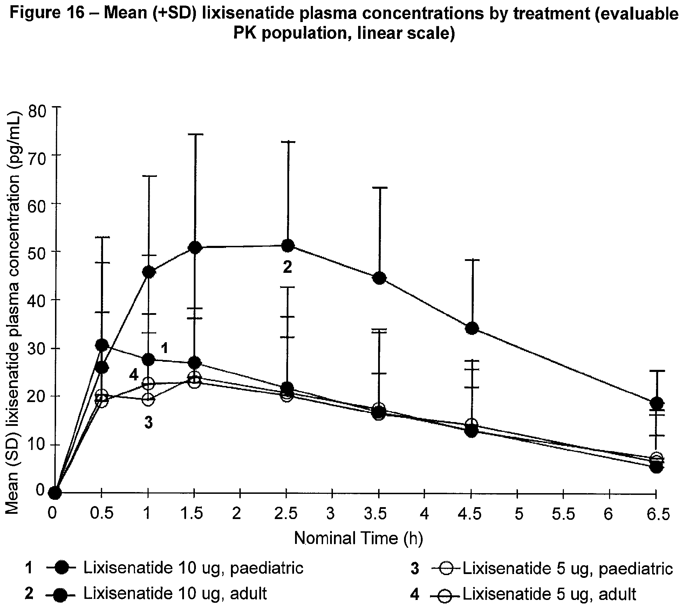

The exposure of lixisenatide in pediatric patients lixisenatide was smaller than in adults at doses of 10 $\mu$g (Figures 15 and 16). The reduction of postprandial plasma glucose (PPG) by treatment with lixisenatide was smaller than in adult patients, and, due to the small number of patients, not significant (Figures 5 to 8).

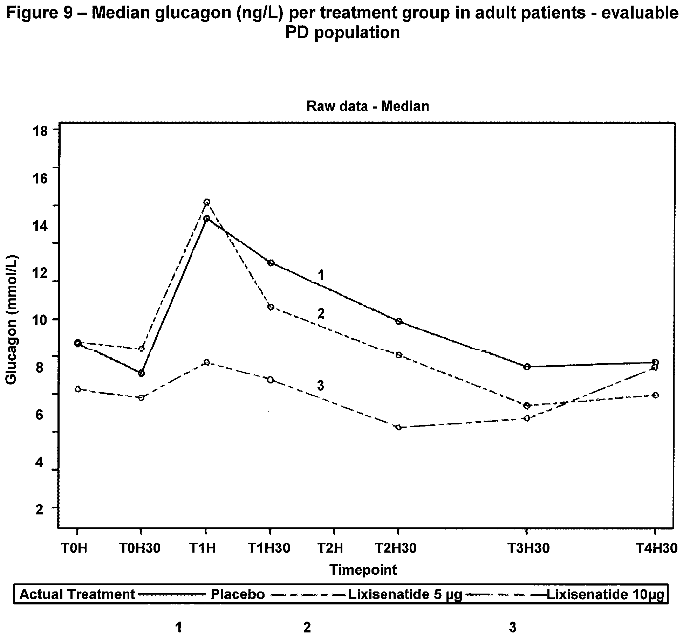

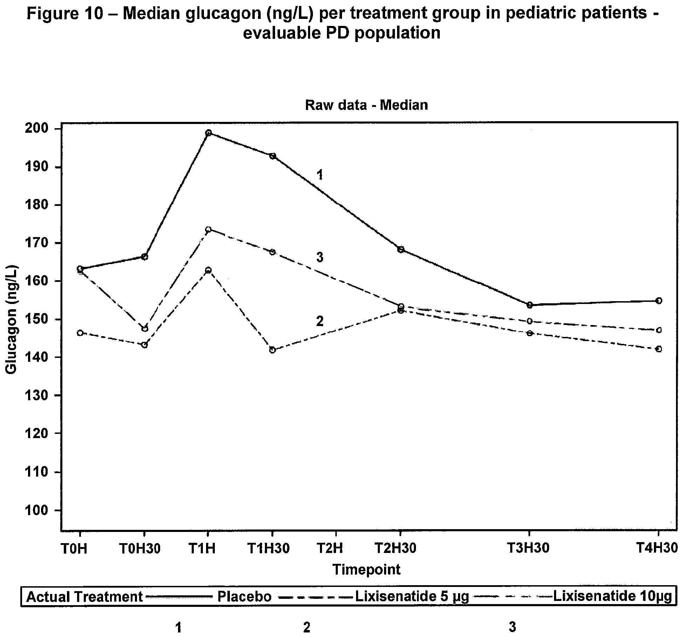

Surprisingly, at a dose of 5 $\mu$g, lixisenatide exhibited a larger reduction in plasma glucacon level than in adult patients (Figures 9 and 10).

In summary, the results of Example 2 indicate differences in the pathophysiology of pediatric type 2 diabetes mellitus patients and adult patients. The fact that lixisenatide can reduce postprandial plasma glucose, postprandial glucagon and insulin secretion in pediatric patients indicates that lixisenatide is effective in the treatment of this patient group.

Example 3 of the present invention describes a randomized, double-blind, placebo-controlled, dose escalation,

study on safety, pharmacokinetics and pharmacodynamics of lixisenatide in pediatric patients with type 2 diabetes not adequately controlled with metformin and/or basal insulin.

A first aspect of the present invention is lixisenatide or/and a pharmaceutically acceptable salt thereof, for use in a method of treatment of pediatric patients suffering from type 2 diabetes mellitus.

Another aspect of the present invention is a pharmaceutical combination for use in a method of treatment of pediatric patients suffering from type 2 diabetes mellitus, said combination comprising

(a) lixisenatide or/and a pharmaceutically acceptable salt thereof, and
(b) metformin or/and a pharmaceutically acceptable salt thereof.

Yet another aspect of the present invention is a pharmaceutical combination for use in a method of treatment of pediatric patients suffering from type 2 diabetes mellitus, said combination comprising

(a) lixisenatide or/and a pharmaceutically acceptable salt thereof,
(b) metformin or/and a pharmaceutically acceptable salt thereof, and
(c) a basal insulin or/and a pharmaceutically acceptable salt thereof.

Yet another aspect of the present invention is a pharmaceutical combination for use in a method of treatment of pediatric patients suffering from type 2 diabetes mellitus, said combination comprising

(a) lixisenatide or/and a pharmaceutically acceptable salt thereof, and
(b) a basal insulin or/and a pharmaceutically acceptable salt thereof.

As used herein, "to be treated according to the present invention", "treatment according to the present invention", or "pediatric treatment according to the present invention" relates to the treatment of pediatric patients suffering from type 2 diabetes mellitus, as defined herein, by (i) lixisenatide or/and a pharmaceutically acceptable salt thereof, or (ii) the pharmaceutical combination as described herein.

The patient in need of the pediatric treatment according to the present invention by may have an age of at least 10 years.

The patient in need of the pediatric treatment according to the present invention as described herein may have an age of less than 18 years.

It is preferred that the patient in need of the pediatric treatment according to the present invention as described herein may have (a) an age of at least 10 years, and (b) and age of less than 18 years.

The patient in need of the pediatric treatment according to the present invention as described herein suffers from type 2 diabetes mellitus.

The pediatric patient to be treated according to the present invention may be a subject suffering from type 2 diabetes mellitus, wherein type 2 diabetes mellitus is not adequately controlled by treatment with metformin monotherapy, for instance with a dose of at least 1.0 g/day metformin or at least 1.5 g/day metformin for 3 months, or/and a dose of at the maximum 2.0 g/day metformin for 3 months.

The pediatric patient to be treated according to the present invention may be a subject suffering from type 2 diabetes mellitus, wherein type 2 diabetes mellitus is not adequately controlled by treatment with a basal insulin or/and metformin, for instance with a dose of at least 1.0 g/day metformin or at least 1.5 g/day metformin for 3 months, or/and a dose of at the maximum 2.0 g/day metformin for 3 months.

The pediatric patient to be treated according to the present invention may be a subject suffering from type 2 diabetes mellitus, wherein type 2 diabetes mellitus is not adequately controlled by treatment with a basal insulin monotherapy.

In the present invention, "not adequately controlled" by the treatment with metformin monotherapy (treatment with metformin alone) means that metformin monotherapy is not sufficient to remove the symptoms of diabetes mellitus. In particular, "not adequately controlled" by the treatment with metformin monotherapy means that the patient does not reach normoglycemic values in terms of, for example, postprandial plasma glucose concentration, glucose excursion or/and fasting plasma glucose concentration.

In the present invention, "not adequately controlled" by the treatment with metformin or/and a basal insulin means that this therapy alone is not sufficient to remove the symptoms of diabetes mellitus. In particular, "not adequately controlled" by the treatment with metformin or/and a basal insulin means that the patient does not reach normoglycemic values in terms of, for example, postprandial plasma glucose concentration, glucose excursion or/and fasting plasma glucose concentration.

In the present invention, "not adequately controlled" by the treatment with a basal insulin monotherapy (treatment with a basal insulin alone) means that this therapy alone is not sufficient to remove the symptoms of diabetes mellitus.

In particular, "not adequately controlled" by the treatment with a basal insulin monotherapy means that the patient does not reach normoglycemic values in terms of, for example, postprandial plasma glucose concentration, glucose excursion or/and fasting plasma glucose concentration.

The term "not adequately controlled" by the treatment with metformin monotherapy in particular relates to the period before onset of treatment according to the present invention. It can be diagnosed before onset of the treatment according to the present invention if monotherapy with metformin adequately controls the type 2 diabetes mellitus or not. For example, such diagnosis may be performed within 1 months, within 2 months or within 3 months before onset of the therapy of the present invention.

The term "not adequately controlled" by the treatment with metformin or/and a basal insulin in particular relates to the period before onset of treatment according to the present invention. It can be diagnosed before onset of the treatment according to the present invention if the therapy with metformin or/and a basal insulin adequately controls the type 2 diabetes mellitus or not. For example, such diagnosis may be performed within 1 months, within 2 months or within 3 months before onset of the therapy of the present invention.

The term "not adequately controlled" by the treatment with a basal insulin monotherapy in particular relates to the period before onset of treatment according to the present invention. It can be diagnosed before onset of the treatment according to the present invention if the therapy with a basal insulin monotherapy adequately controls the type 2 diabetes mellitus or not. For example, such diagnosis may be performed within 1 months, within 2 months or within 3 months before onset of the therapy of the present invention.

By the treatment according to the present invention, adequate control of type 2 diabetes mellitus may be achieved in pediatric patients not adequately controlled with metformin monotherapy.

By the treatment according to the present invention, adequate control of type 2 diabetes mellitus may be achieved in pediatric patients not adequately controlled with metformin or/and a basal insulin.

By the treatment according to the present invention, adequate control of type 2 diabetes mellitus may be achieved in pediatric patients not adequately controlled with a basal insulin monotherapy.

The pediatric patient suffering from type 2 diabetes mellitus to be treated according to the present invention may be obese. A patient can be considered as obese if the body mass index is at least 30 $kg/m^2$. In the present invention, an obese pediatric patient may have a body mass index of at least 30 $kg/m^2$ or at least 31 $kg/m^2$. It is preferred that that the pediatric patient has a body mass index of at least 31 $kg/m^2$.

The pediatric patient suffering from type 2 diabetes mellitus to be treated according to the present invention preferably does not receive an antidiabetic treatment by insulin or/and related compounds.

The pediatric patient suffering from type 2 diabetes mellitus to be treated according to the present invention may suffer from type 2 diabetes mellitus for at least three months. In particular, in the pediatric patient to be treated, type 2 diabetes mellitus has been diagnosed for at least three months before onset of therapy of the present invention.

In the present invention, a pediatric patient may have a HbA1c value in the range of 7 % to 10%, or 7% to 9.9%. In particular the pediatric patient to be treated may have a $HbA_{1c}$ value of at least about 7 %, at least about 7.5 %, at least about 8 %, at least about 8.5 %, at least about 8.65 %, or at least about 9 %.

In particular, in a pediatric patient receiving metformin monotherapy (in particular before onset of therapy according to the present invention), a HbA1c value in the range of 7 % to 10% or 7% to 9.9%, or a $HbA_{1c}$ value of at least about 7 %, at least about 7.5 %, at least about 8 %, at least about 8.5 %, at least about 8.65 %, or at least about 9 % indicates that the type 2 diabetes mellitus is not adequately controlled by metformin monotherapy.

In particular, in a pediatric patient receiving metformin or/and a basal insulin (in particular before onset of therapy according to the present invention), a HbA1c value in the range of 7 % to 10% or 7% to 9.9%, or a $HbA_{1c}$ value of at least about 7 %, at least about 7.5 %, at least about 8 %, at least about 8.5 %, at least about 8.65 %, or at least about 9 % indicates that the type 2 diabetes mellitus is not adequately controlled by metformin or/and a basal insulin.

In particular, in a pediatric patient receiving a basal insulin monotherapy (in particular before onset of therapy according to the present invention), a HbA1c value in the range of 7 % to 10% or 7% to 9.9%, or a $HbA_{1c}$ value of at least about 7 %, at least about 7.5 %, at least about 8 %, at least about 8.5 %, at least about 8.65 %, or at least about 9 % indicates that the type 2 diabetes mellitus is not adequately controlled by a basal insulin monotherapy.

In the present invention, normoglycemic values are blood glucose concentrations of in particular 60 - 140 mg/dl (corresponding to 3.3 to 7.8 mmol/L). This range refers in particular to blood glucose concentrations under fasting conditions and postprandial conditions.

Criteria for a type 2 diabetes mellitus diagnosis include:

- the fasting plasma glucose concentration (FPG) is ≥ 7.0 mmol/L (126 mg/dl), or

- the post challenge plasma glucose concentration is > 11.1 mmol/L (200 mg/dl), performed as described by the World Health Organization (Definition, Diagnosis and Classification of Diabetes Mellitus and its Complications. Part 1: Diagnosis and Classification of Diabetes Mellitus. WHO/NCD/NCS/99.2. Geneva; 1999), using a glucose load containing the equivalent of 75 g anhydrous glucose dissolved in water, or

- symptoms of diabetes and a casual plasma glucose ≥ 200 mg/dl (11.1 mmol/L).

These criteria are described in the Global IDF/ISPAD Guideline for Diabetes in Childhood and Adolescence (International Diabetes Federation, ISBN 2-930229-72-1).

The diagnosis of Type 2 Diabetes should not be based on a single plasma glucose concentration. Diagnosis may require continued observation with fasting and/or postprandial blood glucose levels and/or an oral glucose tolerance test.

According to Craig (Pediatric Diabetes 2014: 15(Suppl. 20): 4-17), fasting plasma glucose (FPG) and post challenge (postload) glucose can be classified as follows:

- FPG < 5.6 mmol/L (100 mg/dL) = normal fasting glucose concentration.

- FPG 5.6 to 6.9 mmol/L (100-125 mg/dL) = impaired fasting glucose concentration.

- FPG ≥ 7.0 mmol/L (126 mg/dL) = provisional diagnosis of diabetes (the diagnosis must be confirmed, as described above)

The corresponding categories when the Oral Glucose Tolerance Test (OGTT) is used are as follows:

- Two hour postload glucose < 7.8 mmol/L (140 mg/dL) = normal glucose tolerance.

- Two hour postload glucose 7.8 to <11.1 mmol/L (140-200 mg/dL) = impaired glucose tolerance.

- Two hour postload glucose ≥ 11.1 mmol/L (200 mg/dL) = provisional diagnosis of diabetes (the diagnosis must be confirmed, as described above).

Impaired glucose tolerance (IGT) and impaired fasting glucose concentration (IFG) are intermediate stages in the natural history of disordered carbohydrate metabolism between normal glucose homeostasis and diabetes.

In the present invention, normoglycemic glucose concentrations can include impaired glucose concentrations, as described herein.

In the present invention, normoglycemic values of fasting plasma glucose are blood glucose concentrations of in particular < 5.6 mmol/L or < 7.0 mmol/L.

In the present invention, normoglycemic values of postprandial plasma glucose, as defined herein, are blood glucose concentrations of in particular <7.8 mmol/L or < 11.1 mmol/L.

The pediatric patient to be treated according to the present invention may have a 2 hours postprandial plasma glucose concentration of at least 11.1 mmol/L, at least 12 mmol/L, or at least 13 mmol/L. These plasma glucose concentrations exceed normoglycemic concentrations.

In particular, in a pediatric patient receiving metformin monotherapy (in particular before onset of therapy according to the present invention), a 2 hours postprandial plasma concentration of at least 11.1 mmol/L, at least 12 mmol/L or at least 13 mmol/L indicates that the type 2 diabetes mellitus is not adequately controlled by metformin monotherapy.

In particular, in a pediatric patient receiving metformin or/and a basal insulin (in particular before onset of therapy according to the present invention), a 2 hours postprandial plasma concentration of at least 11.1 mmol/L, at least 12 mmol/L or at least 13 mmol/L indicates that the type 2 diabetes mellitus is not adequately controlled by metformin or/and a basal insulin.

In particular, in a pediatric patient receiving a basal insulin monotherapy (in particular before onset of therapy according to the present invention), a 2 hours postprandial plasma concentration of at least 11.1 mmol/L, at least 12 mmol/L or at least 13 mmol/L indicates that the type 2 diabetes mellitus is not adequately controlled by a basal insulin monotherapy.

"Postprandial" is a term that is well known to a person skilled in the art of diabetology. The term "postprandial" describes in particular the phase after an ingestion of a meal or/and exposure to glucose under experimental conditions. In a healthy person this phase is characterised by an increase and subsequent decrease in blood glucose concentration. The postprandial phase typically ends up to 2 h after a meal or/and exposure to glucose.

Determination of postprandial plasma glucose is well-known (see, e.g. Crapo et al., Diabetes, 1977, 26(12):1178-1183). A typical standardized breakfast suitable for exposure to glucose under experimental conditions in a meal test is described in the Appendix of Example 2.

The pediatric patient to be treated according to the invention may have a glucose excursion of at least 3 mmol/L, at least 3.5 mmol/L or at least 3.65 mmol/L. In the present invention, the glucose excursion is in particular the difference of the 2 hours postprandial plasma glucose concentration and the plasma glucose concentration prior to a meal test, e.g. the plasma glucose concentration 30 minutes prior to a meal test.

In particular, in a pediatric patient receiving metformin monotherapy (in particular before onset of therapy according to the present invention), a glucose excursion of at least 3 mmol/L, at least 3.5 mmol/L or at least 3.65 mmol/L indicates that the type 2 diabetes mellitus is not adequately controlled by metformin monotherapy

In particular, in a pediatric patient receiving metformin or/and a basal insulin (in particular before onset of therapy according to the present invention), a glucose excursion of at least 3 mmol/L, at least 3.5 mmol/L or at least 3.65 mmol/L indicates that the type 2 diabetes mellitus is not adequately controlled by metformin or/and a basal insulin.

In particular, in a pediatric patient receiving a basal insulin monotherapy (in particular before onset of therapy according to the present invention), a glucose excursion of at least 3 mmol/L, at least 3.5 mmol/L or at least 3.65 mmol/L indicates that the type 2 diabetes mellitus is not adequately controlled by a basal insulin monotherapy.

The pediatric patient to be treated according to the invention may have a fasting plasma glucose concentration of at least 8 mmol/L, or at least 8,5 mmol/L. These plasma glucose concentrations exceed normoglycemic concentrations.

In particular, in a pediatric patient receiving metformin monotherapy (in particular before onset of therapy according to the present invention), a fasting plasma glucose concentration of at least 8 mmol/L, or at least 8,5 mmol/L indicates that the type 2 diabetes mellitus is not adequately controlled by metformin monotherapy.

In particular, in a pediatric patient receiving metformin or/and a basal insulin (in particular before onset of therapy according to the present invention), a fasting plasma glucose concentration of at least 8 mmol/L, or at least 8,5 mmol/L indicates that the type 2 diabetes mellitus is not adequately controlled by metformin or/and a basal insulin.

In particular, in a pediatric patient receiving basal insulin monotherapy (in particular before onset of therapy according to the present invention), a fasting plasma glucose concentration of at least 8 mmol/L, or at least 8,5 mmol/L indicates that the type 2 diabetes mellitus is not adequately controlled by basal insulin monotherapy.

The pediatric patient to be treated according to the invention may have a C-peptide plasma concentration of at least 1.2 nmol/L in fasting conditions.

The pediatric patient to be treated according to the invention may have a plasma glucagon level of at least 140 ng/L in fasting conditions.

In another aspect of the present invention, (i) lixisenatide or/and a pharmaceutically acceptable salt thereof, or (ii) the combination as described herein can be used for improving (i.e. reducing) the 2 hours postprandial plasma glucose in a pediatric patient suffering from type 2 diabetes mellitus. Reduction means in particular that the 2 hours postprandial plasma glucose concentration reaches normoglycemic values or at least approaches these values.

In another aspect of the present invention, (i) lixisenatide or/and a pharmaceutically acceptable salt thereof, or (ii) the combination as described herein can be used for improving (i.e. reducing) the glucose excursion in a pediatric patient suffering from type 2 diabetes mellitus. Reduction means in particular that the glucose excursion reaches normoglycemic values or at least approaches these values.

In another aspect of the present invention, (i) lixisenatide or/and a pharmaceutically acceptable salt thereof, or (ii) the combination as described herein can be used for improving (i.e. reducing) the plasma glucagon concentration in a pediatric patient suffering from type 2 diabetes mellitus.

Lixisenatide or/and a pharmaceutically acceptable salt thereof, or the combination of the present invention can be used in the treatment of one or more of the medical indications described herein, for example in treatment of type 2 diabetes mellitus patients, as described herein, or for conditions associated with type 2 diabetes mellitus, such as for the improvement of glucose excursion, for improvement of the postprandial plasma glucose concentration, or/and for improvement of plasma glucagon concentration.

The plasma glucagon concentration, as used herein, is in particular the postprandial plasma glucagon concentration.

In the present invention, metformin includes pharmaceutically acceptable salts thereof. The person skilled in the art knows suitable pharmaceutically acceptable salts of metformin.

In the present invention, metformin can be administered according to commonly known administration protocols of metformin in accordance with the terms of marketing authorization. Metformin can be administered to patients from 10 years. For example, metformin can be administrated once daily, twice daily or three times a day. In particular, the metformin dose applied before the onset of the therapy as disclosed herein is continued in combination with lixisenatide or/and a pharmaceutically acceptable salt thereof, as disclosed herein.

In the present invention, metformin may be administered orally. The skilled person knows formulations of metformin suitable for treatment of type 2 diabetes mellitus by oral administration. Metformin may be administered to a pediatric patient in need thereof, in an amount sufficient to induce a therapeutic effect. Metformin may be administered in a dose of at least 1.0 g/day or at least 1.5 g/day. Metformin may be administered in a dose of at the maximum of 2.0 g/day. The daily metformin dose can be divided into 2 or three separate doses. For oral administration, metformin may be formulated in a solid dosage form, such as a tablet or pill. Metformin may be formulated with suitable pharmaceutically acceptable carriers, adjuvants, or/and auxiliary substances.

In the present invention, lixisenatide or/and a pharmaceutically acceptable salt may be administered in an add-on therapy to administration of metformin.

In the present invention, the terms "add-on", "add-on treatment" and "add-on therapy" can relate to treatment according to the present invention with metformin and lixisenatide.

In the present invention, the terms "add-on", "add-on treatment" and "add-on therapy" can also relate to treatment according to the present invention with a basal insulin or/and metformin, and lixisenatide.

In the present invention, the terms "add-on", "add-on treatment" and "add-on therapy" can also relate to treatment according to the present invention with a basal insulin and lixisenatide.

Metformin, lixisenatide or/and the basal insulin each may be administered in a once-a-day-dosage. Metformin, the basal insulin and lixisenatide may be administered by different administration routes. Metformin may be administered orally, and lixisenatide and the basal insulin may be administered parenterally.

In particular, "add-on", "add-on treatment" and "add-on therapy" mean that the dose of metformin administered before the onset of the treatment with lixisenatide or/and a pharmaceutically acceptable salt thereof, as disclosed herein, is continued in combination with lixisenatide or/and a pharmaceutically acceptable salt thereof.

In particular, "add-on", "add-on treatment" and "add-on therapy" mean that the dose of the basal insulin administered before the onset of the treatment with lixisenatide or/and a pharmaceutically acceptable salt thereof, as disclosed herein, is continued in combination with lixisenatide or/and the pharmaceutically acceptable salt thereof. Alternatively, the dose of the basal insulin may be reduced in combination with lixisenatide or/and the pharmaceutically acceptable salt thereof.

In the present invention, lixisenatide includes pharmaceutically acceptable salts thereof. The person skilled in the art knows suitable pharmaceutically acceptable salts of lixisenatide. A preferred pharmaceutically acceptable salt of lixisenatide employed in the present invention is the acetate salt of lixisenatide.

In the present invention, lixisenatide or/and the pharmaceutically acceptable salt thereof may be administered to a pediatric patient in need thereof, in an amount sufficient to induce a therapeutic effect.

In the present invention, lixisenatide or/and the pharmaceutically acceptable salt thereof may be formulated with suitable pharmaceutically acceptable carriers, adjuvants, or/and auxiliary substances.

Lixisenatide or/and a pharmaceutically acceptable salt thereof may be administered parenterally, e.g. by injection (such as by intramuscular or by subcutaneous injection). Suitable injection devices, for instance the so-called "pens" comprising a cartridge comprising the active ingredient, and an injection needle, are known. Lixisenatide or/and a pharmaceutically acceptable salt thereof may be administered in a suitable amount, for instance in an amount in the range of 5 µg to 10 µg per dose.

In the present invention, lixisenatide or/and a pharmaceutically acceptable salt thereof may be administered in a daily dose in the range of 5 to 10 µg. Lixisenatide or/and a pharmaceutically acceptable salt thereof may be administered by one injection per day. Lixisenatide or/and a pharmaceutically acceptable salt thereof may be administered about 30 min before breakfast.

In the present invention, lixisenatide or/and a pharmaceutically acceptable salt thereof may be provided in a liquid composition, which preferably is an aqueous formulation. It is preferred that the liquid composition is suitable for parenteral administration, in particular for injection. The skilled person knows such liquid compositions of lixisenatide. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 - 8.5, pH 4.0 - 8.5, or pH 6.0 - 8.5. The pH may be adjusted by a pharmaceutically acceptable diluted acid (typically HCl) or pharmaceutically acceptable diluted base (typically NaOH).

The liquid composition comprising lixisenatide or/and a pharmaceutically acceptable salt thereof may comprise a suitable preservative. A suitable preservative may be selected from phenol, m-cresol, benzyl alcohol and p-hydroxybenzoic acid ester. A preferred preservative is m-cresol.

The liquid composition comprising lixisenatide or/and a pharmaceutically acceptable salt thereof may comprise a tonicity agent. A suitable tonicity agent may be selected from glycerol, lactose, sorbitol, mannitol, glucose, NaCl, calcium or magnesium containing compounds such as $CaCl_2$. The concentration of glycerol, lactose, sorbitol, mannitol and glucose may be in the range of 100 - 250 mM. The concentration of NaCl may be up to 150 mM. A preferred tonicity agent is glycerol.

The liquid composition comprising lixisenatide or/and a pharmaceutically acceptable salt thereof may comprise methionine from 0.5 µg/mL to 20 µg/ml, preferably from 1 µg /ml to 5 µg/ml. Preferably, the liquid composition comprises L-methionine.

In the present invention, the basal insulin includes pharmaceutically acceptable salts thereof. The person skilled in the art knows suitable pharmaceutically acceptable salts.

In the present invention, the basal insulin or/and the pharmaceutically acceptable salt thereof may be administered to a pediatric patients in need thereof, in an amount sufficient to induce a therapeutic effect.

In the present invention, the basal insulin or/and the pharmaceutically acceptable salt thereof may be formulated with suitable pharmaceutically acceptable carriers, adjuvants, or/and auxiliary substances.

The basal insulin or/and a pharmaceutically acceptable salt thereof may be administered parenterally, e.g. by injection (such as by intramuscular or by subcutaneous injection). Suitable injection devices, for instance the so-called "pens" comprising a cartridge comprising the active ingredient, and an injection needle, are known.

Also described herein is a method of pediatric treatment, said method comprising administering to a patient in need of a pediatric treatment, lixisenatide or/and a pharmaceutically acceptable salt thereof.

In this method of treatment, the pediatric patient is a patient as described herein. In particular, the pediatric patient suffers from type 2 diabetes mellitus, as described herein. Lixisenatide is prepared as described herein, in particular as a liquid formulation suitable for parenteral administration.

Also described herein is a method of pediatric treatment, said method comprising administering to a patient in need of a pediatric treatment, a pharmaceutical combination, said combination comprising

(a) lixisenatide or/and a pharmaceutically acceptable salt thereof, and
(b) metformin or/and a pharmaceutically acceptable salt thereof.

Also described herein is a method of pediatric treatment, said method comprising administering to a patient in need of a pediatric treatment, a pharmaceutical combination, said combination comprising

(a) lixisenatide or/and a pharmaceutically acceptable salt thereof, and
(b) a basal insulin or/and a pharmaceutically acceptable salt thereof.

Also described herein is a method of pediatric treatment, said method comprising administering to a patient in need of a pediatric treatment, a pharmaceutical combination, said combination comprising

(a) lixisenatide or/and a pharmaceutically acceptable salt thereof
(b) metformin or/and a pharmaceutically acceptable salt thereof, and
(c) a basal insulin or/and a pharmaceutically acceptable salt thereof.

In these methods of treatment, the pediatric patient is a patient as described herein. In particular, the pediatric patient suffers from type 2 diabetes mellitus, as described herein. Lixisenatide is prepared as described herein, in particular as a liquid formulation suitable for parenteral administration. Metformin is prepared as described herein, in particular for oral administration. The basal insulin is prepared as described herein, in particular as a liquid formulation suitable for parenteral administration.

Also described herein is a method for the improvement of glucose excursion, for the improvement of the postprandial plasma glucose concentration, or/and for the improvement of plasma glucagon concentration, said method comprising administering to a pediatric patient, as described herein, (i) lixisenatide or/and a pharmaceutically acceptable salt thereof, or (ii) the combination as described herein.

Yet another aspect of the present invention is the use of lixisenatide or/and a pharmaceutically acceptable salt thereof, for the manufacture of a medicament for use in the treatment of pediatric patients suffering from type 2 diabetes mellitus. The pediatric patient is a patient as described herein. Lixisenatide is prepared as described herein, in particular as a liquid formulation suitable for parenteral administration.

Yet another aspect of the present invention is the use of a pharmaceutical combination, said combination comprising

(a) lixisenatide or/and a pharmaceutically acceptable salt thereof, and
(b) metformin or/and a pharmaceutically acceptable salt thereof, for the manufacture of a medicament for use in the treatment of pediatric patients suffering from type 2 diabetes mellitus.

Yet another aspect of the present invention is the use of a pharmaceutical combination, said combination comprising

(a) lixisenatide or/and a pharmaceutically acceptable salt thereof, and
(b) a basal insulin or/and a pharmaceutically acceptable salt thereof, for the manufacture of a medicament for use in the treatment of pediatric patients suffering from type 2 diabetes mellitus.

Yet another aspect of the present invention is the use of a pharmaceutical combination, said combination comprising

(a) lixisenatide or/and a pharmaceutically acceptable salt thereof,
(b) metformin or/and a pharmaceutically acceptable salt thereof, and
(c) a basal insulin or/and a pharmaceutically acceptable salt thereof, for the manufacture of a medicament for use in the treatment of pediatric patients suffering from type 2 diabetes mellitus.

In these uses, the pediatric patient is a patient as described herein. Lixisenatide is prepared as described herein, in particular as a liquid formulation suitable for parenteral administration. Metformin is prepared as described herein, in particular for oral administration. The basal insulin is prepared as described herein, in particular as a liquid formulation suitable for parenteral administration.
Yet another aspect of the present invention is the use of (i) lixisenatide or/and a pharmaceutically acceptable salt thereof, or (ii) the combination as described herein, for the manufacture of a medicament for the improvement of glucose excursion, for the improvement of the postprandial plasma glucose concentration, or/and for the improvement of plasma glucagon concentration, wherein the patient to be treated is a pediatric patient suffering from type 2 diabetes mellitus, as described herein.
The invention is further illustrated by the following examples and figures.

**Figure legends**

Figure 1 Graphical study design of Example 1. * Mandatory blood sampling D-30 to D-25 for laboratory tests (eg, anti-IA2 and anti-GAD autoantibodies, fasting C-peptide). The following assessments will be done at TP1, TP2, TP3 and EOS: Physical examination and vital signs, ECG and AE assessment (except Screening).

Figure 2 3 cartridges injections

Figure 3 Body mass index (BMI) for age percentiles by gender: Boys, 2 to 20 years

Figure 4 Body mass index (BMI) for age percentiles by gender: Girls, 2 to 20 years

Figure 5 Mean ± SEM plasma glucose per treatment group in adult patients - evaluable PD population

Figure 6 Mean ± SEM plasma glucose per treatment group in pediatric patients - evaluable PD population

Figure 7 Median plasma glucose (mmol/L) per treatment group in adult patients - evaluable PD population

Figure 8 Median plasma glucose (mmol/L) per treatment group in pediatric patients - evaluable PD population

Figure 9 Median glucagon (ng/L) per treatment group in adult patients - evaluable PD population

Figure 10 Median glucagon (ng/L) per treatment group in pediatric patients - evaluable PD population

Figure 11 Median plasma insulin (pmol/L) per treatment group in adult patients - evaluable PD population

Figure 12 Median plasma insulin (pmol/L) per treatment group in pediatric patients - evaluable PD population

Figure 13 Median C-peptide (nmol/L) per treatment group in adult patients - evaluable PD population

Figure 14 Median C-peptide (nmol/L) per treatment group in pediatric patients - evaluable PD population

Figure 15 Mean (+ SD) lixisenatide plasma concentrations by treatment (full PK population, linear scale)

Figure 16 Mean (+SD) lixisenatide plasma concentrations by treatment (evaluable PK population, linear scale)

Figure 17 Graphical study design of Example 3. * Placebo solution and volume to be injected matching to lixisenatide solution: 50 µl during Weeks 1 and 2 (injector device Tactipen®), 200 µl during Weeks 3 and 4 (green injector device

Delta14®) and 200 μ_ during Weeks 5 and 6 (purple injector device Delta14®)

Example 1

A randomized, double-blind, placebo controlled trial to assess safety, tolerability, pharmacokinetics and pharmacodynamics of lixisenatide in paediatric (10 - 17 years old) and adult patients with type 2 diabetes

| TITLE | A randomized, double-blind, placebo controlled trial to assess safety, tolerability, pharmacokinetics and pharmacodynamics of lixisenatide in paediatric (10 - 17 years old) and adult patients with type 2 diabetes. |
|---|---|
| INVESTIGATOR/TRIAL LOCATION | Multi-center |
| STUDY OBJECTIVE(S) | **Primary objective:**<br>• To investigate the effects of a single subcutaneous lixisenatide dose of 5 µg and 10 µg as compared to placebo in reducing postprandial glucose (PPG) assessed as area under the plasma glucose concentration curve (AUC) after a standardized liquid meal (breakfast) in type 2 diabetic paediatric population (10-17 years old) and adults as controls<br>**Secondary objectives:**<br>To evaluate in both paediatric and adult populations:<br>• pharmacokinetic parameters of lixisenatide in plasma after single subcutaneous ascending doses<br>• the maximum PPG excursion, and on the changes in insulin, C-peptide and glucagon plasma concentrations following a standardized breakfast<br>• safety and tolerability |
| STUDY DESIGN | Phase I, multicenter, double-blind, randomized, placebo controlled, single-dose, 3-period, 3-treatment, 6 sequence cross-over study in paediatric and adult with type 2 diabetic patients (see Section 6.1 )<br>The study is double blind with regard to active treatment versus placebo. The study drug volume (i.e., dose of active drug at 5 µg and 10 µg or matching placebo) is not blinded but placebo volumes matched to 5 µg and 10 µg in a ratio 1:1. |
| STUDY POPULATION<br>**Main selection criteria:** | **Inclusion criteria:**<br>• Male and female patients with type 2 diabetes mellitus (T2DM), as defined by WHO (fasting plasma glucose ≥7 mmol/l (126 mg/dl) or 2 hours postprandial plasma glucose ≥11.1 mmol/l (200 mg/dl)), diagnosed at least 3 months at the time of screening visit, with or without metformin (stable dose for at least 4 weeks prior to randomization)<br>• HbA1c ≥ 7% and ≤ 10% at screening<br>• Fasting C-peptide at screening > 0.6 ng/mL<br>• Negative test for anti-islet cell antibodies (or insulinoma associated protein (IA2)) and anti-glutamic acid decarboxylase (GAD) autoantibodies<br>Paediatric population:<br>• Male and female ≥ 10 and < 18 years of age with at least 3 |

patients below 15 years of age and limited to 3 patients ≥ 16 years of age, BMI > 85th percentile for age and gender (body weight > 50 kg)

Adult population:

- Male and female patients ≥ 18 and ≤ 65 years of age, and with BMI > 25 kg/m2 and ≤ 37 kg/m2

Exclusion criteria:

- Diabetes other than T2DM
- Use of antihyperglycaemic medicinal product(s), other than metformin
- History of unexplained pancreatitis
- Personal or family history of medullary thyroid cancer (MTC) or genetic conditions that predispose to MTC (eg, multiple endocrine neoplasia syndromes)
- Calcitonin ≥ 20pg/mL (5.9 pmol/L)at screening

| | |
|---|---|
| **Total expected number of patients:** | 12 paediatric patients and 12 adult patients with type 2 diabetic patients |
| **INVESTIGATOR/TRIAL LOCATION** | Worldwide |

| STUDY TREATMENT(s) | |
|---|---|

**Investigational Medicinal Product(s) Formulation:**

| Compound | Dose | Form | Route of administration |
|---|---|---|---|
| Lixisenatide | 5 µg in 50 µL | solution for injection 100 µg/mL | subcutaneous injection |
| Lixisenatide | 10 µg in 100 µL | solution for injection 100 µg/mL | subcutaneous injection |

Lixisenatide is supplied as a sterile aqueous solution for subcutaneous (s.c.) injection in a 3-mL glass cartridge.

Placebo is supplied as 3-mL aqueous solution (in cartridge).

Both to be injected with the OptiClik® self-injector device.

**Route(s) of administration:** Thin needles will be used to minimize discomfort.

s.c.

**Dose regimen:** 3 treatment periods each lasting 2 days. In each treatment period patients receive a subcutaneously injected single dose of either 5 µg or 10 µg lixisenatide with 5 µg preceding the 10 µg dose level or volume matched placebo (50 µL or 100 µL).

IMP will be administered in fasted conditions 30 min before a standardized, liquid meal (breakfast).

**Non Investigational Medicinal Product(s)** NA

| PRIMARY ENDPOINT(S) AND MAIN SECONDARY ENDPOINT(S) | Pharmacodynamics: |
|---|---|

Primary endpoint:

- Plasma glucose: GLU-AUC$_{0:30-4:30h}$: area under the curve for plasma glucose concentration time profile calculated from time of standardized breakfast start (30 min after IMP injection=T0.5) until 4 hours later (T4.5) subtracting the pre-

| | |
|---|---|
| | meal value T0.5h |
| | Secondary endpoints: |
| | • Post Prandial Glucose excursion ($PPG_{0:30-4:30h}$): maximum change from time of standardized breakfast start (30 min after IMP injection=T0.5) until 4 hour later (T4.5) in postprandial plasma glucose |
| | • Insulin, C-peptide and glucagon ($AUC_{0:30-4:30h}$) : area under the curve for insulin, C-peptide and glucagon concentrations time profiles from time of standardized breakfast start (30 min after IMP injection=T0.5) until 4 hours later (T4.5) |
| | • Pharmacokinetics: lixisenatide plasma concentration, PK parameters ($C_{max}$, $T_{max}$, $AUC_{last}$, AUC) |
| | • Safety: clinical laboratory, ECG parameters, vital signs, local tolerability and adverse events |
| **ASSESSMENT SCHEDULE** | **Pharmacodynamics:** |
| | Blood samples will be taken immediately prior to IMP injection 30 min before a standardized breakfast, then just prior to the standardized breakfast, and at 30, 60, 90, 120, 180, and 240 min thereon for glucose assessments on Day 1of each of the 3 treatment periods for $GLU\text{-}AUC_{0:30-4:30h}$. |
| | For secondary endpoints including safety refer to study and period flow charts. |
| **STATISTICAL CONSIDERATIONS** | Both cross-overs will be analyzed separately. Results will be compared between the two populations descriptively. |
| | **Pharmacodynamics:** |
| | Analysis of population: The pharmacodynamic population will consist of patients randomized and treated and having blood samples for reliable evaluation. Within each cross-over, the analyses of the primary pharmacodynamic endpoint will be performed based on the pharmacodynamic population. $Glu\text{-}AUC_{0:30-4:30h}$ will be analyzed using a linear mixed model with sequence, period, and treatment effect and patient-within-sequence as random effect, and the T0.5 h plasma glucose concentration as covariate. The least square mean differences between treatment groups and the corresponding 90% confidence interval (CI) will be calculated within the linear mixed model framework. A significance level of $p< 0.05$ will be used. |
| | Secondary pharmacodynamic parameters will be analyzed using the same statistical model as described above with the corresponding T0.5 h values as covariates. |
| | **Pharmacokinetics:** |
| | Log- transformed lixisenatide pharmacokinetic parameters Cmax, AUClast, and AUC will be analyzed using a linear mixed effect model with fixed terms for sequence, treatment and a random term for a patient-within-sequence. Estimates and 90% CI for the geometric mean ratio of 5 µg lixisenatide and versus 10µg lixisenatide will be obtained by computing estimate and 90% CI for the difference between treatment means within the linear mixed effects model framework, and then converting to |

| | ratio by the antilog transformation to the original scale. |
|---|---|
| | **Safety**: |
| | The safety analysis will be based on the review of the individual values (clinically significant abnormalities) and descriptive statistics (summary tables and plots if appropriate) by treatment. |
| | Treatment-emergent adverse events (TEAEs) classified in system-organ classes and preferred terms then summarized by number and percentage of patients and number of TEAEs. Individual clinical laboratory data, vital sign, and ECG data will be listed and flagged for potentially clinically significant abnormalities (PCSAs) and for lower and upper clinical laboratory limits. Frequency of patients with abnormalities and with PCSAs will be summarized for each type of parameter by treatment. |
| **DURATION OF STUDY PERIOD (per patient)** | Screening: D -30 to D -2 prior to inclusion with a minimal period of 25 days |
| | Treatment Period: 3 Periods each lasting 1 day (up to 2 days if there is an institutionalization on D-1 evening ) (discharge in the afternoon of D1 of each period) |
| | EOS: 1 to 6 days after last dosing (D2 to D7 after Period 3) |
| | Total duration: 4 to 7 weeks |

## 1. FLOW CHARTS

### 1.1 GRAPHICAL STUDY DESIGN

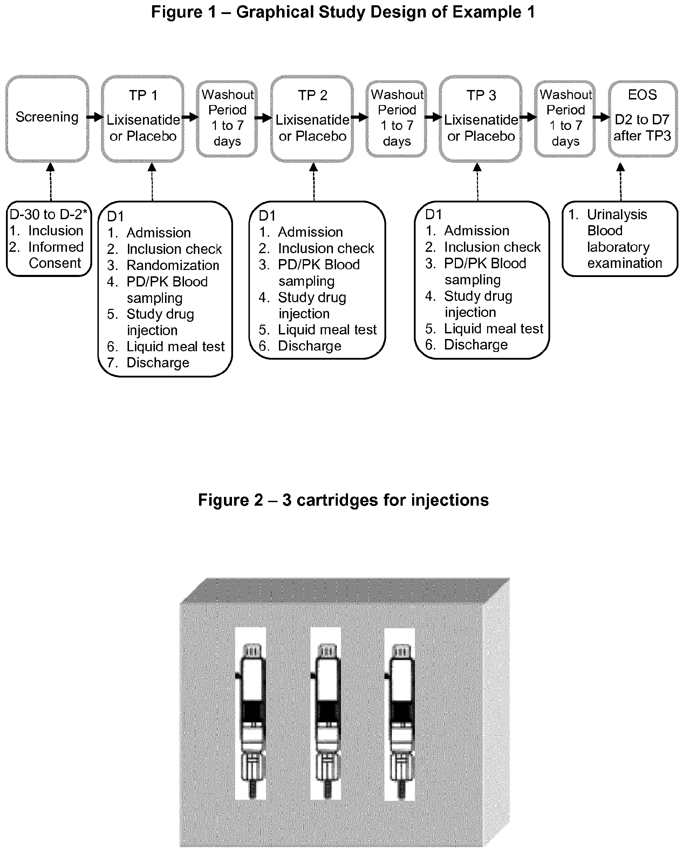

The graphical study design of Example 1 is shown in Figure 1.

### 1.2 STUDY FLOW CHART

| Phase | Screening (e) | Study Treatment Period 1 | Washout Period between Period 1 and 2 | Study Treatment Period 2 | Washout Period between Period 2 and 3 | Study Treatment Period 3 | End-of-study visit |
|---|---|---|---|---|---|---|---|
| **DAY** | D-30 to D-2 | D1 | 1 day - 7 days | D1 | 1 day-7 days | D1 | D2 to D7 of Period 3 |
| Informed Consent | X | | | | | | |
| Institutionalization | | X (a) | | X (a) | | X (a) | |
| Discharge | X | X | | X | | X | |
| Medical/surgical History | X | X | | X (b,e) | | X (b,e) | |
| Prior/Concomitant medication | X | X | | X | | X | X |
| Height | X | | | | | | |
| Body weight | X | X | | X | | X | X |
| Urine Drug Screen, Alcohol test (c) | X | X | | X | | X | |
| Serologies (d) | X | | | | | | |
| Anti-lixisenatide antibodies | | X (e) | | | | | |
| Autoantibodies test (f) | X | | | | | | |
| Randomization (g) | | X | | | | | |
| IXRS call (h) | X | X | | | | X | X |
| Standardized meal test (breakfast) | | X | | X | | X | |
| IMP Administration | | X | | X | | X | |
| **SAFETY** | | | | | | | |
| Physical examination | X | X | | X | | X | X |
| Blood pressure/Heart rate (i) | X | X | | X | | X | X |
| Body temperature | X | X(e) | | X(e) | | X(e) | X |

| DAY | D-30 to D-2 | D1 | 1 day - 7 days | D1 | 1 day - 7 days | D1 | D2 to D7 of Period 3 |
|---|---|---|---|---|---|---|---|
| 12-lead ECG (j) | X | X | | X | | X | X |
| Blood Laboratory Examination (k) | X | | | | | | X |
| Pregnancy test (l) | X | X(e) | | X (e) | | X (e) | |
| Urinalysis (m) | X | | | | | | X |
| AE/SAE collection | X | X | X | X | X | X | X |
| **PHARMACOKINETICS** | | | | | | | |
| PK sample | | X | | X | | X | |
| **PHARMACODYNAMICS (n)** | | | | | | | |
| HbA1c | X | | | | | | |
| Insulin, C-peptide, glucagon, plasma glucose | X (o) | X | | X | | X | |

EP 3 244 912 B1

(continued)

| DAY | D-30 to D-2 | D1 | 1 day - 7 days | D1 | 1 day - 7 days | D1 | D2 to D7 of Period 3 |
|---|---|---|---|---|---|---|---|
| DAY | D-30 to D-2 | D1 | 1 day - 7 days | D1 | 1 day - 7 days | D1 | D2 to D7 of Period 3 |

a Only a single visit (Day 1) scheduled per period including the tests and examination to be done before IMP OR at the convenience of patient and the possibilities of the investigational site, institutionalization on Day -1 afternoon/evening may be planned for starting all examinations and tests to bedone before the IMP administration b Period 2 and 3 just check absence of infection and adherence to study restrictions before IMP administration c Urine drug screen: amphetamines/metamphetamines, barbiturates, benzodiazepines, cannabinoids, cocaine, opiates d Hepatitis B antigen, hepatitis C antibodies, anti-HIV1 and anti-HIV2 antibodies.

e Before study drug administration f Blood sampling **to be done at latest on Day -25** for obtaining the results of autoantibody (anti-IA2 and anti-GAD) testing before the randomization g Randomization and allocation of the patient's treatment group using a centralized treatment allocation system h Allocation of the patient's number at screening -On Day 1 (Period 1), allocation of the patient's treatment kit to be used and track of drug inventory - At period 3, end of treatment period and at EOS end of the study -Call to IXRS for screen failures or drop-out if any i Vital signs (Heart rate and blood pressure) measured after 10 min in supine resting position j 12- lead ECG will be recorded after at least 10 min in supine position. Automatic reading will be performed.

k Hematology: Red Blood Cell Count, Hematocrit, Hemoglobin, White Blood Cell Count with differential (Neutrophils, Lymphocytes, Monocytes, Basophils, Eosinophils), Platelets; Serum Chemistry: Sodium, Potassium, Chloride, Calcium, AST, ALT, alkaline phosphatise, gamma-glutamyl transferase (GGT), total and conjugated bilirubin, Urea, Creatinine, Glucose, Albumin, total Protein, total Cholesterol, Triglycerides, creatine phosphokinase (CPK), amylase, lipase) and at screening only calcitonin for all patients l In females of reproductive potential (Tanner Stage $\geq$ 3), serum beta-HCG only at screening and urinary pregnancy test at each treatment period m Urinalysis: proteins, glucose, blood (erythrocytes/leucocytes), ketone bodies, pH n Pharmacodynamic parameters in Central laboratory o Only 2 tests: plasma glucose and C-peptide in fasting condition

## 1.3 PERIOD 1 FLOW CHART

| Day per period | D1 – Period 1 | | | | | | | | |
|---|---|---|---|---|---|---|---|---|---|
| Indicative clock time (a) | 07:30 (a) | 08:00 | 08:30 | 09:00 | 09:30 | 10:00 | 11:00 | 12:00 | 14:00 |
| IMP relative time (theoretical) (b) | T0 | T0.5 | T1 | T1.5 | T2 | T2.5 | T3.5 | T4.5 | T6.5 |
| Institutionalization (c) | ←-------------------------------------------------------------------------------------------------- | | | | | | | | |
| Discharge (d) | | | | | | | | | X |
| Inclusion/exclusion criteria | X (k) | | | | | | | | |
| Physical examination | X (k) | | | | | | | | X |
| Concomitant medication | ←-------------------------------------------------------------------------------------------------- | | | | | | | | |
| Body weight | X (k) | | | | | | | | |
| Urine Drug Screen (e) | X (k) | | | | | | | | |
| Alcohol Test | X (k) | | | | | | | | |
| Anti-lixisenatide antibodies (f) | X (k) | | | | | | | | |
| Randomization (g) | X (k) | | | | | | | | |
| IXRS call (h) | X (k) | | | | | | | | |
| Meals | | BR(i) | | | | | | LU | |
| Investigational Medicinal Product s.c. Administration | X | | | | | | | | |
| **SAFETY** | | | | | | | | | |
| Blood Pressure/Heart Rate | X (k) | | | | X | | | | X |
| Body temperature | X (k) | | | | | | | | |
| 12-lead ECG (j) | X (k) | | | | | | | | X |
| Blood Laboratory | | | | | | | | | |
| Urinalysis | | | | | | | | | |
| Urinary pregnancy test (l) | X (k) | | | | | | | | |
| AE/SAE collection | ←-------------------------------------------------------------------------------------------------- | | | | | | | | |

| Day per period | D1 – Period 1 | | | | | | | | |
|---|---|---|---|---|---|---|---|---|---|
| Indicative clock time (a) | 07:30 (a) | 08:00 | 08:30 | 09:00 | 09:30 | 10:00 | 11:00 | 12:00 | 14:00 |
| IMP relative time (theoretical) (b) | T0 | T0.5 | T1 | T1.5 | T2 | T2.5 | T3.5 | T4.5 | T6.5 |
| **PHARMACOCINETICS (PK)** | | | | | | | | | |
| PK samples | X (k) | X | X | X | | X | X | X | X |
| **PHARMACODYNAMICS (m)** | | | | | | | | | |
| Plasma glucose | X (k) | X | X | X | X | X | X | X | |
| Insulin, C-peptide and glucagon | X (k) | X | X | X | | X | X | X | |

a   Indicative clock times are approximate times used to provide a clear understanding regarding the timing for dosing, procedures and assessments. The first tests and examination can start between 07:30 and 9:00 and the successive timepoints will be adjusted in the respect of the time intervals (ie, T0.5, T1...) per protocol.
b   Time (decimal hours) is expressed in reference to the IMP administration
c   Only a single visit (Day 1) scheduled per period including the tests and examination to be done before IMP OR at the convenience of patient and the possibilities of the investigational site, institutionalization on Day -1 afternoon/evening may be planned for starting all examinations and tests to be done before the IMP administration
d   Patient discharged with the Investigator's approval
e   Urine drug screen: amphetamines/metamphetamines, barbiturates, benzodiazepines, cannabinoids, cocaine, opiates
f   Blood sampling only
g   Randomization and allocation of the patient's treatment group using a centralized treatment allocation system
h   Allocation of the patient's treatment kit to be used and track of drug inventory
i   Standardized liquid meal
j   12- lead ECG will be recorded after at least 10 min in supine position. Automatic reading, digitalized for all timepoints, and in triplicate for baseline at T0
k   Prior to study drug administration
l   In females of reproductive potential (Tanner Stage ≥3)
m   All parameters are to measured in central Laboratory

| Day per period | D1 – Period 2 / 3 | | | | | | | | | |
|---|---|---|---|---|---|---|---|---|---|---|
| Indicative clock time (a) | 07:30 (a) | 08:00 | 08:30 | 09:00 | 09:30 | 10:00 | 11:00 | 12:00 | | 14:00 |
| IMP relative time (theoretical) (b) | T0 | T0.5 | T1 | T1.5 | T2 | T2.5 | T3.5 | T4.5 | | T6.5 |
| Institutionalization (c) | ←---------------------------------------------------------------------------------- | | | | | | | | | |
| Discharge (d) | | | | | | | | | | X |
| Inclusion/exclusion criteria | X (k) | | | | | | | | | |
| Physical examination | X (k) | | | | | | | | | X |
| Concomitant medication | ←---------------------------------------------------------------------------------- | | | | | | | | | |
| Body weight | X (k) | | | | | | | | | |
| Urine Drug Screen (e) | X (k) | | | | | | | | | |
| Alcohol Test | X (k) | | | | | | | | | |
| Randomization | | | | | | | | | | |
| IXRS call (f) | | | | | | | | | | X |
| Meals | | BR(g) | | | | | | | LU | |
| Investigational Product Administration | X | | | | | | | | | |
| **SAFETY** | | | | | | | | | | |
| Blood Pressure/Heart Rate | X (k) | | | | X | | | | | X |
| Body temperature | X (k) | | | | | | | | | |
| 12-lead ECG (h) | X (k) | | | | | | | | | X |
| Blood Laboratory | | | | | | | | | | |
| Urinalysis | | | | | | | | | | |
| Urinary pregnancy test (i) | X (k) | | | | | | | | | |
| AE/SAE collection | ←---------------------------------------------------------------------------------- | | | | | | | | | |

| Day per period | D1 – Period 2 / 3 | | | | | | | | |
|---|---|---|---|---|---|---|---|---|---|
| Indicative clock time (a) | 07:30 (a) | 08:00 | 08:30 | 09:00 | 09:30 | 10:00 | 11:00 | 12:00 | 14:00 |
| IMP relative time (theoretical) (b) | T0 | T0.5 | T1 | T1.5 | T2 | T2.5 | T3.5 | T4.5 | T6.5 |
| **PHARMACOCINETICS (PK)** | | | | | | | | | |
| PK samples | X (k) | X | X | X | | X | X | X | X |
| **PHARMACODYNAMICS (j)** | | | | | | | | | |
| Plasma glucose | X (k) | X | X | X | X | X | X | X | |
| Insulin, C-peptide and glucagon | X (k) | X | X | X | | X | X | X | |

a   Indicative clock times are approximate times used to provide a clear understanding regarding the timing for dosing, procedures and assessments. The first tests and examination can start between 07:30 and 9:00 and the successive timepoints will be adjusted in the respect of the time intervals (ie, T0.5, T1...) per protocol.

b   Time (decimal hours) is expressed in reference to the IMP administration

c   Only a single visit (Day 1) scheduled per period including the tests and examination to be done before IMP OR at the convenience of patient and the possibilities of the investigational site, institutionalization on Day -1 afternoon/evening may be planned for starting all examinations and tests to be done before the IMP administration

d   Patient discharged with the Investigator's approval

e   Urine drug screen: amphetamines/metamphetamines, barbiturates, benzodiazepines, cannabinoids, cocaine, opiates

f   Call to IXRS in Period 3 only (or earlier in case of drop-out)

g   Standardized liquid meal

h   12-lead ECG will be recorded after at least 10 min in supine position. Automatic reading, digitalized for all timepoints, and in triplicate for baseline at T0

i   In females of reproductive potential (Tanner Stage ≥3)

j   All PD parameters are to measured in the central Laboratory

k   Prior to study drug administration

**2 (void)**

**3 LIST OF ABBREVIATIONS**

Pharmacokinetic parameter definitions are provided in Section 9.3.5.

| | |
|---|---|
| AE: | Adverse event |
| ARAC: | Allergic Reaction Assessment Committee |
| ARAC: | Allergic Reaction Assessment Committee |
| BMI: | Body mass index |
| CRF: | Case Report Form |
| CV: | cardiovascular |
| ECG: | Electrocardiogram |
| FSH: | Follicle- stimulating hormone |
| GAD: | Glutamic acid decarboxylase |
| GLP-1: | Glucagon-like peptide-1 |
| IA2: | Insulinoma associated protein |
| IMP: | Investigational Medicinal Product |
| IXRS: | Interactive Voice and Web Response System |
| MTC: | Medullary thyroid cancer |
| PPG: | Postprandal glucose |
| SD: | standard deviation |
| SEM: | standard error of the mean |
| SU: | Sulfonylurea |
| T2DM: | Type 2 diabetes mellitus |

**4 INTRODUCTION AND RATIONALE**

**4.1 INTRODUCTION**

Lixisenatide is an exendin analog with agonistic activity on Glucagon-like peptide-1(GLP-1) receptor. The principal therapeutic potential of lixisenatide to lower blood glucose in T2DM patients has been established in clinical studies. Sanofi-aventis is initiating global registration submissions including a Marketing Authorization Application (MAA) submission by Centralized Procedure in the European Union. A total of 42 clinical studies were conducted or are ongoing, including 24 Phase 1 studies, 5 Phase 2 studies and 13 Phase 3 studies.

A large Phase 3 program (the "GetGoal" clinical trial program) conducted throughout 48 countries and approximately 900 sites have been initiated to assess the safety and efficacy of lixisenatide. The GetGoal program has enrolled more than 4500 adult patients with T2DM (more than 2700 of them receiving lixisenatide). This program includes 10 studies with a duration ranging from 12 to more than 76 weeks. In addition to the GetGoal program, one Phase 3b study has been completed and a second Phase 3b study and a large placebo-controlled study to evaluate cardiovascular outcomes during treatment with lixisenatide are ongoing.

In the phase 3 studies that have been completed and analyzed so far [AVE0010 Clinical Investigator's Brochure, latest version]:

•  The efficacy of lixisenatide on glycemic control was confirmed

•  Lixisenatide was safe and well tolerated:

  -  As expected for a GLP-1 receptor agonist the most frequent adverse events were gastrointestinal in nature, mainly nausea, with low rates of vomiting and diarrhea. Most of these events were transient, mild to moderate in intensity and resolved spontaneously without sequelae.

  -  Reported hypoglycemia events were mostly mild to moderate in intensity. The incidence was similar to placebo when lixisenatide was used in monotherapy. In the EFC10887 study, in which 70% of the patients were receiving a background treatment with basal insulin in combination with a sulfonylurea (SU), the percentage of patients with symptomatic hypoglycemia was higher with lixisenatide (42.9%) versus placebo (23.6%). However, in the subgroup of patients not treated with a SU, the incidence of patients with symptomatic hypoglycemia was similar in the placebo and lixisenatide groups (32.6% with lixisenatide and 28.3% with placebo).

- In a comparative study versus exenatide (10 $\mu$g twice daily), significantly fewer patients treated with lixisenatide 20 $\mu$g once daily experienced symptomatic hypoglycemia events (5.0% in the lixisenatide arm vs 14.6% in the exenatide arm). Lixisenatide also offered better gastrointestinal tolerability with fewer patients experiencing nausea or vomiting.

There have been no paediatric clinical trials conducted as of today with lixisenatide.

More detailed information is provided in the Clinical Investigator's Brochure (1).

## 4.2 RATIONALE

### 4.2.1 Study rationale

Until recently, T2DM was almost exclusively an adult disease. Coinciding with the increasing prevalence of obesity in children, the incidence of T2DM in children and adolescents has markedly increased to the point that it accounts for as many as one third of all the new cases of T2DM diagnosed in adolescent.

Children/adolescents with T2DM are usually diagnosed over the age of 10 years, in middle to late puberty, when due to physiological changes in the GH/IGF-1 axis insulin resistance develops. Like in adults, the incidence of type 2 diabetes in children/adolescents is highest in some ethnic populations (e.g. American Indians, African American, Asian/pacific Islander and Hispanics) (2).

Diabetes is a therapeutic area for which the EMA Paediatric Working Party considers that research and development of medicinal products for children should be performed. Type 2 diabetes may have an earlier and more aggressive course in paediatric patients; therefore, they are likely to be at higher risk for developing complications and need the best possible glycemic control in the early stage of their disease.

As of today, metformin is commonly selected as the first pharmacotherapy in managing T2DM in children above 10 years and in adolescent in addition to diet and exercise (5, 6, 7). This drug has indeed shown to be safe and effective in randomized controlled trials carried out in this population (8) Nevertheless, in many patients, progression is rapid, and control of hyperglycaemia may become insufficient even at maximal tolerated doses of metformin.

Therefore, we propose to evaluate pharmacokinetics, pharmacodynamics and safety / efficacy of lixisenatide in a paediatric population.

### 4.2.2 Population to be studied

The population to be studied comprises patients with diabetes mellitus type 2 on diet and exercise, with or without a stable treatment of metformin, with an age of 10 to 17 years for the paediatric population and 18 to 65 years for the adult population. BMI will be either > 25 kg/m$^2$ (adults) or BMI > 85th percentile for age and gender (paediatric population).

### 4.2.3 Design rationale and risk assessment

The cross-over, blinded and randomized design allows enhancing the sensitivity to assess true effects by analyzing for differences between lixisenatide and placebo within each participant, while avoiding influence of between patient variability.

Subjects with diabetes mellitus type 2 with a background metformin therapy (with stable dose $\pm$ 10 % for at least 4 weeks prior to randomization) can be included and their metformin therapy will not be changed throughout the study. As insulin releasing treatments (e.g. sulfonylureas) require long wash out periods, subjects on insulin secretagogues will not be asked to participate.

A dose of 10 $\mu$g results in mean peak plasma concentrations of about 50 pg/mL about 2 hours after injection (1).

In a phase I study, single doses of lixisenatide from 3 $\mu$g lower PPG in T2DM patients but at least 10 $\mu$g lixisenatide caused a clear attenuation of the rise in plasma glucose induced by a standardized liquid meal administered 1 hour after dosing (Study AVE0010A/01-016, see details in the Clinical Investigator's brochure (1)).

The elimination half life for lixisenatide administered to adult T2DM patients is around 3 to 4 hours. The quick disappearance of lixisenatide from the circulation when absorption is complete enables short wash-out periods of 1 day. As a consequence the end-of-study visit can occur within a week.

Lixisenatide has been studied in subjects with type 2 diabetes mellitus, and has a record of safety and tolerability which allow further single dose experiments. The most common adverse effects upon single dose administration in patients with type 2 diabetes mellitus were headache, nausea, and injection site reactions. As GLP-1 mediated insulin release is depending on plasma glucose concentration, and decreases to absence with lower glucose concentration, the risk for hypoglycemia is very limited.

Hospitalization and close supervision of participants by professional staff members in the research unit on Day 1 ensure maximum protection against consequences of unforeseen adverse events.

### 4.2.4 Dose, Regimen, and Treatment Duration Rationale

The maximum dose evaluated in the ongoing phase III program is 20 μg QD with a preceding starting dose of 10 μg. In this planned study (PKD11475) the dose of 5 μg corresponds to 50 % of the starting dose in adults. Randomization will assure that in any patient the first lixisenatide treatment will be at a dose level of 5 μg.

Since pharmacodynamic effects of lixisenatide such as lowering of PPG after a test meal could be demonstrated even after the very first dose, a single-dose study is considered appropriate to compare pharmacodynamic effects between adult and paediatric populations.

### 4.2.5 Specific parameters rationale

#### *4.2.5.1 Postprandial plasma glucose after a standardized breakfast*

Lixisenatide is known to exert glucoregulatory effects, including enhancement of glucose-dependent insulin secretion, reduction of glucagon secretion, reduction of food intake, and slowing of gastric emptying. After a meal, the gastrointestinal tract regulates the rate at which carbohydrate and nutrients are absorbed and it is known to release regulatory peptides that stimulate insulin secretion from pancreas. Although the rate of gastric emptying does not affect insulin secretion directly, it regulates the delivery of nutrients to the small intestine and, therefore, has a major impact on the timing and magnitude of the blood glucose excursion, thereby modulating insulin secretion indirectly.

Therefore, beside the assessment of postprandial plasma glucose (primary endpoint) after a standardized meal, study objectives include the evaluation of the effects of lixisenatide on secretion of insulin, glucagon and C-peptide.

#### *4.2.5.2 Specific safety parameters*

Amylase and lipase: Because some cases of acute pancreatitis have been reported with marketed GLP-1 agonists (7), patients with amylase and lipase above 3 times the upper limit of normal at screening will be excluded from the study, and amylase and lipase will be monitored.

## 5 STUDY OBJECTIVES

### 5.1 PRIMARY

- To investigate the effects of a single subcutaneous lixisenatide dose of 5 μg and 10 μg as compared to placebo in reducing postprandial glucose (PPG) assessed as area under the plasma glucose concentration curve (AUC) after a standardized liquid meal (breakfast) in type 2 diabetic paediatric population (10-17 years old) and adults as controls.

### 5.2 SECONDARY

To evaluate in both paediatric and adult populations:

- Pharmacokinetic parameters of lixisenatide in plasma after single subcutaneous ascending doses

- The maximum PPG excursion, and on the changes in insulin, pro-insulin, C-peptide and glucagon plasma concentrations following a standardized breakfast

- Safety and tolerability.

## 6 STUDY DESIGN

### 6.1 DESCRIPTION OF THE PROTOCOL

Graphical study design/flow charts - please refer to flow charts in Section 1.

This is a phase I, multicenter, double-blind, randomized, placebo controlled, single-dose, 3-period, 3-treatment, 6 sequence cross-over study in paediatric and adult with type 2 diabetic patients.

The study is double blind with regard to active treatment versus placebo. The study drug volume (i.e., dose of active drug at 5 μg and 10 μg or matching placebo) is not blinded.

There are 3 treatment periods 1-7 days apart, each period lasting only one day (Day 1) with an early start in the morning up to the beginning of the afternoon. However, according to the possibilities of the investigational site and at the convenience of patient, the period may start on Day -1 (afternoon/evening) for all examinations and tests to be done before the IMP administration and before the standardized breakfast (liquid meal). **After the dinner on Day-1, patients should stay in fasted conditions for at least 8 hours (food and drink are not allowed except water) up to the standardized breakfast test meal.**

In each treatment period, the patients will receive a subcutaneously injected single dose of either 5 μg or 10 μg lixisenatide with 5 μg preceding the 10 μg dose level or volume matched placebo (50 μL or 100 μL) before a standardized test meal.

There are 6 sequences with 3 treatment periods, lixisenatide 5 μg, lixisenatide 10 μg, placebo-controlled group (volume of 50 μL or 100 μL) as described in Table 1

Table 1 - Randomization schedule per study population (children/adults)

| Sequence | Number of patients | Period 1 Treatment | Period 2 Treatment | Period 3 Treatment |
|---|---|---|---|---|
| 1 | 2 | Lixisenatide 5 μg (50 μL) i.e. 5 Units* | Lixisenatide 10 μg (100 μL) i.e. 10 Units | Placebo (100 μL) i.e. 10 Units* |
| 2 | 2 | Lixisenatide 5 μg (50 μL) i.e. 5 Units* | Lixisenatide 10 μg (100 μL) i.e. 10 Units | Placebo (50 μL) i.e. 5 Units* |
| 3 | 2 | Placebo (50 μL) i.e. 5 Units* | Lixisenatide 5 μg (50 μL) i.e. 5 Units* | Lixisenatide 10 μg (100 μL) i.e. 10 Units* |
| 4 | 2 | Placebo (100 μL) i.e. 10 Units* | Lixisenatide 5 μg (50 μL) i.e. 5 Units* | Lixisenatide 10 μg (100 μL) i.e. 10 Units* |
| 5 | 2 | Lixisenatide 5 μg (50 μL) i.e. 5 Units* | Placebo (50 μL) i.e. 5 Units* | Lixisenatide 10 μg (100 μL) i.e. 10 Units |
| 6 | 2 | Lixisenatide 5 μg (50 μL) i.e. 5 Units* | Placebo (100 μL) i.e. 10 Units* | Lixisenatide 10 μg (100 μL) i.e. 10 Units* |

* Number of Units indicated in the Opticlick® device

### 6.1.1 Screening period

Overall, the screening period can start on Day -30 up Day -2 before the start of the treatment period. However, laboratory blood sampling must be performed from Day -30 to Day -25 to get the results (ie, Anti-GAD and anti-IA2 antibodies) before Day 1.

Patients with type 2 diabetes are screened firstly from Day -30 up to Day -2 (screening phase) and those meeting all inclusion criteria are candidates for a final selection on Day 1 (or Day -1) at the study site (inclusion phase). Patients to be enrolled in this study are patients with type 2 diabetes diagnosed at least 1 year for adults and at least 3 months for paediatric population before the time of screening visit, and patients who are not treated with antihyperglycaemic medicinal product other than metformin with a stable dose(±10%) for at least 4 weeks prior to randomization (Day 1) (see Section 7.2).

The first measurements of HbA1c, plasma glucose and C-peptide must be obtained for checking the inclusion criteria of the patients.

Patients meeting all the inclusion criteria and presenting no exclusion criteria are eligible for the treatment period starting on Day 1.

### 6.1.2 Treatment period

At the convenience of patients and according to the possibilities of the investigational centre, patient may be institutionalized in the afternoon or evening on Day -1 up to the beginning of the afternoon on Day 1.

After the dinner on Day-1 of each treatment period, patients must fast for at least 8 hours (food and drink are not allowed except water) prior to the IMP administration on Day 1 of each treatment period.

On Day 1 of the treatment period 1, patient will undergo the final inclusion examinations and baseline safety assessments will be evaluated prior to the first IMP administration and before the standardized breakfast test meal.

On Day 1 of each Period 1, patients will be randomly assigned to one of the six treatment sequences consisting of a 3-period cross-over, separated by washout periods of at least one day up to 7 days between each period (see details Section 6.1.1).

**An 'Independent Person' will be assigned at the investigational centre solely for the administration of IMP, to prevent un-blinding of the clinical team during the conduct of the study.** However, activities which are not prone to any bias should be allowed, e.g. data entry of forms filled in by the clinical team, checking position of ECG electrodes etc.

One single SC dose of the following treatment will be administered at each period of treatment

- One 5 $\mu$g dose of lixisenatide (50 $\mu$E, 5 Units indicated on the Opticlick®)

- One 10 $\mu$g dose of lixisenatide (100 $\mu$E, 10 Units indicated on the Opticlick®)

- 50 $\mu$L placebo solution (5 Units indicated on the Opticlick®) or 100 $\mu$L placebo solution (10 Units indicated on the Opticlick®)

At each treatment period, patients will undergo an 8-point plasma glucose profile, a 7-point profile of insulin, C-peptide and glucagon, and a 8-point pharmacokinetic profile up to 6.5h after the IMP administration as well as safety assessments before leaving the CRO or investigational centre.

### 6.1.3 End-of-study visit

The end of study visit should be scheduled on D2 to D7 of the Period 3.

Concerning the procedure to be followed in case of premature permanent discontinuation of treatment with investigational product, please refer to Section 11 .

### 6.2 DURATION OF STUDY PARTICIPATION

### 6.2.1 Duration of study participation for each patient

- Screening duration: 25 (blood sampling for autoantibodies testing) to 30 days

- 3 treatment period(s): only 1 day (1 treatment day) to 2 days (if patient will arrive on Day -1) each

- Wash-out period between each period: at least 1 day up to 7 days

- End of study: 1 to 6 days after last dosing (D2 to D7 after Period 3)

- Total study duration from screening per patient: 4 to 7 weeks at maximum

However, patient participation could be prolonged in case of safety concerns (see Section10.3.3.1).

### 6.2.2 Determination of end of clinical trial (all patients)

The end of the clinical trial is defined as the day the last patient completed his/her last visit planned in the protocol.

## 6.3 STUDY COMMITTEES

### 6.3.1 Allergic Reaction Assessment Committee

Since lixisenatide is a peptide that may potentially generate allergic reactions, an Allergic Reaction Assessment Committee (ARAC) has been set up. The ARAC is a committee of experts in the field of allergy, independent from the Sponsor and the investigators, implemented to assess allergic reactions or allergic-like reactions that may occur during the study. The mission of the ARAC is to adjudicate, in a timely manner, all allergic, or possible allergic events. The ARAC is blinded regarding the study treatment.

Sometimes transient, injection site reactions, irritant in nature may occur, requiring no intervention and being of dubious significance. These reactions would not be considered to be allergic reactions.

Virtually all symptoms listed on the CRF "Allergic Reaction Complementary Form" are possible adverse reactions that may be allergic in nature and may need to be addressed after medical judgment, excluding another etiology than allergy.

Adverse events that may constitute an allergic reaction (e.g., generalized itch, nasal itch, swelling at injection site, flushing, hives, swelling at lips, eyes, face, tongue, hands, feet, lump in throat, difficulty to swallow, hoarseness, change in pitch of voice, incapacity to speak, wheezing, chest tightness, stridor, etc) should be considered to be reported on the Allergic Reaction Complementary Form.

Adverse events that are obviously not of allergic origin (e.g., local injection site reactions) should not be recorded on the Allergic Reaction Complementary Form.

The ARAC reviews the reported cases and determines the nature of the events, confirms the allergic nature or alternative diagnosis based on the information reported by the investigator. A detailed charter describes the ARAC procedures.

## 7 SELECTION OF PATIENTS

### 7.1 NUMBER OF PATIENTS PLANNED

Twelve (12) paediatric patients and 12 adult diabetic patients are to be enrolled for final Pharmacodynamics evaluation.

### 7.2 INCLUSION CRITERIA

Patients meeting all of the following criteria will be considered for enrollment into the study:

### Demography

I01. Age eligibility for paediatric population: $\geq$ 10 years and <18 years with at least 3 patients below 15 years and no more than 3 patients aged between 16 and 18 years (see below in Table 2)

**Table 2 - Subset definition for paediatric population**

| Age range (years) | Number of paediatric patients (n=12) |
| --- | --- |
| Age range $\geq$ 10 and <15 | 3 to 10 |
| Age range $\geq$ 15 and < 16 | 1 to 8 |
| Age range $\geq$ 16 and <18 | 1 to 3 |

Age eligibility for adults: >18 and $\leq$ 65 years
I02. BMI > 85th percentile for age and gender in children, body weight > 50 kg (Appendix A); BMI > 25 kg/m$^2$ and $\leq$ 37 kg/m$^2$ for adults

### Health status

I03. Male and female patients with type 2 diabetes mellitus, as defined by WHO (fasting plasma glucose $\geq$7 mmol/l

(126 mg/dl) or 2 hours postprandial plasma glucose ≥11.1 mmol/l (200 mg/dl)), diagnosed for at least 1 year for adults and at least 3 months for paediatric population at the time of screening visit, with or without metformin (stable dose ±10% for at least 4 weeks prior to randomization)

I 04. HbA1c ≥ 7% and ≤ 10% at screening

I 05. Fasting C-peptide at screening > 0.6 ng/mL

I 06. Negative test for anti-insulinoma associated protein (IA2) and anti-glutamic acid decarboxylase (GAD) autoantibodies

I 07. Menstruating females must have a negative pregnancy (serum beta HCG) test for inclusion (Tanner Stage ≥ 3)

I 08. Women of childbearing potential (including sexual active girls) must use a double contraceptive method throughout the study as judged by the investigator, except if she has undergone sterilization at least 3 months prior to the time of screening or is postmenopausal. The accepted double contraception methods include use of a highly effective method of birth control (intrauterine device or hormonal contraception) in addition to one of the following contraceptive options: (1) condom; (2) diaphragm or cervical/vault cap; (3) spermicide (CPMP/ICH/286/95, modification) Note: Menopause is defined as being over 60 years of age, or between 45 and 60 years of age and being amenorrheic for at least 2 years with plasma FSH level > 30 UI/L.

**Regulations**

109. Adult patient having given written informed consent prior to undertaking any study-related procedure and for minor's, provision of Informed Consent Form signed by the patient's parent(s)/legal representative. In addition, provision of Assent Form signed by minor patient or Informed Consent Form signed by emancipated or mature minors (defined by local lows)

I 10. Covered by a health insurance system where applicable, and/or in compliance with the recommendations of the national laws in force relating to biomedical research, (to be adapted if needed, country specific)

I 11. Not under any administrative or legal supervision.

**7.3 EXCLUSION CRITERIA**

**7.3.1 Exclusion criteria related to study methodology**

E 01. If female, pregnancy (defined as positive urinary pregnancy test), breast-feeding

E 02. Diabetes other than type 2 diabetes

E 03. History of metabolic acidosis, including diabetic ketoacidosis within 1 year prior to screening

E 04. Hemoglobinopathy or hemolytic anemia

E 05. History of myocardial infarction, stroke, or heart failure requiring hospitalization within 6 months prior to the time of screening, history or presence of clinically significant diabetic retinopathy, history or presence of macular edema likely to require laser treatment within the study period

E 06. Cardiovascular, hepatic, neurological, endocrine disease, active malignant tumor or other major systemic disease or patients with short life expectancy making implementation of the protocol or interpretation of the study results difficult (euthyroid patients on replacement therapy will be included if the dosage of thyroxin is stable for at least three months prior to screening Visit)

E 07. For adults, uncontrolled or inadequately controlled hypertension at the time of screening with a resting systolic or diastolic blood pressure > 160 mmHg or > 95 mmHg, respectively

E 08. For children, abnormal blood pressure levels greater or equal to 90th percentile adjusted for age, gender and height percentile (Appendix B)

E 09. Positive test for insulinoma associated protein (IA2) and glutamic acid decarboxylase (GAD) autoantibodies

E 10. Any clinically significant abnormality identified on physical examination, laboratory tests or vital signs at the time of screening that in the judgment of the investigator or any sub investigator would preclude safe completion of the study

E 11. Receipt of blood or plasma products within 3 months prior to the time of screening

E 12. Investigator or any sub investigator, pharmacist, study coordinator, other study staff or relative thereof directly involved in the conduct of the protocol

E 13. Patients considered by the investigator or any sub investigator as inappropriate for this study for any reason (e.g. impossibility to meet specific protocol requirements, such as scheduled visits, being able to do self-injections, etc)

E 14. Use of other oral or injectable antidiabetic or hypoglycemic agents other than metformin (e.g., alpha glucosidase inhibitor, exenatide, DPP-IV inhibitors, insulin etc.) within 3 months prior to the time of screening

E 15. Use of systemic glucocorticoids (excluding topical application or inhaled forms) for one week or more within 3 months prior to the time of screening

E 16. For children, known allergy to local anesthetics (e.g., Emla® , Elamax® cream, Ethyl Chloride)

E 17. Likelihood of requiring treatment during the screening phase and treatment phase with drugs not permitted by the clinical study protocol

E 18. Use of any investigational drug within 3 months prior to screening

**7.3.2 Exclusion criteria related to the current knowledge of lixisenatide and/or metformin**

**Exclusion criteria related to lixisenatide:**

E 19. Clinically relevant history of gastrointestinal disease associated with prolonged nausea and vomiting, including, but not limited to gastroparesis and gastroesophageal reflux disease requiring medical treatment, within 6 months prior to the time of screening

E 20. Any previous treatment with lixisenatide

E 21. Allergic reaction to any GLP-1 agonist in the past (e.g. exenatide, liraglutide) or to metacresol

E 22. History of unexplained pancreatitis, chronic pancreatitis, pancreatectomy, stomach/gastric surgery, inflammatory bowel disease

E 23. Personal or family history of medullary thyroid cancer (MTC) or genetic conditions that predispose to MTC (e.g., multiple endocrine neoplasia syndromes)

E 24. Known history of drug or alcohol abuse within 6 months prior to the time of screening

E 25. Laboratory findings at the time of screening:

- In adults

  - ALT > 3 times the upper limit of the normal laboratory range

  - Total bilirubin: > 1.5 times the upper limit of the normal laboratory range (except in case of Gilbert's syndrome)

  - Hemoglobin < 11 g/dL and/or neutrophils < 1,500/mm3 and/or platelets < 100,000/mm3

- In paediatrics:

  - Elevations in blood tests of renal (serum creatinine > 1.0 mg/dL) and/or liver (ALT, AST and/or bilirubin) >2 times the upper limit of normal (ULN) for age.

  - Hemoglobin < 11 g/dL and/or neutrophils < 1,500/mm3 and/or platelets < 100,000/mm3

- In adults/paediatrics:

  - Calcitonin $\geq$ 20 pg/mL

  - Amylase and/or lipase above 3 times the upper limit

  - Positive result on any of the following tests: hepatitis B surface (HBs Ag) antigen, anti-hepatitis C virus (anti-HCV) antibodies, anti-human immunodeficiency virus 1 and 2 antibodies (anti-HIV1 and anti HIV2 Ab).

E 26. Positive alcohol test.

**Exclusion criteria related to the background therapy (i.e. metformin):**

E 27. Renal impairment in adult defined with creatinine clearance < 60 mL/min using the Cockcroft- Gault Formula (see Appendix C)

A patient may not be enrolled in this study more than once (i.e. randomized twice).

## 8 TREATMENTS

### 8.1 DIET AND EXERCISE

Lifestyle and diet therapy provided before the time of screening is to be continued during the study in a similar manner. Dietary and lifestyle counseling should be given by a Registered Dietitian or other qualified nutrition professional (eg, diabetic educator, etc) and should be consistent with the recommendations of international or local guidelines (with regard to the distribution of calories among carbohydrates, proteins, and fats, exercises, etc) for type 2 diabetic patients.

At each of 3 treatment periods, adult and paediatric patients will ingest a standardized meal test, 30 min. after the IMP administration to assess fasting and post-prandial glucose.

For adults and paediatrics, the standardized breakfast meal is a 400 mL drink (Ensure Plus® Drink, Abbott). It contains 600 kcal and is composed of 53.8% carbohydrate, 16.7% protein and 29.5% fat (see details in Appendix D).

The composition and the quantity of the standardized meal must be identical at each treatment period.

The standardized meal for all adult and paediatric patients should be consumed within a 15-minute period.

### 8.2 INVESTIGATIONAL MEDICINAL PRODUCT

#### 8.2.1 Lixisenatide/placebo

- Lixisenatide pharmaceutical form: Sterile aqueous solution for subcutaneous (s.c.) injection in a 3-mL glass cartridge, containing the active ingredient 300 $\mu$g (i.e. 100 $\mu$g/mL), glycerol, sodium acetate trihydrate, methionine, meta-cresol, HCL/NaOH and water for injection.

- Control drug: matching placebo, aqueous solution for subcutaneous injection.

- Route and method of administration: Subcutaneous injection using the pen-type injector (OptiClik®). Lixisenatide injection will be performed in the clinical unit by a person experienced with s.c. Administration will be by deep s.c., alternating between the left and right anterolateral and the left/right posterolateral abdominal walls. Within a given area, location should be changed (rotated) at each time to prevent injection site skin reaction.

- Dose of the lixisenatide investigational medicinal product (IMP) per administration: Once injection in the morning of Day 1 of each period

- Timing: lixisenatide will be administered at around 07:30 in the morning in fasted condition (breakfast will be taken 30 minutes after the injection)

- For the correct dosing of Lixisenatide and placebo volume, the units of the OptiClik® pen have to be settled as described in Table 1.

- Lixisenatide IMP will be provided by the sponsor.

### 8.2.2 Description of the injector device OptiClik®

A pen-type injector (OptiClik®) with Optifine 8TM (8 mm x31G) needles from Ypsomed are provided to each investigational centre for the injection of lixisenatide or its placebo, specifically labeled for the use of the study ("lixisenatide") in accordance with applicable regulatory requirements. Handling procedure of the pen-type injector and administration technique of lixisenatide is provided in a specific manual.

Pen-device or cartridges related issues (malfunctions) should be reported to the sponsor or the Wharehouse by the means of a procedure on product technical complaint (PTC) forms, which is described in a separate manual.

### 8.2.3 Dosage schedule

According to the randomization schedule (Section 6.1), the lixisenatide dose per injection or the placebo volume (Day 1) is to be administered 30 minutes before breakfast and for the correct dosing, the units of the OptiClik® pen will be administered as follows:

- 5 $\mu$g lixisenatide = 05 Units indicated on OptiClik® (= 50 $\mu$L)

- 10 $\mu$g lixisenatide = 10 Units indicated on OptiClik® (= 100 $\mu$L)

- 50 $\mu$L (0.05 mL) placebo = 05 Units indicated on OptiClik®

- 100 $\mu$L (0.10 mL) placebo = 10 Units indicated on OptiClik®

### 8.3 NON INVESTIGATIONAL MEDICINAL PRODUCTS

The possible background therapy (ie, metformin only) is not considered as non investigational medicinal product.

### 8.4 DESCRIPTION OF BLINDING METHODS

The lixisenatide investigational product and placebo are indistinguishable.

The treatment allocation (on Day 1 of each period) will be double-blinded and will be done according to a randomization list. The treatment codes will be generated according to sanofi-aventis procedure.

For blinding purposes the on-site administration of lixisenatide or its placebo will therefore be performed by an independent person who is not a member of the clinical study team at the CRO or investigational site. This "unblinded" person should not be involved in activities which could be biased by the knowledge of the treatment assignment (e.g. AE assessments, access to pharmacodynamic data). However, activities which are not prone to any bias should be allowed, e.g. blood sampling, vital signs, ECG recording, etc.).

OptiClik® pens will be loaded with either lixisenatide or placebo containing cartridges on-site. Furthermore lixisenatide or its placebo will be administered at different doses resulting also in different volumes. The volume to be injected (see Section 8.2.3) must be set on the OptiClik® pen and is visible to the "unblinded" person responsible for the injection. The "unblinded" person responsible for administration will set the volume to be injected on the OptiClik® pen shortly before injection.

The ARAC members will review and adjudicate allergic reactions or allergic-like reactions in a blinded manner.

Samples collected during the lixisenatide treatment periods only will be analyzed for plasma concentrations. Therefore the bioanalyst(s) at sanofi-aventis responsible for the determination of lixisenatide plasma concentrations will be unblinded to the randomization code. The results of these assessments will not be provided to the study personnel when the study is ongoing except for urgent safety issues.

## 8.5 METHOD OF ASSIGNING PATIENTS TO TREATMENT GROUP

At the screening visit, the procedure for assigning of patient number will start only after the provision of the written informed consent by the adult patients to be enrolled or the provision of Informed Consent Form signed by the patient's parent(s)/legal representative of the patient to be enrolled and also the provision of Assent Form or Informed Consent Form signed by the paediatric patient (see inclusion criteria I09).

Then, the investigator or designee has to contact the IXRS and has to provide some information to the system (e.g.: date of birth/age of the patient, background oral or injectable antidiabetic drugs other than metformin: yes/no, ..). The Interactive Voice and Web Response System (IXRS) will ensure that the enrolment for children will be controlled in respect of the obligations to recruit at least 3 paediatric patients between 10 and 15 years, at least 1 paediatric patient between 15 and 16 years, and no more than 3 paediatric patients above 16 years (inclusion criterion 101).

If criteria are in agreement with the above statement, IXRS will allocate an incremental patient number according to the chronological order of inclusion. The patient number will be a 9-digit patient number combined of 3 components (XXX-001-XXX), of which the first 3 digits are the country number (e.g.: for Germany: 276; Mexico: 484; South Africa: 710; UK: 826; US site: 840) the middle 3 digits are the site number (starting with 001) and the last 3 digits are the patient incremental number within the site. The patient number remains unchanged during the study and allows the patient to be identified during the whole study.

On Day 1 of Period 1, the investigator or the designee will contact IXRS and has to provide the following information to the system: patient number, age, and negative test for anti-GAD and anti-IA2 antibodies: yes/no. If the patient complies with all inclusion/exclusion criteria, this patient will be considered as randomized. A randomized patient is defined as a patient who is registered, who complies with all inclusion/exclusion criteria and assigned to his /her treatment kit number.

The randomization treatment kit number list is generated centrally by sanofi. The randomization list will be provided by sanofi to the IXRS vendor. The allocation of the treatment kit number to the patients will be performed by IXRS. Patients will receive IMP according to their randomization treatment kit number.

The randomization ratio will be 1:1 for the 2 lixisenatide dose levels (5 $\mu$g and 10 $\mu$g) and 1:1 for the placebo volumes (50 $\mu$L and 100 $\mu$L) and 2:1 for each lixisenatide dose versus each placebo volume.

The administration order of the 3 study drugs (5 $\mu$g lixisenatide, 10 $\mu$g lixisenatide, 100 $\mu$L placebo or 50 $\mu$L placebo) as defined by the randomization plan is defined using the centralized treatment allocation system (IXRS) on Day 1 of the first period after the safety assessments prior to the first IMP injection. The "independent person" (see Section 6.1.2), will administer the first study treatment on Day 1 at each study period in the respect of the written information received by IXRS. The CRO or the investigational site will call IXRS at the end of the last period.

Potential replacement patients will have a different identification number (ie, 500 + the number of replaced patients). Each patient will receive the same treatment sequence (the same order of the treatment as the withdrawn patient.

Notes: The randomization of a patient should occur as close as possible to the first administration of the IMP. Baseline parameters will be the parameters available the closest before the randomization.

## 8.6 PACKAGING AND LABELING

Cartridges (ie, disposable part of OptiClik®) will be used once at each treatment period and are packaged in multiple treatment boxes. Each Box per patient will contain 3 cartridges for injections (1 cartridge to be used per treatment period for a single administration).

A treatment box containing three cartridges is shown in Figure 2.

Dispensation scheme is described in the study flow chart (please refer to Section 1.3 and Section 1.4)

The content of the labeling is in accordance with the local regulatory specifications and requirements.

## 8.7 STORAGE CONDITIONS AND SHELF LIFE

All study drug boxes will be stored in an appropriate safe and locked room under the responsibility of the Investigator or other authorized persons (e.g., pharmacists), and must be accessible only to authorized personnel.

Prior to the first use, the investigational products (cartridges) have to be stored between +2°C and +8°C (between 36°F and 46°F), protected from light, and must not be frozen.

When used, the cartridges should be kept. At each treatment period on Day 1, a new cartridge should be replaced. One OptiClik® pen per patient will be used for the 3 single injections.

## 8.8 RANDOMIZATION CODE BREAKING DURING THE STUDY

Please refer to Section 9.5.

In case of an adverse event (AE), the code will not be broken except in the circumstances when knowledge of the IMP is essential for treating the patient. If possible, a contact should be initiated with the Sponsor's monitoring team or medical expert before breaking the code.

No code-breaking material is provided to the investigators. For each patient, code-breaking could be performed by the investigator calling the IXRS system.

The code-breaking material is also kept at the entity responsible for the "24 hour alert system"; but this system should be used in very exceptional cases only (i.e., unavailability of IXRS system or inability to contact investigator and/or site staff). The investigators will be informed by the sanofi-aventis clinical monitoring team about the availability of the local code-breaking material.

A patient card, including the relevant "24 hour alert system" telephone number will be provided to every patient who participates in the study.

If the blind is broken, the Investigator will document the date of opening and reason for code breaking in source data.

In case the blind code is broken, the treatment with the lixisenatide (or placebo) investigational product should be permanently discontinued, and the patient handled according to the procedure described in Section 11.4. The Investigator must document the date, time of day and reason for code breaking. In case of SAE, the instructions for SAE reporting are to be followed (please refer to Section 10.2.2).

## 8.9 RESPONSIBILITIES

The Investigator, the clinical site pharmacist, or other personnel allowed to store and dispense lixisenatide, its placebo and the injector pen Opticlick® (referred by Investigational medicinal product) will be responsible for ensuring that the IMP used in the clinical trial is securely maintained as specified by the Sponsor and in accordance with the applicable regulatory requirements.

All IMPs shall be dispensed in accordance with the Investigator's prescription and it is the Investigator's responsibility to ensure that an accurate record of IMP issued and returned is maintained.

Any quality issue noticed with the receipt or use of lixisenatide and its placebo provided by the sponsor (deficiency in condition, packaging, appearance, pertaining documentation, labeling, expiration date, etc.) or OptiClick® should be promptly notified to the Sponsor, who will initiate a complaint procedure.

A potential defect in the quality of IMP provided by the sponsor may prompt to initiation of a recall procedure by the Sponsor. In this case, the Investigator will be responsible for promptly addressing any request made by the Sponsor, in order to recall IMP and eliminate potential hazards.

Under no circumstances will the Investigator supply lixisenatide and its placebo provided by the Sponsor to a third party, allow the IMP provided by the Sponsor to be used other than as directed by this clinical trial protocol, or dispose of IMP provided by the Sponsor in any other manner.

## 8.10 CONCOMITANT TREATMENT

Specific treatments, which are ongoing before the study and/or prescribed or changed during the study, must be recorded in the CRF and Source Data (please refer to Section 12.2).

### 8.10.1 Concomitant Diabetes therapy

Patients may be enrolled with metformin background therapy at a stable dose ($\pm$10%) for at least 4 weeks prior to randomization). The metformin dose should be kept unchanged throughout the study. It should be administered according to the approved label.

### 8.10.2 Prohibited concomitant therapy

The following drugs (already listed as exclusion criteria, see Section 7.3) are not permitted during the study (up to the end-of study visit):

1. Any other any oral or injectable antidiabetic or hypoglycemic agents other than metformin (e.g., alpha glucosidase inhibitor, exenatide, DPP-IV inhibitors, insulin, TZD, SU etc.)

2. Systemic glucocorticoids (excluding topical application or inhaled forms) administered for one week or more should be discontinued within 3 months prior to the time of screening.

### 8.10.3 Permitted concomitant therapy

Any therapy other than the prohibited concomitant therapy described above, is allowed and has to be recorded in the source data (please refer to 12.2) and the e-CRF.

Note: *For oral treatments that are dependent on threshold concentrations for efficacy, such as contraceptives (pill) and antibiotics, patients should be advised to take those treatments at least 1 hour before study drug injection or about 11 hours after study drug injection.*

### 8.11 TREATMENT ACCOUNTABILITY AND COMPLIANCE

The independent person designed by the investigational site (see Section 8.4) will document dates, time and dose of each self injection of lixisenatide and placebo and the oral daily dose of metformin, if any and will complete the appropriate "Treatment Log Form".

The Monitoring Team in charge of the study then checks the CRF data by comparing them with the date and time of IMP.

### 8.12 RETURN AND/OR DESTRUCTION OF TREATMENTS

Investigational medicinal product reconciliation must be performed at the site or CRO by the Pharmacist or other personnel allowed and the monitoring team using treatment log forms and documented on center IMP inventory countersigned by the Pharmacist /Investigator and the monitoring team.

A written authorization for destruction will be given by the clinical trial team once the IMP reconciliation is achieved. This destruction can be performed at site depending on IMP specificities and local requirements or IMP can be returned to the Sponsor for destruction.

## 9 ASSESSMENT OF INVESTIGATIONAL MEDICINAL PRODUCT

### 9.1 PHARMACODYNAMICS

All pharmacodynamics parameters will be performed by a Central Laboratory. Detailed information on sample drawing, management and analysis will be provided.

- Plasma glucose concentrations

- Insulin, C-peptide and glucagon plasma concentrations

(see study and period flow charts for detailed assessment schedule)

### 9.1.1 Assessment methods

Plasma glucose, insulin, C-peptide and glucagon are to be sampled at pre-specified times and determined by specific validated assays. The exact time of sample collection must be recorded on the CRF. Special procedures for storage and shipping of pharmacodynamic samples will be described in a separate technical manual provided by the Central Laboratory.

### 9.1.2 Pharmacodynamic parameters

#### 9.1.2.1 Primary parameter(s)

- GLU- AUC$_{0:30\ -4:30}$ after each lixisenatide dose (5 $\mu$g, 10 $\mu$g) compared to placebo

GLV-AVC$_{0:30-4:30h}$: area under the plasma glucose concentration time profile from time of the standardized breakfast start (30 min after IMP injection and pre-meal plasma glucose =T0.5) until 4 hours later (T4.5) subtracting the pre-meal value. AUC will be calculated using the trapezoidal rule.

### 9.1.2.2 Secondary parameter(s)

- Post-prandial plasma glucose (PPG) excursion after each lixisenatide dose administration (5 $\mu$g, 10 $\mu$g) compared to placebo

PPG excursion will be calculated from the difference between the maximum after the standardized breakfast and before lunch minus the pre-meal plasma glucose (T0.5).

- AUC$_{0:30-4:30}$ of insulin, C-peptide and glucagon concentrations after each lixisenatide dose (5 $\mu$g, 10 $\mu$g) compared to placebo:

The area under the concentration time profile from time of standardized breakfast start (30 min after IMP injection and pre-meal plasma glucose =T0.5) until 4 hours later (T4.5). AUC will be calculated using the trapezoidal rule.

### 9.1.3 Assessment schedule

The assessment timing can be found in the period flow chart (please refer to Section 1.3 and Section 1.4

**Table 3 - Number of samples**

|  | Plasma Glucose | Insulin, C-peptide | Glucagon |
| --- | --- | --- | --- |
| By patient / Screening | 1 | 1[a] | 0 |
| By patient per Period | 8 | 7 | 7 |
| Total by patient | 25 | 22 | 21 |
| Total for study, n = 24 patients[b] | 600 | 528 | 504 |

[a] C-peptide only - [b] to be added replacement patients, if any

### 9.2 SAFETY

### 9.2.1 Baseline demographic characteristics:

Baseline demographic characteristics will consist of:

1. Age (years)

2. Height (cm)

3. Body mass index

4. Gender

5. Tanner staging (*screening only*)
The Tanner stages are stages (5 stages) of physical development in children, adolescents, and adults (9, (10). The stages define physical measurements of development based on external primary and secondary sex characteristics, such as the size of the breasts, genitalia, and development of pubic hair. Due to natural variation, individuals pass through the Tanner stages at different rates, depending in particular on the timing of puberty. The Tanner stages will be used to assist in defining females of childbearing potential during the screening physical examination.

6. Diabetes history including :

- Date of the diagnosis of type 2 diabetes

- If metformin co-administered, start date of treatment with metformin, daily dose of metformin at Baseline

### 9.2.2 Safety assessment at baseline and during the study

The tolerability investigations at baseline and during the study will consist of:

1. Physical examination (includes at a minimum: heart and respiratory auscultation; peripheral arterial pulse; pupil, knee, Achilles, and plantar reflexes; peripheral lymph nodes and abdomen examination).

2. Body weight (kg);

3. Body temperature (°C);

4. Vital signs (heart rate, systolic and diastolic blood pressure measured after 10 minutes in supine resting position);

5. Laboratory tests (in fasting conditions for blood samples):

- Hematology: red blood cell count, hematocrit, hemoglobin, white blood cell count with differential count (neutrophils, eosinophils, basophils, monocytes, and lymphocytes), platelets

- Biochemistry:

  - Plasma/serum electrolytes: sodium, potassium, chloride, calcium

  - Liver function: AST, ALT, alkaline phosphatase, gamma-glutamyl transferase, total and conjugated bilirubin

  - Renal function: urea, creatinine

  - Metabolism: glucose, albumin, total proteins, total cholesterol, triglycerides

  - Potential muscle toxicity: creatine phosphokinase

  - Pancreas: amylase and lipase

  - Calcitonin (tyroidc-cell tumor marker) at screening only

6. Serum $\beta$-HCG only at screening in females of reproductive potential (Tanner stage $\geq$ 3);

7. Urinary pregnancy test for menstruating females before each treatment period;

8. Plasma follicle-stimulating hormone (FSH), if applicable, at screening to confirm postmenopausal status;

9. Serology tests: hepatitis B antigen, hepatitis C antibodies, anti-HIV1 and anti-HIV2 antibodies;

10. At screening only: serum test for islet cell antibodies and glutamic acid decarboxylase (GAD) antibodies, fasting C-peptide and HbA1c;

11. Urinalysis: proteins, glucose, erythrocytes, leucocytes, ketone bodies, and pH.

- Qualitative: A dipstick is to be performed on a freshly voided specimen for qualitative detection using a reagent strip.

- Quantitative: A quantitative measurement for glucose, protein, erythrocytes, and leucocytes count will be required in the event that the urine sample test is positive for any of the above parameters by urine dipstick (eg, to confirm any positive dipstick parameter by a quantitative measurement).

12. Urine drug screen: amphetamines/methamphetamines, barbiturates, benzodiazepines, cannabinoids, cocaine, and opiates.

13. Alcohol breath test.

14. Adverse events, spontaneously reported by the patient or observed by the Investigator, will be monitored;

15. Standard 12-lead ECGs are recorded after at least 10 minutes in supine position using an electrocardiographic device. The electrodes will be positioned at the same place for each ECG recording throughout the study (attachment sites of the leads will be marked with an indelible pen).

Each ECG consists of a 10-second recording of the 12 leads simultaneously, leading to.

- A single 12-lead ECG (25 mm/s, 10mm/mV) printout with heart rate, PR, QRS, QT, QTc automatic correction evaluation (by the ECG device), including date, time, initials, and number of the patient, signature of the research physician, and at least 3 complexes for each lead. The Investigator's medical opinion and automatic values will be recorded in the e-CRF. This printout will be retained at the site.

Warning at each period: Whenever measurements of vital signs, ECG, and blood samples for pharmacokinetics, pharmacodynamics, or safety coincide, the following order will be respected: ECG, vital signs, pharmacodynamics, pharmacokinetics, and then safety samples. In order to respect exact timing of pharmacokinetics samples (refer to flow chart for time window allowance for pharmacodynamic and pharmacokinetics samples), the other measurements will be done ahead of the scheduled time. The assessment schedule should be adapted to the design of the study.

### 9.2.3 Anti-lixisenatide antibodies

Plasma samples from all patients will be collected to determine anti-lixisenatide antibodies on Day 1/ period 1 before the first study drug administration only. Procedures for collection, storage, and shipment will be provided in a separate manual.

**Table 4 - Number of plasma samples for anti-lixisenatide antibodies**

|  | Anti-lixisenatide antibodies |
| --- | --- |
| Total by patient (once D1/P1) | 1 |
| Total for patients (n=24) | 24 |

**Table 5 - Bioanalytical method**

| Analyte | Anti- Lixisenatide antibodies |
| --- | --- |
| Matrix | Plasma |
| Analytical Technique | BIAcore |
| Lower Limit of Quantification | cut-off |
| Assay Range | not relevant |
| Assay Volume | 100 μL |
| Site of Bioanalysis | Dept. of Disposition, Safety and Animal Research (DSAR), sanofi aventis, Frankfurt |
| Method Reference | RPSMPK-DOH0754-BM1-EN-E01 |

### 9.3 PHARMACOKINETICS

### 9.3.1 Sampling times

The sampling times for blood collection can be found in the period flow chart (please refer to Section 1.3 and Section 1.4).

### 9.3.2 Number of pharmacokinetic samples

**Table 6 - Number of plasma samples for AVE0010**

| | AVE0010 |
|---|---|
| By patient per period | 8 |
| Total by patient (x3 periods) | 24 |
| Total for patients n=24 (up to 36) | 24* 24= 576 (up to 864) |

### 9.3.3 Sample handling procedure for pharmacokinetic samples

Procedures for collection, storage, and shipment will be provided in a separate manual.

### 9.3.4 Bioanalytical methods

All lixisenatide plasma samples from patients having received lixisenatide were analyzed as described in Table 7, with a lower limit of quantification.

**Table 7- Summary of bioanalytical method**

| Analyte | Lixisenatide |
|---|---|
| Matrix | Plasma |
| Analytical technique | Double-antibody sandwich ELISA |
| Lower limit of quantification | 5.5 pg/mL |
| Assay volume | 120 $\mu$L |
| Site of bioanalysis | Biomarker/Biologicals, DSAR, sanofi aventis, Frankfurt |
| Method reference | DOH1154 |

### 9.3.5 Pharmacokinetic parameters

Lixisenatide plasma concentrations at predefined timepoints will be documented. The pharmacokinetic parameters will be calculated, using non-compartmental methods for lixisenatide plasma concentrations after single dose. The parameters will include, but may not be limited to the following.

**Table 8 - List of pharmacokinetic parameters and definitions**

| Parameters | Drug/Analyte | Matrix | Definition/Calculation |
|---|---|---|---|
| $C_{max}$ | AVE0010 | Plasma | Maximum plasma concentration observed |
| $t_{max}$ | AVE0010 | Plasma | Time to reach $C_{max}$ |
| $AUC_{last}$ | AVE0010 | Plasma | Area under the plasma concentration versus time curve calculated using the trapezoidal method from time zero to the real time |
| AUC | AVE0010 | Plasma | Area under the plasma concentration versus time curve extrapolated to infinity according to the following equation: $$AUC = AUC_{last} + \frac{C_{last}}{3_z}$$ ($C_{last}$ is the last quantifiable concentration, and $\lambda_z$ the rate constant of the terminal phase) Values with a percentage of extrapolation >20% will not be taken into account in the descriptive statistics |

### 9.4 SAMPLED BLOOD VOLUME

The approximate total sampled blood volume in children is 144 ml (approximate due to discarded blood when catheter is set up at each period). The amount of blood volume per visit will not exceed 46 mL (the highest at period 1). The approximate total sampled blood volume in adults is 144 mL (approximate due to discarded blood when catheter is set up at each period).

## 9.5 MEASURES TO PROTECT BLINDING OF THE TRIAL

For the purpose of IMP dispensing and administration and bioanalytical assessment of PK and anti-lixisenatide antibody samples, the following persons will be unblinded (refer to section 8.8 for the IMP dispensing procedure restricted to the independent on-site person of the CRO/investigational centre). A copy of the randomization list will be provided only to the bioanalyst responsible for lixisenatide concentration measurements.

In case of an adverse event, the Investigator should only break the code in exceptional circumstances when knowledge of the Investigational Product is essential for treating the patient (refer to section 8.8).

Nevertheless, for safety reason, the treatment code will be unblinded for reporting to the health authorities of any suspected unexpected serious adverse reaction (SUSAR), ie, any serious adverse event that is both unexpected (per the investigator's brochure) and reasonably associated with the use of the IMP according to either the judgment of the Investigator and/or the Sponsor.

The ARAC is blinded for the adjudication of allergic and allergic-like cases (please also refer to Section 6.3.1).

All persons at sanofi-aventis and at any CRO involved in the study including laboratory and eventually pharmacodynamic assessors will be blinded to the randomization code.

## 10 PATIENT SAFETY

The Investigator is the primary person responsible for taking all clinically relevant decisions on safety issues.

If judged necessary, the opinion of a Specialist should be envisaged in a timely manner (eg, acute renal failure, convulsions, skin rashes, angioedema, cardiac arrest, electrocardiographic modifications, etc).

In case of dermatologic lesions, the realization of photographs is strongly recommended in addition to quick Dermatologist advice.

### 10.1 ADVERSE EVENT MONITORING

All events will be managed and reported in compliance with all applicable regulations, and included in the final clinical study report.

### 10.2 DEFINITIONS OF ADVERSE EVENTS

#### 10.2.1 Adverse event

An **adverse event** (AE) is any untoward medical occurrence in a patient administered a pharmaceutical product and which does not necessarily have to have a causal relationship with this treatment.

- Mild = no modification of daily activities and does not require mandatory corrective/symptomatic treatment.

- Moderate = hinders normal daily activities and/or requires mandatory corrective/symptomatic treatment.

- Severe = prevents daily activities and requires mandatory corrective/symptomatic treatment.

#### 10.2.2 Serious adverse event

A **serious adverse event** (SAE) is any untoward medical occurrence that at any dose:

- Results in death, or

- Is life-threatening, or

Note: The term "life-threatening" in the definition of "serious" refers to an event in which the patient was at risk of death at the time of the event; it does not refer to an event which hypothetically might have caused death if it were more severe.

- Requires inpatient hospitalization or prolongation of existing hospitalization, or

- Results in persistent or significant disability/incapacity, or

- Is a congenital anomaly/birth defect, or

- Is a medically important event:

  - Medical and scientific judgment should be exercised in deciding whether expedited reporting is appropriate in other situations, such as important medical events that may not be immediately life-threatening or result in death or hospitalization but may jeopardize the patient or may require intervention to prevent one of the other outcomes listed in the definition above.

Note: Examples of such events are intensive treatment in an emergency room or at home for allergic bronchospasm, blood dyscrasias, convulsions, ALT > 3 x ULN + total bilirubin > 2 x ULN or asymptomatic ALT increase > 10 x ULN, or development of drug dependency or drug abuse.

Unblinding of SUSAR by the Sponsor is described in Section 9.5.

## 10.3 OBLIGATION OF THE INVESTIGATOR REGARDING SAFETY REPORTING

### 10.3.1 General guidelines for reporting adverse events

All AEs, regardless of seriousness or relationship to IMP, spanning from the signature of the informed consent form until the end of the study as defined by the protocol, are to be recorded on the corresponding page(s) or screen(s) of the case report form for included patients. For screen failed patients, recording in the case report form is only performed in case of SAE occurring during the screening period or in case of AE when some screening procedures expose the patient to safety risks (eg, any substance administered as pretreatment or for phenotyping, invasive tests performed or chronic treatment interrupted).

Whenever possible, diagnosis or single syndrome should be reported instead of symptoms. The Investigator should specify the date of onset, intensity (see definitions in Section 10.2.1), action taken with respect to IMP corrective treatment/therapy given, additional investigations performed (eg, in the case of dermatologic lesions photographs are required), outcome, and Investigator's opinion as to whether there is a reasonable possibility that the AE was caused by the IMP.

In order to ensure the safety of the patients, the Investigator should take appropriate measures to follow all AEs until clinical recovery is complete and laboratory results have returned to normal, or until progression has been stabilized, or until death. This may imply that observations will continue beyond the last planned visit per protocol, and that additional investigations may be requested by the monitoring team.

When treatment is prematurely discontinued, the patient's observations will continue until the end of the study for that patient as defined by the protocol.

Laboratory, vital signs, or ECG abnormalities are to be recorded as AEs only if:

- symptomatic, and/or

- requiring either corrective treatment or consultation, and/or

- leading to IMP/NIMP discontinuation or modification of dosing, and/or

- fulfilling a seriousness criterion, and/or

- defined as an AESI.

### 10.3.2 Guidelines for reporting serious adverse events

In the case of a SAE, the Investigator must immediately:

These first 4 bullets should be applicable in case of paper case report form used.

- ENTER the information related to the serious adverse event in the appropriate screens of the e-CRF; the system will automatically send the notification to the monitoring team after approval by the Investigator within the e-CRF or after a standard delay.

- SEND (preferably by fax or e-mail) the photocopy of all examinations carried out and the dates on which these examinations were performed, to the representative of the monitoring team whose name, fax number, and e-mail address appear on the clinical trial protocol. Care should be taken to ensure that the patient's identity is protected and the patient's identifiers in the clinical trial are properly mentioned on any copy of source document provided to the Sponsor. For laboratory results, include the laboratory normal ranges.

- All further data updates should be recorded in the e-CRF as appropriate, and further documentation as well as additional information (for laboratory data, concomitant medication, patient status, etc) should be sent (by fax or e-mail) to the monitoring team within 1 working day of knowledge. In addition, any effort should be made to further document within the week (7 days) following initial notification any serious adverse event that is fatal or life threatening.

- A back-up plan is used (using paper flow) when the e-CRF system does not work.

These next 3 bullets will be applicable in case of e-CRF is used for a Back-up plan)

- SEND (preferably by fax or e-mail) the signed and dated corresponding page(s) in the case report form to the representative of the monitoring team whose name, fax number, and e-mail address appear on the clinical trial protocol.

- ATTACH the photocopy of all examinations carried out and the dates on which these examinations were performed. Care should be taken to ensure that the patient's identity is protected and the patient's identifiers in the clinical trial are properly mentioned on any copy of source document provided to the Sponsor. For laboratory results, include the laboratory normal ranges.

- All further documentation should be sent to the monitoring team within 1 working day of knowledge. In addition, every effort should be made to further document within the week (7 days) following initial notification any serious adverse event that is fatal or life threatening.

- Any SAE brought to the attention of the Investigator at any time after the end of the study for the patient and considered by the Investigator to be caused by the IMP with a reasonable possibility, should be reported to the monitoring team.

### 10.3.3 Guidelines for reporting adverse events of special interest

The need for specific monitoring, documentation, and management of AESI are described in this section.

For each defined adverse events of special interest, consider carefully the need to collect additional specific information that would impact the study and/or the case report form design, such as:

- Preexisting related condition or lifestyle of interest for the adverse event (eg, habits, cardiovascular risk factor, etc)

- Expected list of associated signs and symptoms

- Corrective actions (eg, treatment discontinuation, concomitant treatment, etc)

- Diagnostic actions (eg, test(s) or procedure(s) results, etc)

- Additional descriptive factors

- Sequelae

### *10.3.3.1 Reporting of adverse events of special interest with immediate notification*

For AESI with immediate notification, the Sponsor is to be informed immediately (ie, within 1 working day), as per SAE notification guidelines described in Section 10.3.2, even if a seriousness criterion is not met, using the corresponding pages of the case report form (to be sent) or screens in the e-CRF.

- ALT increase ≥2 x ULN (refer to related decision chart in Appendix A)

- QTc ≥500 ms

In the event of prolongation of QTc interval (automatic measurement) ≥500 ms, confirmed by a manual reading by the Investigator or a physician delegated by the Investigator using the Fridericia formula for correcting QT, the patient should be placed under supervision in a specialized setting. Investigational medicinal product administration must be stopped and appropriate blood samples collected. Subsequent ECG monitoring of the patient should then be performed on a regular and clinically responsible basis until the QTc interval returns to a safe value as determined by the Investigator in agreement with the Sponsor.

- Pregnancy

    - Pregnancy occurring in a female patient included in the clinical trial: Pregnancy will be recorded as an adverse event of special interest with immediate notification in all cases. It will be qualified as a serious adverse event only if meeting one of the seriousness criteria.

    - In the event of pregnancy, IMP should or must be discontinued.

    - Follow-up of pregnancy will be mandatory until its outcome has been determined.

- Symptomatic overdose with IMP

    - An overdose (accidental or intentional) with the IMP is an event suspected by the Investigator and defined as at least twice of the intended dose within the intended therapeutic interval, adjusted according to the tested drug

### 10.3.3.2 Reporting of adverse events of special interest without immediate notification

- Asymptomatic overdose with IMP (Refer to Section 10.3.3.1)

- Symptomatic hypoglycemia (see definition below) with an accompanying **plasma glucose < 60 mg/dL** (3.3 mmol/L) or associated with prompt recovery after oral carbohydrate administration if no plasma glucose measurement is available.

Symptomatic hypoglycemia is defined as an event with clinical symptoms that are considered to result from a hypoglycemic episode (e.g., sweating, palpitations, hunger, restlessness, anxiety, fatigue, irritability, headache, loss of concentration, somnolence, psychiatric or visual disorders, transient sensory or motor defects, confusion, convulsions, or coma).
Symptoms with an associated blood glucose measurement ≥ 60 mg/dL (3.3 mmol/L) should not be reported as a hypoglycemia.
In the present study, the possibility of self-measured blood glucose (SMBG) will be possible, whenever the patients feel hypoglycemic symptoms. Patients should be instructed to measure plasma glucose levels prior to the administration of glucose or carbohydrate intake.
Symptomatic hypoglycemia is to be reported as an adverse event. It should be recorded in the CRF on the specific AE form for symptomatic hypoglycemia. Additional information should be collected on a specific symptomatic hypoglycemic event complementary form.

- Severe symptomatic hypoglycemia

Severe symptomatic hypoglycemia is defined as an event with clinical symptoms that are considered to result from hypoglycemia in which the patient required the assistance of another person, because the patient could not treat him/herself due to acute neurological impairment directly resulting from the hypoglycemic event, and one of the following:

- The event was associated with a **plasma glucose level below 36 mg/dL (2.0 mmol/L).**

- If no blood glucose measurement is available, then the event was associated with prompt recovery after oral carbohydrate, intravenous glucose, or glucagon administration.

The definition of severe symptomatic hypoglycemia includes all episodes in which neurological impairment was severe enough to prevent self-treatment and which were thus thought to place patients at risk for injury to themselves or others. Note that "requires assistance" means that the patient could not help himself or herself. Someone being kind that assists spontaneously the patient when not necessary does not qualify as "requires assistance."

Severe symptomatic hypoglycemia will be qualified as an SAE only if it fulfills SAE criteria.

- Suspected Pancreatitis

In case of severe, persistent abdominal pain, which can radiate to the back, often with characteristic positional features, with possible occurrence of nausea, vomiting, fever and leucocytosis, further measurement of amylase and lipase should be performed. The diagnosis of pancreatitis may be supposed also if other causes of abdominal pain are excluded (i.e., gallbladder disease, etc) and elevated amylase/lipase is seen and in addition pancreatic changes are seen on ultrasound and/or CT or MRI (with contrast, as appropriate).
Pancreatic enzymes (amylase, lipase) must be measured.

→ Amylase and lipase values greater than 2-fold ULN should be repeated within 7 days.
→ Amylase and lipase values greater than 3-fold ULN should be repeated within 48 hours. If the value remains above 2-fold ULN, it should be repeated weekly until it is less than 2-fold ULN. Amylase and lipase elevations without associated clinical symptoms should receive a gastroenterologic evaluation with additional imaging, as appropriate.
All the laboratory or clinical documentations should be collected. As soon as there are signs, symptoms and results of investigations exploring suspected pancreatitis (eg, laboratory results, imaging reports, gastroenterologist's evaluations, etc) related to suspected pancreatitis, the investigator must document and report them on a specific e-CRF form.

With any diagnosis of acute pancreatitis, the investigational treatment and other potentially suspect drugs should be stopped and the patient followed further clinically

- Allergic or allergic-like reaction

In case a patient experiences an allergic reaction or an allergic-like reaction this has to be reported as an adverse event. Additional information is collected on specific allergic reaction forms. Allergic, or possible allergic reactions will be adjudicated by the Allergic Reaction Assessment Committee (ARAC, Section 6.3.1).

### 10.3.4 Guidelines for management of specific laboratory abnormalities

Once the patient is included in the clinical trial, the following laboratory abnormalities must be monitored, documented, and managed,

- Neutropenia

- Thrombocytopenia

- Acute renal insufficiency

- Suspicion of rhabdomyolysis

### 10.4 OBLIGATIONS OF THE SPONSOR

During the course of the study, the Sponsor will report in an expedited manner:

- All SAEs that are both unexpected and at least reasonably related to the IMP (SUSAR), to the Health Authorities, IRB/IECs as appropriate and to the Investigators.

- All SAEs that are expected and at least reasonably related to the IMPs to the Health Authorities, according to local regulations.

## 11 HANDLING OF PATIENT WITHDRAWAL

The basis of reason for treatment withdrawal should be identified.

### 11.1 LIST OF TREATMENT WITHDRAWAL CRITERIA

Refer to Section 10.3
Pregnancy will lead to permanent treatment discontinuation in all cases (Refer to Section 10.3.3.1).

### 11.2 REASONS FOR TREATMENT WITHDRAWAL

Patients can withdraw from the treatment if they decide to do so, at any time, and for any reason, or this may be the Investigator's decision.

### 11.3 REPLACEMENTS OF PATIENTS

A patient who prematurely end his/her treatment study participation after the start of the baseline period and who received study drug can be replaced in order to obtain as far as possible 12 completed patients per population. In the event of discontinuation due to occurrence of AE, the replacement will be discussed between the Investigator and the Sponsor. Replacement patients must meet all inclusion and exclusion criteria.
The replacement patients will have a different patient number, by adding 500 to the number of patient replaced.

### 11.4 FOLLOW-UP PROCEDURE FOR TREATMENT WITHDRAWAL

All study treatment withdrawals should be recorded by the Investigator on the appropriate case report form pages or screens for e-CRF when considered as confirmed.
If possible, patients are to be assessed using the procedure planned for the end-of-study visit, including a pharmacokinetic sample if appropriate.
For any patient who fails to return to the site, the Investigator should make every effort to recontact the patient (eg, contact the patient's family or private physician, review available registries or health care database), and to determine his/her health status, including at least his/her vital status. Attempts to contact the patient must be documented in the patient's records (eg, times and dates of attempted telephone contact, receipt for sending a registered letter).
Patients withdrawn from the study must not be reincluded in the study. Their inclusion and treatment numbers must not be reused.

## 12 STUDY PROCEDURES

### 12.1 VISIT SCHEDULE

The study consists of a screening period up to 4 weeks followed by a randomized, double-blind, 3 crossover treatment periods 1-7 days apart. Each period will last one day only (but, if all examinations and tests to be performed before dosing on Day 1, are not possible, a visit on Day -1 (afternoon) at the convenience of patient and the possibilities of the investigational site (e.g., an institutionalization / an accommodation for one night before Day 1 at each period).
At each treatment period, the patients will receive a subcutaneously injected single dose of either 5 $\mu$g or 10 $\mu$g lixisenatide with 5 $\mu$g preceding the 10 $\mu$g dose level or volume matched placebo (50 $\mu$L or 100 $\mu$L). The end-of-study visit is scheduled between Day 2 and Day 7 of the treatment period 3.
All the in-clinic (Day 1) visits should take place in the morning at approximately the same time.

### 12.1.1 Screening procedures

Screening procedures will be carried out within 30 days prior to inclusion but blood sampling should **be done at latest on Day -25** to obtain the results before the randomization (Dayl).
For paediatric study population:
At this first contact the study will be explained to the patient's parents or legal guardian (hereinafter the "parent"). The parent will receive verbal information concerning the aims and methods of the study, its constraints and risks, and the study duration. Written informed consent must be signed by the parent prior to any investigations. In addition, provision of Assent Form will be signed by minor patients or Informed Consent Form will be signed by emancipated or mature minors (defined by local lows).

For adult study population:
The patient will receive information on the study objective(s) and procedures from the Investigator. The adult patient will have to sign the informed consent prior to any action related to the study.

For all patients, the screening visit will include the following investigations (refer to Section 9.2):

1. Demographics: age, sex, race, height, body weight in kg, BMI [Body Mass Index = weight (in kg)/height (in cm)$^2$];

2. For paediatric population: tanner staging (*screening only*).

3. Relevant medical history including personal or familial history of medullary thyroid cancer (MTC) or a genetic condition that predisposes to MTC, patient's allergy medical history, risk factors for pancreatitis [e.g., habits of alcohol consumption (none, $\leq$ 2 drinks / day or > 2)] and surgical history;

4. Physical examination (cardiovascular system, chest and lungs, thyroid, abdomen, nervous system, skin and mucosae, and musculo-skeletal system);

5. History of type 2 diabetes history (date of the diagnosis of diabetes);

6. Concomitant and previous medication including antidiabetic treatments in the last 3 months prior to study entry, start date of treatment with metformin if administered, daily dose of metformin at Baseline (refer to the inclusion criteria I01, Section 7.2) ;

7. ECG (standard 12-lead), vital signs measurements (heart rate, systolic and diastolic blood pressure measured after 10 minutes rest in supine position);

8. Body temperature

9. Urine drug screen: amphetamines/methamphetamines, barbiturates, benzodiazepines, cannabinoids, cocaine, and opiates;

10. Alcohol test;

11. Laboratory tests in fasting condition with hematology and clinical chemistry including serum , antibody test (anti-IA2, and anti-GAD), fasting C-peptide, HbA1c, fasting plasma glucose, calcitonin, serologies (hepatitis B antigen, hepatitis C antibodies, anti-HIV1 and anti-HIV2 antibodies), urinalysis, $\beta$-HCG blood test solely in females of reproductive potential (Tanner Stage $\geq$ 3), serum FSH (in adult women, if applicable, to confirm postmenopausal status);

12. Each centre will call IXRS to receive an incremental identification number of their patient corresponding to his/her order of enrollment in the study (refer to Section 8.5);

Patients who meet all the inclusion criteria and none of the exclusion criteria will be eligible for the inclusion visit (Day 1).

### 12.1.2 Description by type of visit

#### 12.1.2.1 Treatment period 1

**Inclusion procedures**

The inclusion visit will be carried out on the day of inclusion (Day 1) and will include the following investigations (refer to Section 9.2):

1. For safety and practical reasons, for paediatric population, topical application of cream or other anesthesic local application (e.g.; EMLA®) can be applied on the forearm approximately 1 hour before the venipuncture at the site where the catheter for blood sampling will be in place for reducing pain in patients, especially in children;

2. Physical examination: medical history, weight, and body temperature;

3. ECG and vital signs measurements;

4. Urinary pregnancy test in females with reproductive potential (Tanner stage ≥ 3);

5. Urine drug screen: amphetamines/methamphetamines, barbiturates, benzodiazepines, cannabinoids, cocaine, and opiates;

6. Alcohol test;

Rechecking of any baseline parameter is to be limited to one time except when the measurement has not been obtained in accurate conditions. The last value should be considered as the baseline value and reported in the case report form. If a parameter at baseline is part of specific inclusion criteria, rechecking is not permitted; one abnormal value is cause for exclusion.

Patients who meet all the inclusion criteria and none of the exclusion criteria will be eligible for inclusion in the study. Final inclusion and randomization will be performed just before the IMP administration on Day 1.

For safety and practical reasons, approximately 15 minutes before blood sampling, an indwelling catheter may be inserted in a peripheral vein of the forearm in order to obtain blood samples. Between samplings, the catheter will be locked with a mandrel. Heparin use during blood draws is NOT allowed (to avoid any potential contamination with heparin which may interfere with the drug/antibody assays). 0.9% saline can be used to flush the collection catheter. Prior to collection of the blood sample via an indwelling catheter, 0.5 mL of blood has to be drawn and discarded to avoid dilution.

When the patient is confirmed for the study inclusion, the treatment period will include the following investigations (refer to Section 9.2):

1. According to the procedure described in Section 8.5, the Pharmacist or the Independent person will call IXRS to receive the treatment kit number allocation;

2. Blood sampling for anti-lixisenatide antibodies test before the IMP administration;

3. 0.5h before breakfast, self injection of lixisenatide at the dose of 5 $\mu$g (5 Units indicated on OptiClick®) or 10$\mu$g QD (10 Units indicated on OptiClick®) or placebo (50 $\mu$L or 100 $\mu$L), under the observation of the medically qualified designee. However, for blinding purposes the on-site administration of lixisenatide or its placebo will therefore be performed by an independent person who is not a member of the clinical study team at the CRO or investigational site;

4. Standardized breakfast (refer to Section 8.4 , Appendix D) given 30 minutes after the first blood pharmacodynamic sampling, corresponding to the pre-specified time T0.5h.;

5. Blood sampling for the evaluation of plasma glucose, insulin, C-peptide- and glucagon starting between approximately 07:30 and 09:00 (T0 before the study drug administration) with 7 (8 for plasma glucose) pre- specified timepoints: T0, T0.5 (just before breakfast), T1, T1.5, T2 (plasma glucose only), T2.5, T3.5, and T4.5 (before lunch);

6. Blood sampling for PK starting between approximately 07:30 and 09:00 (T0, before the study drug administration) and at 7 pre- specified timepoints after dosing: T0.5 (just before breakfast), T1, T1.5, T2.5, T3.5, T4.5 (before lunch) and T6.5;

7. vital signs measurements at T2.5 and T6.5

8. Physical examination and ECG before discharge (T6.5);

9. Recording of adverse events and concomitant medication, if any;

10. Patients will be discharged after a complete review of the available safety data by the Investigator;

11. Patients will be instructed to come back to the study site within 8 days (between Day 2 and Day 8);

### *12.1.2.2 Treatment periods 2 and 3*

The treatment period will include the following investigations (refer to Section 9.2):

1. For safety and practical reasons, for paediatric population, topical application of cream or patch (eg, EMLA®) approximately 1 hour before the venipuncture at the site where the catheter for blood sampling will be in place (if patient is definitively selected) for reducing pain in patients, especially in children.

2. For safety and practical reasons, approximately 15 minutes before blood sampling, an indwelling catheter may be inserted in a perpheral vein of the forearm in order to obtain blood samples. Between samplings, the catheter will be locked with a mandrel. Heparin use during blood draws is NOT allowed (to avoid any potential contamination with heparin which may interfere with the drug/antibody assays). 0.9% saline can be used to flush the collection catheter. Prior to collection of the blood sample via an indwelling catheter, 0.5 mL of blood has to be drawn and discarded to avoid dilution.

3. Recording of adverse events and concomitant medication, if any;

4. Urine drug screen: amphetamines/methamphetamines, barbiturates, benzodiazepines, cannabinoids, cocaine, and opiates before IMP administration ;

5. Alcohol test before IMP administration;

6. Physical examination including body weight, body temperature and the respect of the adherence to study restrictions before IMP administration;

7. ECG and vital signs measurements before IMP administration ;

7. Urinary pregnancy test in females with reproductive potential (Tanner stage $\geq$ 3) before IMP administration;

8. 0.5h before breakfast, self injection of lixisenatide at the dose of 5 $\mu$g (5 Units indicated on OptiClick®) or 10$\mu$g QD (10 Units indicated on OptiClick®) or placebo (50 $\mu$L or 100 $\mu$L), under the observation of the medically qualified designee. However, for blinding purposes the on-site administration of lixisenatide or its placebo will therefore be performed by an independent person who is not a member of the clinical study team at the CRO or investigational site;

9. Standardized breakfast (refer to Section 8.1), given 30 minutes after the first blood pharmacodynamic sampling, corresponding to the pre-specified time T0.5h.;

10. Blood sampling for the evaluation of plasma glucose, insulin, C-peptide- and glucagon starting between approximately 07:30 and 09:00 (T0 before the study drug administration) with 7 (8 for plasma glucose) pre- specified timepoints: T0, T0.5 (just before breakfast), T1, T1.5, T2 (plasma glucose only), T2.5, T3.5, and T4.5 (before lunch);

11. Blood sampling for PK starting approximately between 07:30 and 09:00 (T0, before the study drug administration) and at 7 pre- specified timepoints after dosing: T0.5 (just before breakfast), T1, T1.5, T2.5, T3.5, T4.5 (before lunch) and T6.5;

12. Vital signs measurements at T2 and T6.5

13. ECG and physical examination performed at T6.5;

14. Recording of adverse events and concomitant medication, if any;

15. Patients will be discharged after a complete review of the available safety data by the Investigator;

16. At period 3, each centre will call IXRS to inform that all treatments have been administered (end of the treatment period) for the given patient;

17. Patient will be instructed to come back to the study site within 8 days (between Day 2 and Day 8);

**Ambulatory period(s)**

During the study, patients should immediately contact the Investigator or one of the clinical unit managers in the event of any unexplained symptom or any unexpected effect or event occurring during the study. For this reason, patients will be informed that they can contact the clinical unit by telephone 24 hours a day. Patients must give the Investigator a telephone number where they can be contacted in an emergency. Patients must carry with them, during ambulatory study period(s), the patient card indicating the patient number and the emergency telephone number provided by the study site.

### 12.1.2.3 End-of-study visit

The end-of-study visit will be performed between 1 to 6 days after last dosing (D2 to D7 after Period 3); it will include the following investigations (refer to Section 9.2):

1. Physical examination including body weight;

2. ECG and vital signs measurements;

3. Laboratory tests in fasting conditions with hematology and clinical chemistry;

4. Urinalysis;

5. Recording of adverse events and concomitant medication, if any;

6. IXRS call for the end of the study for the patient given;

### 12.1.3 Study restriction(s)

The effects of the glycemic index (GI) of carbohydrate eaten the previous night on the glycaemic response to a standard test meal eaten subsequently in the morning (breakfast) have been described (11, 12). As far as possible, recommendation will be given to patient to eat a pasta course at the dinner preceding Day 1 of each period.
After the dinner on Day-1, patients should stay in fasted conditions for at least 8 hours (food and drink are not allowed except water) up to the standardized test meal.
At each site visit, on Day 1 of each period, patients should refrain from drinking alcohol, tea, coffee, chocolate, quinine, or caffeine-containing beverages. Consumption of citrus fruits and their juices is prohibited during the treatment period (Day1 of each period). Smoking and tobacco use will not be allowed from 1 day prior to institutionalization throughout the study duration until the end-of-study visit. Patients will receive standardized meal test (liquid test for breakfast) (see Section 8.1).
Patients will be requested to follow a stable lifestyle **with no intensive physical activity** for the duration of the study until the end-of-study visit.

### 12.2 DEFINITION OF SOURCE DATA

All evaluations that are reported in the case report form must be supported by appropriately identified source documentation.

- Agreement and signature of informed consent mentioning the study identification,

- Patient identification, last participation in a clinical trial, medical history, associated diseases, and data related to the studied pathology,

- Contraception method for women of childbearing potential,

- Previous and concomitant medication,

- Study identification,

- Dates of administration and doses of lixisenatide or placebo,

- Start date of metformin if patients are treated with, and daily dose at screening,

- Dates of visits and assessments including the examination report,

- Vital signs, height, body weight,

- laboratory assessments, ECG;

- Pharmacodynamic and pharmacokinetic time points

- start/end of meals

- ECG records signed and dated,

- Adverse events and follow-up:

- In case of SAE, the site should file in the source document at least copies of the hospitalization reports (if appropriate) and any relevant examination reports documenting the follow-up of the SAE.

- Date of premature study discontinuation (if any) and reason.

Source documentation may be found in the following:

- Patient's identity,

- Medical history,

- Nursing notes,

- Physician's notes,

- Patient's diaries.

- start/end of ECG

## 13 STATISTICAL CONSIDERATIONS

The material in Section 13 of the clinical trial protocol constitutes the statistical analysis plan for the study. Should this plan need revision during the study to accommodate clinical trial protocol amendments or to adapt to unexpected issues in study execution and data that affect planned analyses, a statistical analysis plan will be issued prior to database lock.

### 13.1 DETERMINATION OF SAMPLE SIZE

Power calculation was based on the results of the double-blind, placebo-controlled, single-dose, study AV-E0010/01-016 performed in patients with type 2 diabetes. The following table (Table 11) summarizes the results of the comparison of single doses of 3 μg and 10 μg lixisenatide with placebo for the AUC1-5h of plasma glucose. It can be assumed that the effect of 5 μg is more pronounced than in 3 μg.

**Table 11** - **Results from study AVE0010 / 01-016 - Statistical Analysis of Area Under Curve for plasma Glucose: Pairwise Comparisons Between Dose Groups 3 and 5 μg versus placebo**

| Dose | N | Mean difference to Placebo (mg/dl) | Standard Error (mg/dl) |
|---|---|---|---|
| 3 μg | 4 | 154 | 50 |
| 10 μg | 4 | 347 | 50 |

Power calculation was performed for a 2-group t-test (Crossover ANOVA) for differences in means between active treatment and placebo for different standard deviations of 70, 100 and 150 mg.h/dL to take into consideration a possible higher variation in paediatrics than in adults. A type 1 error alpha = 5 % and a Bonferroni corrected alpha = 2.5 % was used for the power calculation.

**Table 12 - Power calculation for Plasma Glucose AUC - alpha = 5 %**

| | Power calculation for 12 patients with alpha = 5% for blood glucose AUC | | | | | |
| | Dose levels | | | | | |
| | 5 μg | 10 μg | 5 μg | 10 μg | 5 μg | 10 μg |
|---|---|---|---|---|---|---|
| Within-patient standard deviation (mg.h/dL) | 70 | 70 | 100 | 100 | 150 | 150 |
| Difference in means (mg.h/dL) | 155 | 350 | 155 | 350 | 155 | 350 |
| Power (%) | 99 | 99 | 92 | 99 | 62 | 99 |
| Total N | 12 | 12 | 12 | 12 | 12 | 12 |

2-sided t-test (Crossover ANOVA) for difference of means

**Table 13 - Power calculation for Blood Glucose AUC - alpha = 2.5 %**

| | Power calculation for 12 patients with alpha = 2.5% for plasma glucose AUC | | | | | |
| | Dose levels | | | | | |
| | 5 μg | 10 μg | 5 μg | 10 μg | 5 μg | 10 μg |
|---|---|---|---|---|---|---|
| Within-patient standard deviation (mg.h/dL) | 70 | 70 | 100 | 100 | 150 | 150 |
| Difference in means (m.hg/dL) | 155 | 350 | 155 | 350 | 155 | 350 |
| Power (%) | 99 | 99 | 85 | 99 | 48 | 99 |
| Total N | 12 | 12 | 12 | 12 | 12 | 12 |

2-sided t-test (Crossover ANOVA) for difference of means

With a total of 12 patients, a crossover design would have 99% power to detect a difference in the mean corrected plasma glucose-$AUC_{0:30h-4:30h}$, between lixisenatide and placebo of 19.43 mmol.h/L (350 mg.h/dL) assuming a within-standard deviation of 5.55 mmol.h/L (100 mg.h/dL), using a 2-group t-test with a 0.05 two-sided significance level, and 92% power to detect a difference in means of 8.60 mmol.h/L (155 mg.h/dL). Additional details are provided in 16-1-1-protocol [13].

## 13.2 PATIENT DESCRIPTION

### 13.2.1 Disposition of patients

A detailed description of patient accountability including count of patients randomized and treated (i.e. having a randomization number assigned and who received at least one administration of the Investigational Medicinal Product (IMP)), and who discontinued along with the main reason for discontinuation and who requested treatment discontinuation, will be generated by population (paediatric and adult) and treatment group within population.

Patient disposition at the end-of-study (EOS) visit will be presented in a listing sorted by population and patient within sequence, including patients' status (alive or dead) at the end of the study with the date of last study drug intake, date of last available information and method of contact, date of EOS visit, reason for discontinuation, and whether the blind was broken on site at time of discontinuation. All withdrawals from the study, taking place on or after IMP administration, will be fully documented in the body of the CSR.

In case of code broken for medical and accidental reasons on site, a listing of concerned patients will be provided, specifying the reason (AE/SAE or other), the date and time of code breaking and the person who broke the code.

A listing of comments on the e-CRF related to investigational product compliance and dosing, safety (adverse events, laboratory, vital signs and ECG data) or other comments will be provided.

### 13.2.2 Protocol deviations

During the review of the database, the compliance with the protocol will be examined with regard to inclusion and exclusion criteria, treatment compliance, prohibited therapies, and timing and availability of planned assessments. Protocol deviations will be identified by the study team before database lock and listed in the Data Review and Surveillance Report, including missing data and study drug discontinuations, and classified as important or other deviations.

Individual deviations to inclusion and exclusion criteria as reported by the Investigator will be listed.

If any, important deviations will be listed by population (paediatric, adult) and patient and/or described in the body of the clinical study report.

## 13.3 ANALYSIS POPULATION

A summary table of count of patients included in each analysis population (pharmacodynamic, pharmacokinetic and safety) will be provided by population (paediatric and adult) and by treatment within population. All exclusions from any analysis populations will be fully documented in the CSR.

### Safety population

All randomized patients exposed to the IMP (regardless of the amount of treatment administered) will be included in the safety population.

### Pharmacokinetics population

Two pharmacokinetic (PK) populations will be considered.

- The full analyses population including all patients without any major deviations related to study drug administration, and for whom any pharmacokinetic parameters are available.

- The evaluable population including patients from full analyses population who completed both lixisenatide treatments in compliance with the protocol and having blood samples for reliable evaluation.

The primary PK population is the evaluable population. The full analyses population will only be analyzed if the number of evaluable patients differ by more than 3 (>= 3).

The placebo treatment period can not be considered.

### Pharmacodynamic population

Two pharmacodynamic (PD) populations will be considered:

- The full analyses population including all randomized and treated patients without any important deviation related to IMP administration for whom the primary PD data is considered sufficient and interpretable.

- The evaluable PD population including patients from the full analyses population who completed all 3 treatment periods in compliance with the protocol and having blood samples for reliable evaluation.

Patients will be analyzed as treated. The primary PD population is the evaluable population. The full analyses population will only be analyzed if the number of evaluable patients differ by more than 3 (>= 3).

## 13.4 DEMOGRAPHIC AND BASELINE CHARACTERISTICS

### 13.4.1 Patient demographic characteristics, medical history and diagnoses

Continuous variables (age, weight, BMI, duration of diabetes, duration of anti-diabetic treatment, age at onset of diabetes) and qualitative variables (gender, race, pubertal stage) will be summarized by descriptive statistics by treatment group within population (paediatric, adult), and for all patients for the safety population and for the PD and/or PK population, if relevant.

All demographic data will be listed.

### 13.4.2 Baseline pharmacodynamic parameters

The baseline will be the Day 1 pre-dose measurement of each parameter.

**13.4.3 Baseline safety parameters**

Baseline for safety parameters will be defined as the last available and evaluable parameter value before and closest to the first dosing on Day-1/Day 1 in each period for vital sign parameters and for ECG parameters and during screening for laboratory data.

Baseline definitions specific to each type of safety parameter will be detailed in corresponding Sections 13.8.3.2 to 13.8.5).

**13.5 EXTENT OF STUDY TREATMENT EXPOSURE AND COMPLIANCE**

A summary table presenting the exposure of treatment (ie, duration of IMP in days, defined by: end date of administration - start date of administration +1) will be provided by treatment group within population, on the safety population.

The following listings will be provided:

- Details of drug dosing (actual treatment received, date and time of IMP intake, route of administration, intended and actual dose received)

- The patients receiving IMP from specified batch

- Randomization scheme

- A listing of meal data.

**13.6 PRIOR/CONCOMITANT MEDICATION/THERAPY**

Previous medications and concomitant treatments will be coded according to the World Health Organization - Drug Dictionary (WHO-DD, last version available). Patients who took medications that were stopped before the first IMP dosing, and/or patients who received concomitant treatments with the IMP will be listed. In addition, a separate listing of the previous anti-diabetic medication will be provided.

**13.7 ANALYSIS OF PHARMACODYNAMIC VARIABLES**

**13.7.1 Description of pharmacodynamic variable(s)**

The pharmacodynamic data will be collected and managed by a Central Laboratory. The pharmacodynamic parameters will be derived from using plasma glucose, insulin, C-peptide and glucagon concentrations.

All the pharmacodynamic analyses will be performed using the evaluable PD population. If the evaluable PD population differs by more than 3 patients (>=3) then the full analyses population will be analyzed in addition.

The paediatric and the adult population will be analyzed separately. Results will be compared between paediatrics and adults descriptively.

***13.7.1.1 Primary variable***

The following PD variable will be considered as primary:

- (GLU-AUC$_{0:30-4:30h}$) calculated as the area under the plasma glucose concentration time curve from time of breakfast start (30 min after IMP injection i.e. T0.5h) until 4 hours later (T4.5h) subtracting the pre-meal value T0.5.

The trapezoidal rule will be used to calculate the AUC.

***13.7.1.2 Secondary variables***

The following variables will be used as secondary for pharmacodynamic analyses:

- Post-prandial plasma glucose (PPG) excursion: PPG excursion will be calculated from the difference between the maximum after the standardized breakfast and before lunch subtracting the pre-meal plasma glucose (T0.5).
- AUC0:30 -4:30 of insulin, C-peptide and glucagon concentrations: the area under the concentration time profile from time of standardized breakfast start (30 min after IP injection and pre-meal plasma glucose = T0.5) until 4 hours later (T4.5). AUC will be calculated using the trapezoidal rule.

### 13.7.2 Primary analysis

GLU-AUC0:30-4:30h will be analyzed using the following analyses of covariance (ANCOVA) model with treatment (lixisenatide 5 $\mu$g and 10 $\mu$g and placebo pooled across both placebo formulations), sequence (6 sequences), period (1, 2 and 3) as fixed effects, and patient-within-sequence as random effect, and the T0.5h plasma glucose concentration as covariate using SAS® PROC MIXED procedure:

$$\text{GLU-AUC0:30-4:30h} = \text{Treatment} + \text{period} + \text{sequence} + \text{patient (sequence)} + \text{plasma glucose T0.5} + \text{error}$$

In case the number of patients in at least one sequence is too small, the model has to be adapted by removing the sequence effect.

The least square mean differences between treatment groups and the corresponding 90% confidence interval (CI) will be calculated within the linear mixed model framework. A significance level of $p< 0.05$ will be used. No adjustment for multiplicity will be performed.

### 13.7.3 Secondary analysis/analysis of secondary variables

The secondary pharmacodynamic parameters PPG and the AUCs of insulin, C-peptide, and glucagon will be analyzed using the same statistical model as described above with the corresponding T0.5 h values as covariates.

GLU-AUC0:30-4:30h, PPG and the AUCs of insulin, C-peptide and glucagon will be compared between the paediatric and adult populations descriptively.

Individual and mean ($\pm$SEM) profiles of plasma glucose, insulin, C-peptide and glucagon will be plotted by population and treatment group

Raw data and derived parameters will be listed.

### 13.8 ANALYSIS OF SAFETY DATA

The safety evaluation will be based upon the review of the individual values (clinically significant abnormalities), descriptive statistics (summary tables, graphics) and if needed on statistical analysis (appropriate estimations, confidence intervals), following the sanofi-aventis guideline for reporting of phase 1 studies "Summarizing and reporting Clinical pharmacology trial data". All the safety analyses will be performed using the safety population.

For all safety data, the observation period will be divided into three segments:

- The pre-treatment phase defined as the time between the patients give informed consent and the first IMP administration.

- The on-treatment phase defined as the time from the first IMP administration up to 24 hours after last administration of IMP (included).

- The post-treatment phase will be defined as the time after the on-treatment phase.

All analyses will be based on the on-treatment phase.

For the adults a sanofi-aventis specific list of criteria defining "Potentially Clinical Significant Abnormalities" (PCSAs) will be used for the statistical analysis and presentation of laboratory parameters, vital signs and ECG data. The last version for definition of PCSAs available at the time of database lock will be used.

### 13.8.1 Adverse events

Adverse events will be coded according to the Medical Dictionary for Regulatory Activities (MedDRA, last available version).

They will be classified into predefined standard categories according to chronological criteria:

- Pre-treatment AEs are defined as AEs that occurred, worsened (according to investigator opinion) or became serious during the pre-treatment phase.

- Treatment emergent AEs (TEAEs) are defined as AEs that occurred or worsened or became serious during the on-treatment phase.

- Post-treatment AEs are defined as AEs that occurred worsened or became serious during the post-treatment phase.

Treatment emergent adverse events will be assigned to the treatment group received at the time of the AE onset.

If the start date (or time) of an AE is incomplete or missing, then the AE will be considered as a TEAE unless a partial date (or time) shows it as a pre- or post-treatment event. If a TEAE develops on one period and worsens in the following period, it will be considered treatment emergent for both periods.

All AEs reported in the study will be listed, sorted by population (paediatric, adult) and patient, onset date and time. Nevertheless, the analyses of the AEs will focus on the TEAEs.

### 13.8.1.1 Treatment-emergent adverse events

The following frequency distributions of TEAEs (incidence tables) will be provided for the safety population by treatment within population for the total on-treatment period:

- Overview of TEAE: Number and percentage of patients with at least one TEAE, severe TEAEs, serious TEAEs, TEAEs leading to treatment discontinuation and, if any occurred, TEAEs leading to death

- Summary of treatment-emergent adverse events by primary system organ class and preferred term - Number and percentage of patients with at least one TEAE

- Summary of treatment-emergent adverse events by primary system organ class and preferred term - Number and percentage of patients and the number of events

- Listing of patients presenting treatment emergent adverse events by population, treatment, system organ class and preferred term

### 13.8.1.2 Deaths, serious, and other significant adverse events

Deaths, serious AEs, and other significant AEs (eg, related to specific laboratory abnormalities) will be listed individually and described in the study report in detail.

### 13.8.1.3 Adverse events leading to treatment discontinuation

In case of any occurrences, individual patient listings will be generated for all adverse events leading to treatment discontinuation.

### 13.8.1.4 Allergic reactions

### Listings for allergic reactions

Any cases of allergic reaction will be documented as adverse events with detailed complementary information. A listing of individual data (separate from the listing of all adverse events) will be provided, sorted by patient, onset date and time, irrespective of the definition of the on-treatment phase, including specifically description of the adverse event, symptoms of the adverse event, possible etiologies, actions taken, vital signs measurements (at outset, during reaction and at recovery) and a description of the allergic or allergic-like event.

The assessment of all these cases by the Allergic Reaction Assessment Committee (ARAC) will be also listed, including notably whether the event reported constitutes an allergic reaction, and if it does, its diagnosis and severity grade.

All cases will be described in detail in the CSR.

### Allergic medical history and family medical history

Allergic medical history and family medical history is to be documented for patients with any occurrence of potential allergic reaction and will be coded according to the MedDRA dictionary (latest version in use at time of database lock). All details of allergic medical history and of allergic family medical history will be listed on an individual basis.

### 13.8.1.5 Pancreatitis

Any cases of pancreatitis will be documented as adverse events with detailed complementary information. A listing of individual data (separate from the listing of all adverse events) will be provided, sorted by patient, onset date and time, irrespective of the definition of the on-treatment phase, including notably description of the adverse event, values of amylase and lipase, gastroenterologist's evaluation and potential causes of the pancreatitis. All cases will be described in detail in the clinical study report.

### 13.8.1.6 Hypoglycemia

Symptomatic hypoglycemia will be reported together with all adverse events.

### 13.8.2 Clinical laboratory evaluations

### 13.8.2.1 Biochemistry, hematology and coagulation data

**Baseline definition**

The values to be used as baselines will be the values collected during screening assessments. If any of the scheduled baseline tests are repeated for any patient, the last rechecked values will be considered as baselines, provided they were done before the first IMP administration and in the same conditions (e.g. fasting for glucose).

**Abnormalities analyses**

For parameters with laboratory ranges and/or abnormality criteria, an "on-treatment" analysis will be performed using all post-baseline assessments done during the on-treatment phase, including all unplanned and rechecked values. Since laboratory assessments will be performed during screening and at end-of-study visit (EOS), no on-treatment measurements are pre-planned, only unscheduled values can occur during the on-treatment phase.
Data will be analyzed quantitatively using descriptive statistics, qualitatively by tabulating clinical abnormalities using Sponsor or regulatory criteria.
If appropriate, counts of patients with out-of-normal laboratory range values will be provided in summary tables showing shifts from normal and abnormal baselines to post-baseline abnormalities, presented by population and treatment group. The same type of summary tables will be provided for out-of-normal laboratory range values. These tables are split by normal/abnormal status and missing value at baseline (if any).

**Descriptive statistics and plots**

For ALT, AST, ALP, neutrophils, platelets and creatinine, amylase, lipase raw data and changes from baseline (percent change for creatinine) to EOS will be summarized in descriptive statistics, by population and treatment group.

**Listings**

All individual data, for planned urinalysis, hematology and biochemistry, including rechecked values, will be listed by biological function. If any, data from unscheduled laboratory tests will also be listed. In these listings, individual data will be flagged when lower or higher than the lower or upper laboratory limits and/or when reaching the absolute limit of the Sponsor or regulatory criteria, when defined. A listing of out-of-normal range definitions will also be provided.
A listing of liver function data for patients experiencing at least one of the following situations will be provided as an in-text table:

- ALT >3 ULN and total bilirubin > 2 ULN during the study, with at least one of them being post first dose, irrespective of the definition of the on-treatment phase

- Conjugated bilirubin >35% of total bilirubin and total bilirubin >1.5 ULN, on the same sample post first dose, irrespective of the definition for the on-treatment phase.

If any, a listing related to increase in ALT $\geq$ 2 ULN will be provided, including notably the information on IMP intake, medical and surgical history, alcohol habits, trigger factors, event details with ALT values, associated signs and symptoms.

### 13.8.2.2 Urinalysis

All qualitative urinary test results (dipstick) and results from urinary pregnancy test, including rechecked values, will be listed.

### 13.8.3 Vital signs

### 13.8.3.1 Blood pressure and heart rate

Heart rate (HR) and systolic and diastolic blood pressure (SBP and DBP) will be analyzed as raw parameter value and change from baseline (for supine position only), and as orthostatism parameter (standing-supine parameter values, when applicable).

The values to be used as baseline will be the pre-dose measurement on Day 1 of each period. If any of the scheduled baseline tests are repeated for any patient, the last rechecked values will be considered as baseline, provided they were done before IP administration.

Data will be analyzed quantitatively using descriptive statistics, qualitatively by tabulating clinical abnormalities using Sponsor or regulatory criteria.

For heart rate and blood pressures, raw data and changes from each baseline to EOS (for supine position only) will be summarized in descriptive statistics, for each type of measurement and by population and treatment group.

### 13.8.3.2 Weight and body mass index

The values to be used as baselines will be the Day -1 value. Weight will be analyzed as raw parameter value and percent change from baseline. Individual BMI will be calculated for any post-baseline weight assessment time point.

For weight, an "on-treatment" analysis will be performed using all post-baseline assessments done during the on-treatment period, including rechecked values

Individual data for weight and BMI data will be listed.

### 13.8.4 Electrocardiogram

ECG parameters obtained from automatic reading of 12-lead ECG are used to support the safety analysis.

The values to be used as the baseline will be the Day 1 predose value of each period. If any of the scheduled baseline tests are repeated for any patient, the rechecked values will be considered as baselines, provided they were done before the first drug administration of the period.

HR, PR-, QRS-, QT, and corrected QT-interval (QTc) will be analyzed as raw parameter value and change from baseline to EOS.

Data will be analyzed quantitatively using descriptive statistics, qualitatively by tabulating clinical abnormalities using Sponsor or regulatory criteria.

For all parameters, raw data and changes from baseline to EOS will be summarized in descriptive statistics, by population, parameter, population and treatment group.

Individual data for all parameters, including rechecked values, will be listed.

In addition, patients with prolonged QTc (>450 ms) and/or change from baseline in QTc >60 ms will also be listed separately, using all post-dose timepoints.

A listing of patients with at least one abnormality in qualitative assessment (ie, abnormal ECG) after the 1st dosing will be also provided.

### 13.8.5 Other related safety parameters

### 13.8.5.1 Anti-AVE0010 antibodies

Patient listing will be provided for anti-lixisenatide antibodies at baseline. If appropriate, frequency distributions will be provided.

## 13.9 ANALYSIS OF PHARMACOKINETIC DATA

### 13.9.1 Pharmacokinetic parameters

The list of pharmacokinetics parameters is listed in Section 9.3.5.

### 13.9.2 Statistical analysis

Pharmacokinetic parameters of lixisenatide will be summarized by descriptive statistics (such as mean, geometric mean, median, standard deviation (SD), standard error of the mean (SEM), coefficient of variation (CV), (minimum, and maximum) by population and for each treatment under the responsibility of Drug Disposition, Safety and Animal Research, sanofi. Other statistical analyses described below will be performed under the responsibility of Biostatistics, sanofi.

The primary analysis will be based on the evaluable population.

Log- transformed lixisenatide pharmacokinetic parameters Cmax, AUClast, and AUC will be analyzed using a linear mixed effect model with fixed terms for treatment, sequence, period and a random term for a patient-within-sequence. If the number of patients per sequence is too low the model might be adapted by excluding the sequence effect.

Estimates and 90% CI for the geometric mean ratio of 5 $\mu$g lixisenatide and versus 10$\mu$g lixisenatide will be obtained by computing estimate and 90% CI for the difference between treatment means within the linear mixed effects model framework, and then converting to ratio by the antilog transformation to the original scale.

## 13.10 PHARMACOKINETIC/PHARMACODYNAMIC ANALYSIS

If appropriate, an explorative PK/PD analysis of lixisenatide concentrations vs pharmacodynamics will be performed.

## 13.11 INTERIM ANALYSIS

No interim analysis is planned

## 14 ETHICAL AND REGULATORY STANDARDS

## 14.1 ETHICAL PRINCIPLES

This clinical trial will be conducted in accordance with the principles laid down by the 18th World Medical Assembly (Helsinki, 1964) and all applicable amendments laid down by the World Medical Assemblies, and the ICH guidelines for Good Clinical Practice (GCP).

In compliance with sanofi-aventis public disclosure commitments, this clinical trial will be recorded on public registry web sites (eg, clinicaltrials.gov before the enrollment of the first patient). The registry will contain basic information about the trial sufficient to inform interested patients (and their healthcare practitioners) on how to enroll in the trial.

## 14.2 LAWS AND REGULATIONS

This clinical trial will be conducted in accordance with all international guidelines, national laws, and regulations of the country(ies) in which the clinical trial is performed, as well as any applicable guidelines for adults and paediatrics.

## 14.3 INFORMED CONSENT

The Investigator (according to applicable regulatory requirements), or a person designated by the Investigator and under the Investigator's responsibility, should fully inform the patient of all pertinent aspects of the clinical trial including the written information giving approval/favorable opinion by the ethics committee (IRB/IEC) and Health Authorities (according to local regulations). All participants should be informed to the fullest extent possible about the study, in language and terms they are able to understand.

Prior to a patient's participation in the clinical trial, the written informed consent form should be signed, name filled in, and personally dated by the patient or by the patient's legally acceptable representative, and by the person who conducted the informed consent discussion. A copy of the signed and dated written informed consent form will be provided to the patient.

For the children participation, local law must be observed in deciding whether one or both parents/guardians consent is required. If only one parent or guardian signs the consent form, the Investigator must document the reason for only one parent or guardian's signature.

In addition, participants will assent as detailed below or will follow the Ethics Committee (IRB/IEC) approved standard practice for pediatric participants at each participating center (age of assent to be determined by the IRB's/IEC's or be consistent with the local requirements): Participants who can read the Assent Form will do so before writing their name and dating or signing and dating the form.

Participants who can write but cannot read will have the assent form read to them before writing their name on the form.

## 14.4 INSTITUTIONAL REVIEW BOARD/INDEPENDENT ETHICS COMMITTEE (IRB/IEC)

As required by local regulation, the Investigator and/or the Sponsor must submit this clinical trial protocol to the appropriate IRB/IEC and Health Authorities (according to local regulations), and is required to forward to the respective other party a copy of the written and dated approval/favorable opinion of the ethics committee (IRB/IEC) (signed by the chairman with IRB/IEC composition) and Health Authorities (according to local regulations).

The clinical trial (study number, clinical trial protocol title and version number), the documents reviewed (clinical trial protocol, informed consent form, investigator's brochure, Investigator's curricula vitae, etc) and the date of the review should be clearly stated on the written IRB/IEC and Health Authorities (according to local regulations)approval/favorable opinion.

Investigational medicinal product will not be released at the study site and the Investigator will not start the study before the written and dated approval/favorable opinion is/are received by the Investigator and the Sponsor.

During the clinical trial, any amendment or modification to the clinical trial protocol should be submitted to the IRB/IEC and Health Authorities (according to local regulations)before implementation, unless the change is necessary to eliminate an immediate hazard to the patients, in which case the IRB/IEC should be informed as soon as possible. It should also be informed of any event likely to affect the safety of patients or the continued conduct of the clinical trial, in particular any change in safety. All updates to the investigator's brochure will be sent to the IRB/IEC.

A progress report is sent to the IRB/IEC and Health Authorities (according to local regulations)at least annually and a summary of the trial's outcome at the end of the clinical trial.

## BIBLIOGRAPHIC REFERENCES

1. Investigator's Brochure Lixisenatide, Edition No. 8, 01st April 2011

2. Centers for Disease Control and Prevention. National diabetes fact sheet United States, 2003: general information. Available at: http://www.cdc.gov/diabetes/pubs/factsheet.htm. Accessed June 6, 2008.

3. Canadian Diabetes Association. Clinical Practice Guidelines Expert Committee. Canadian Diabetes Association 2008. Clinical Practice Guidelines for the Prevention and Management of Diabetes in Canada. Canadian Journal of Diabetes 2008:S161-S167. http://www.diabetes.ca/files/cpg2008/cpg-2008.pdf

4. Pinhas-Hamiel O., Zeitler P. Clinical presentation and treatment of type 2 diabetes in children. Pediatric Diabetes 2007;8(9):16-27

5. American Diabetes Association. Type 2 diabetes in children and adolescents. Diabetes Care 2000; 23(3); 381-389.

6. IDF Clinical Guidelines Task Force. Global guideline for Type 2 diabetes. Brussels: International Diabetes Federation, 2005

7. Exenatide (marketed as Byetta) information; http:// www.fda.gov/cder/drug/infopage/exenatide/ default.htm

8. Jones KL, Arslanian S, Peterokova VA, Park J-S, Tomlinson MJ: Effect of metformin in pediatric patients with type 2 diabetes: a randomized controlled trial. Diabetes Care 25:89-94, 2002

9. Tanner JM, Davies, PS. Clinical longitudinal standards for height and height velocity for North American children. J Pediatr 1985;107(3):317-329.

10. Tanner JM, Whitehouse RH, Takaishi M. Standards from birth to maturity for height, weight, height velocity, and weight velocity: British children, 1965. II. Arch Dis Child. 1966;41(220):613-635

11. Wolever TMS, Jenkins D. JA, Ocana A.M, Rao VA, Collier G.C. Second-meal effect: low-glycemic -index foods eaten at dinner improve subsequent breakfast glycemic response. Am J CLin Nutr 1988;48:1041-7.

12. Nilsson A, Ostman E, Preston T and Björck. Effects of Gi vs content of cereal fibre of the evening meal on glucose tolearance at a subsequent standardized breakfast. Eur. J Clin Nutr.2008 62, 712-720.

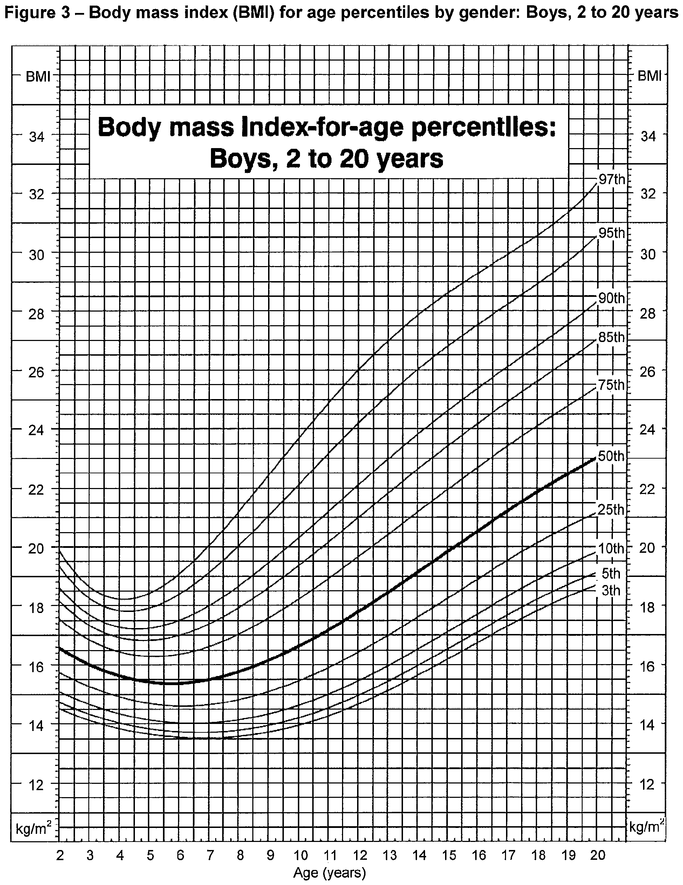

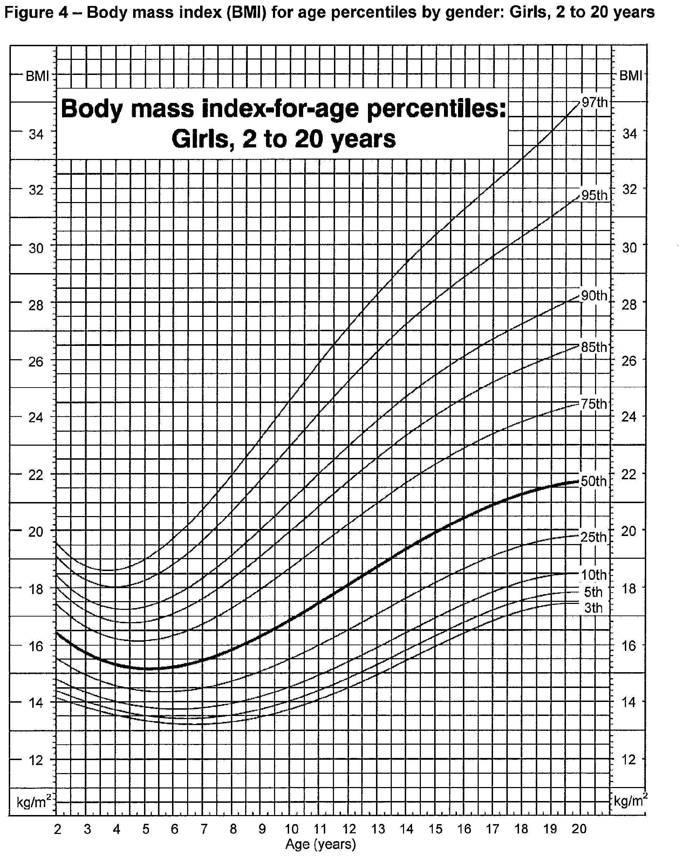

The body mass index (BMI) for age percentiles by gender is shown in Figures 3-4, also referred to as Appendix A. Figure 3 shows the body mass index-for-age percentiles for boys from 2 to 20 years. Figure 4 shows the body mass index-for-age percentiles for girls from 2 to 20 years.

**Appendix B Blood pressure levels by gender, age and height percentile**

| 90th Percentile of Blood Pressure in Boys 2 to 17 Years of Age According to Height Percentile | | | | | | | | | | |
|---|---|---|---|---|---|---|---|---|---|---|
| 90th % Systolic BP for Height Percentile of: | | | | | | 90th % Diastolic BP for Height Percentile of: | | | | |
| Age | 5th | 25th | 50th | 75th | 95th | 5th | 25th | 50th | 75th | 95th |
| 2 | 98 | 100 | 102 | 104 | 105 | 55 | 56 | 57 | 58 | 59 |
| 4 | 102 | 105 | 107 | 109 | 110 | 62 | 63 | 64 | 65 | 66 |
| 6 | 105 | 108 | 110 | 111 | 113 - | 67 | 69 | 70 | 70 | 71 |
| 8 | 107 | 110 | 112 | 114 | 115 | 71 | 72 | 73 | 74 | 75 |
| 10 | 110 | 113 | 115 | 117 | 118 | 73 | 74 | 75 | 76 | 77 |
| 12 | 115 | 117 | 119 | 121 | 123 | 75 | 76 | 77 | 78 | 78 |
| 14 | 120 | 123 | 125 | 126 | 128 | 76 | 77 | 78 | 79 | 80 |
| 16 | 125 | 128 | 130 | 132 | 133 | 79 | 80 | 81 | 82 | 83 |
| 17 | 128 | 131 | 133 | 134 | 136 | 81 | 82 | 83 | 84 | 85 |
| 90th Percentile of Blood Pressure in Girls 2 to 17 Years of Age According to Height Percentile | | | | | | | | | | |
| 90th % Systolic BP for Height Percentile of: | | | | | | 90th % Diastolic BP for Height Percentile of: | | | | |
| Age | 5th | 25th | 50th | 75th | 95th | 5th | 25th | 50th | 75th | 95th |
| 2 | 99 | 100 | 102 | 103 | 104 | 57 | 58 | 58 | 59 | 60 |
| 4 | 101 | 103 | 104 | 106 | 107 | 63 | 64 | 65 | 65 | 66 |
| 6 | 104 | 106 | 107 | 109 | 110 | 67 | 68 | 69 | 69 | 70 |
| 8 | 108 | 110 | 111 | 112 | 113 | 70 | 71 | 71 | 72 | 73 |
| 10 | 112 | 114 | 115 | 116 | 117 | 73 | 73 | 74 | 75 | 76 |
| 12 | 116 | 118 | 119 | 120 | 121 | 75 | 76 | 76 | 77 | 78 |
| 14 | 119 | 121 | 122 | 124 | 125 | 77 | 78 | 79 | 79 | 80 |
| 16 | 122 | 123 | 125 | 126 | 127 | 79 | 79 | 80 | 81 | 82 |
| 17 | 122 | 124 | 125 | 126 | 128 | 79 | 79 | 80 | 81 | 82 |

**Appendix C Calculation of Creatinine-Clearance by Cockroft and Gault**

Male:
$$\text{Creatinine clearance[mL/min]} = \frac{(140 - \text{age [years]}) \times \text{weight [kg]}}{\text{creatinine [mg/dL]} \times 72}$$

Female:
$$\text{Creatinine clearance[mL/min]} = \frac{(140 - \text{age [years]}) \times \text{weight [kg]} \times 0.85}{\text{creatinine [mg/dL]} \times 72}$$

**Appendix D: Meal test (standardized breakfast)**

Ensure Plus Next Generation Vanilla

List of ingredients in descending order:
Water, maltodextrin, hydrolized corn starch, sucrose, milk protein isolate, canola oil, CASEINATES (calcium caseinate, sodium caseinate), corn oil, MINERALS (sodium citrate, potassium citrate, magnesium chloride, potassium chloride, magnesium phosphate dibasic, calcium phosphate tribasic, potassium phosphate dibasic, ferrous sulfate, zinc sulfate, manganese sulfate, cupric sulfate, sodium molybdate, potassium iodide, chromium chloride, sodium selenate), soy protein isolate, flavoring, soy lecithin, cellulose, VITAMINS (choline chloride, ascorbic acid, dl-alpha tocopheryl acetate, niacinamide, calcium pantothenate, pyridoxine hydrochloride, thiamine hydrochloride, riboflavin, Vitamin A palmitate, beta carotene, folic acid, phylloquinone, biotin, Vitamin D3, cyanocobalamin), sodium carboxymethyl cellulose, gellan gum.
May contain: sodium chloride.

Approximate analysis

|  | UNIT | PER 200 ML |
|---|---|---|
| Energy EU kcal | Kcal | 300 |
| Energy EU | kJ | 1263 |
| Protein | 9 | 12.50 |
| Fat | 9 | 9.84 |
| Carbohydrate | 9 | 40.40 |
| Water | 9 | 154.86 |
| VITAMINS |  |  |
| Vitamin A (palmitate) | mcg RE | 175 |
| Vitamin A (palmitate) | IU | 584 |
| Vitamin A ($\beta$-carotene) | mcg RE | 58 |
| Vitamin A ($\beta$-carotene) | IU | 584 |
| Vitamin $D_3$ | mcg | 4.0 |
| Vitamin $D_3$ | IU | 160 |
| Vitamin E | IU | 6.4 |
| Vitamin $K_1$ | mcg | 24 |
| Vitamin C | mg | 24 |
| Folic acid | mcg | 80 |
| Vitamin $B_1$ | mg | 0.40 |
| Vitamin $B_2$ | mg | 0.54 |
| Vitamin $B_6$ | mg | 0.54 |
| Vitamin $B_{12}$ | mcg | 1.1 |
| Niacin equivalent | mg | 5.2 |

(continued)

|  | UNIT | PER 200 ML |
|---|---|---|
| Panthothenic acid | mg | 2.2 |
| Biotin | mcg | 12 |
| Choline | mg | 110 |
| MINERALS |  |  |
| Sodium | mg | 184 |
| Potassium | mg | 320 |
| Chloride | mg | 220 |
| Calcium | mg | 120 |
| Phosphorus | mg | 200 |
| Magnesium | mg | 60 |
| Iron | mg | 4.2 |
| Zinc | mg | 3.6 |
| Manganese | mg | 1.0 |
| Copper | mcg | 360 |
| Iodine | mcg | 44 |
| Selenium | mcg | 17 |
| Chromium | mcg | 15 |
| Molybdenum | mcg | 32 |

**Example 2**

**A randomized, double-blind, placebo controlled trial to assess safety, tolerability, pharmacokinetics and pharmacodynamics of lixisenatide in paediatric (10-17 year old) and adult patients with type 2 diabetes**

| **Sponsor** / **Company:** Sanofi<br>**Drug substance(s):** Lixisenatide (AVE0010) | |
|---|---|
| **Title of the study:** | A randomized, double-blind, placebo controlled trial to assess safety, tolerability, pharmacokinetics and pharmacodynamics of lixisenatide in paediatric (10-17 years old) and adult patients with type 2 diabetes |
| **Study center(s):** | Six centers from 4 countries (pediatric patients from 4 centers in Mexico, South Africa, and the United States [US] and adult patients from 2 centers in the US and United Kingdom) |
| **Study period:**<br>    Date first patient enrolled: 24/May/2012<br>    Date last patient completed: 04/Mar/2014 | |
| **Phase of development:** Phase 1 | |
| **Objectives:**<br>*Primary objective:* | |

(continued)

| | |
|---|---|
| • | To investigate the effects of a single subcutaneous (SC) lixisenatide dose of 5 $\mu$g and 10 $\mu$g as compared to placebo in reducing postprandial plasma glucose (PPG) assessed as area under the plasma glucose concentration curve after a standardized liquid meal (breakfast) in type 2 diabetic pediatric population (10-17 years old) and adults as controls. |

*Secondary objectives:*

To evaluate in both pediatric and adult populations:

| | |
|---|---|
| • | Pharmacokinetic (PK) parameters of lixisenatide in plasma after single SC ascending doses. |
| • | The maximum PPG excursion, and the changes in insulin, C-peptide, and glucagon plasma concentrations following a standardized breakfast. |
| • | Safety and tolerability |

**Methodology:** Multicenter, double-blind, randomized, placebo-controlled, single-dose, 3-period, 3-treatment, 6-sequence crossover study in pediatric and adult patients with type 2 diabetes mellitus (T2DM)

**Number** of patients: Planned: 12 pediatric patients/12 adult patients

Randomized: 12 pediatric patients/13 adult patients

Treated: 12 pediatric patients/12 adult patients

**Evaluated:**

**Overview of study populations**

| | Pediatric patients | Adult patients |
|---|:---:|:---:|
| Number of patients for: | | |
| Evaluable pharmacodynamics population (N) | 9[a] | 12 |
| Full analysis pharmacodynamics population (N) | 12 | 12 |
| Evaluable pharmacokinetics population (N) | 8b | 10[b] |
| Full analysis pharmacokinetics population (N) | 12 | 12 |
| Safety population (N) | 12 | 12 |

| | |
|---|---|
| a | Three patients excluded: 2 patients had vomiting within 4 hours after the standardized meal test and 1 patient ingested only half of the standardized meal test. |
| b | Four pediatric and 2 adult patients excluded: lixisenatide plasma concentrations below lower limit of quantification (LLOQ) in all samples in at least one period or no more than 3 consecutive samples above LLOQ in at least 1 period. |

**Diagnosis and criteria for inclusion:**

Male and female patients with T2DM, with or without metformin (at a stable dose for at least 4 weeks prior to randomization); $HbA_{1c} \geq 7\%$ and $\leq 10\%$ at screening; fasting C-peptide >0.6 ng/mL at screening; negative test for anti-insulinoma-associated protein and anti-glutamic acid decarboxylase autoantibodies.

Pediatric population: Male and female patients $\geq 10$ and <18 years of age with at least 3 patients below 15 years of age and no more than 3 patients $\geq 16$ and <18 years of age, body mass index (BMI) >85th percentile for age and gender, and BMI $\leq 50$ kg/m$^2$ (body weight >50 kg)

Adult population: Male and female patients $\geq 18$ and $\leq 65$ years of age, and with BMI >25 kg/m$^2$ and $\leq 37$ kg/m$^2$.

**Study treatments**

**Investigational medicinal product(s):** Lixisenatide and placebo

Formulation: Lixisenatide (100 $\mu$g/mL) and placebo, provided as solutions for injection in a 3-mL glass cartridge

Route(s) of administration: SC injection with pen-type injector (OptiClik®)

Dose regimen: In each of the 3 treatment periods, patients were administered, in fasted conditions, a single dose of 5 $\mu$g lixisenatide or 10 $\mu$g lixisenatide (with 5 $\mu$g preceding the 10 $\mu$g dose level) or placebo (50 or 100 $\mu$L), 30 minutes before a standardized liquid breakfast.

(continued)

| |
|---|
| **Duration of treatment:** Three treatment periods, each lasting 1 day (up to 2 days in case of institutionalization on the evening of Day-1).<br>**Duration of observation:** Up to 7 weeks for each patient including a screening period of up to 28 days, 3 treatment periods of up to 2 days separated each by a washout period of 1 to 7 days and an end-of-study visit 1 to 6 days after the last investigational medicinal product (IMP) administration. |

**Criteria for evaluation:**

**Pharmacodynamics:**

*Primary endpoint:*

- Plasma glucose: corrected plasma glucose-$AUC_{0:30h-4:30h}$: area under the curve for plasma glucose concentration-time profile calculated from time of standardized breakfast start (30 minutes after IMP injection and premeal plasma glucose=T0H30) until 4 hours later (T4H30) after subtracting the premeal value (T0H30)

*Secondary endpoints:*

- PPG-excursion$_{0:30h-4:30h}$: maximum change in PPG from time of standardized breakfast start (30 minutes after IMP injection=T0H30) until 4 hours later (T4H30)

- $AUC_{0:30h-4:30h}$ of plasma glucose, insulin, C-peptide, and glucagon: area under the curve for plasma glucose, insulin, C-peptide or glucagon concentration-time profiles from time of standardized breakfast start (30 minutes after IMP injection=T0H30) until 4 hours later (T4H30)

Safety: Patients were monitored for safety via adverse events (AEs) reported by the patient or noted by the Investigator, physical examination, body temperature, standard clinical laboratory evaluations, vital signs, and electrocardiogram (ECG) parameters.

**Pharmacokinetics:** Lixisenatide plasma concentration, PK parameters (maximum plasma concentration observed [$C_{max}$], time to reach Cmax [$t_{max}$], area under the plasma concentration versus time curve calculated using the trapezoidal method from time zero to the real time [$AUC_{last}$], area under the plasma concentration versus time curve extrapolated to infinity [AUC], area under the plasma concentration versus time calculated using the trapezoidal method from time T0H30 to T4H30 [$AUC_{0:30h-4:30h}$]).

**Pharmacokinetic/Pharmacodynamic sampling times and bioanalytical methods:**

Blood samples for pharmacodynamic (PD) analysis were collected at each treatment period for plasma glucose, glucagon, insulin and C-peptide assessments: blood samples were taken 30 minutes before a standardized breakfast and prior to dosing (T0), then immediately prior to the standardized breakfast (T0H30 hours), and thereafter at T1, T1H30, T2, T2H30, T3H30, and T4H30 (ie, 30, 60, 90, 120, 180, and 240 minutes postbreakfast) for $AUC_{0:30h-4:30h}$ for plasma glucose, glucagon, insulin, and C-peptide measurements.

The quantitative analysis of plasma glucose was assessed using the Gluco-quant Glucose/hexokinase assay for glucose from Roche Diagnostics, Mannheim, Germany. The range of the method was 3-1000 mg/dL, with 1 mg/dL as limit of detection (LOD), 3 mg/dL as lower limit of quantification (LLOQ), and 1000 mg/dL as upper limit of quantification.

The method for quantitative analysis for human C-peptide was assessed using the Electro Chemiluminescence Immuno Assay (ECLIA) from Roche Diagnostics, Mannheim, Germany. The range of the method was 0.2-25 ng/mL, with an LLOQ of 0.2 ng/mL and an LOD of 0.07 ng/mL.

The method for quantitative analysis of glucagon was assessed using the radioimmunoassay (RIA) from Euro-Diagnostica, Malmö, Sweden. The range of the method was 4.7-150 pmol/L.

The method for quantitative analysis of insulin was assessed using the ECLIA assay from Roche Diagnostics Deutschland GmbH, Mannheim, Germany. The range of the method was 1-875 mIU/L, with an LLOQ of 1 mIU/L and an LOD of 0.3 mIU/L.

Blood samples for PK analysis were collected at each treatment period for the determination of lixisenatide plasma concentrations: blood samples were taken 30 minutes before a standardized breakfast and prior to dosing (T0), and thereafter at T0H30, T1, T1H30, T2H30, T3H30, T4H30, and T6H30.

Lixisenatide plasma concentrations were determined using a validated double-antibody sandwich enzyme-linked immunosorbent assay method with an LLOQ of 5.5 pg/mL.

(continued)

| Anti-lixisenatide antibody status and, if positive, anti-lixisenatide antibody concentrations were determined using the validated BIAcore technique with a study-specific, and thus not prospectively determined, cutoff as LLOQ. Blood samples were taken only on Day 1/Period 1 before the first IMP administration. |
| --- |

**Statistical methods:**

Pediatric and adult patients were analyzed separately. Results were compared between the 2 populations descriptively.

*Pharmacodynamics:*

Within each crossover, the analyses of the primary PD endpoint were performed based on the evaluable PD population, using the full analysis PD population as supportive analyses. Corrected plasma glucose $AUC_{0:30h-4:30h}$ was analyzed using a linear mixed-effect model with sequence, period, and treatment effect as fixed effects, and patient within sequence as random effect, and the T0H30 plasma glucose concentration as covariate. The least square (LS) mean differences between treatment groups and the corresponding 95% confidence intervals (CIs) were estimated within the linear mixed model framework. A significance level of $p < 0.05$ was used.

Secondary PD parameters were analyzed using the same statistical model as described above with the corresponding T0H30 values as covariates.

*Pharmacokinetics:*

The statistical analyses of PK parameters were done on the evaluable PK population, using the full analysis PK population as supportive analyses.

Log-transformed lixisenatide PK parameters $C_{max}$, $AUC_{last}$, and $AUC_{0:30h-4:30h}$ were analyzed using a linear mixed-effect model with fixed terms for sequence, treatment and a random term for a patient-within-sequence. Estimates and 90% CIs for the geometric mean ratio of lixisenatide 10 $\mu$g versus lixisenatide 5 $\mu$g were obtained by computing estimate and 90% CIs for the difference between treatment means within the linear mixed-effects model framework, and then converting to ratio by the antilog transformation to the original scale.

*Safety:*

The safety analysis was based on the review of the individual values (clinically significant abnormalities) and descriptive statistics (summary tables and plots if appropriate) by treatment.

Treatment-emergent adverse events (TEAEs) classified in system organ classes (SOCs) and preferred terms were summarized by number and percentage of patients and number of TEAEs. Individual clinical laboratory data, vital signs, and ECG data were listed and flagged for potentially clinically significant abnormalities (PCSAs) and for lower and upper clinical laboratory limits. Frequency of patients with abnormalities and with on-treatment PCSAs were summarized for each type of parameter by treatment.

**Summary:**

**Population characteristics:**

Twelve pediatric and 12 adult patients with T2DM were randomized and treated. One additional adult patient was randomized but not treated (this patient withdrew from the study due to personal reasons before the first IMP administration). All patients were on concomitant metformin therapy during the study.

Demographics and baseline characteristics for pediatric and adult patients are summarized in the table below.

**Demographics, patient, and disease characteristics at baseline in pediatric and adult patients, safety population**

|  | Pediatric patients | Adult patients |
| --- | --- | --- |
| N | 12 | 12 |
| Mean age (years) [min-max] | 13.9 [10-17] | 51.3 [41-60] |
| Age group (years) (n, %) | | |
| [10-15] | 7 (58.3%) | |
| [15-16] | 2 (16.7%) | |
| [16-18] | 3 (25.0%) | |
| [18-50] | | 5 (41.7%) |
| [50-65] | | 7 (58.3%) |
| Sex (n [%]) Male | 6 (50%) | 9 (75%) |

(continued)

| | | 6(50%) | 3 (25%) |
|---|---|---|---|
| Female | | | |
| Race (n [%]) | | | |
| Caucasian/white | | 1 (8.3%) | 6 (50%) |
| Asian/oriental | | | 1 (8.3%) |
| Other[a] | | 11 (91.7%) | 5 (41.7%) |
| Mean weight (kg) [min-max] | | 84.69 [56.0-129.0] | 92.58 [74.7-135.3] |
| Mean BMI $(kg/m^2)$ [min-max] | | 31.42 [22.7-44.1] | 31.79 [27.0-36.1] |
| Duration of diabetes (years): median [min-max] | | 1.56 [0.5-7.9] | 4.45 [1.9-20.4] |
| Duration of metformin treatment (years): median [min-max] | | 1.56 [0.5-7.6] | 2.13 [0.4-7.4] |
| Mean $HbA_{1c}$ (%) [min-max] | | 8.65 [7.0-9.9] | 8.43 [7.2-9.1] |

| | | |
|---|---|---|
| a | | Among 11 pediatric patients, 7 self-reported as Hispanic and 4 self-reported as a group of mixed race in South Africa (the Cape Colored), Five adult patients self-reported as Hispanic or Latino. |

**Pharmacodynamic results:**

*Primary pharmacodynamic endpoints:*

In the pediatric evaluable PD population, the corrected plasma glucose-$AUC_{0:30h-4:30h}$ was decreased by single doses of lixisenatide 5 and 10 µg compared to placebo, but the differences versus placebo were not statistically significant. For the primary endpoint (corrected plasma glucose-$AUC_{0:30h-4:30h}$), the LS mean difference between the lixisenatide 5 µg dose and placebo was -3.92 mmol.h/L; 95% CI: -8.17 to 0.34 mmol.h/L, p=0.0681 (-70.56 mg.h/dL; 95% CI: -147.15 to 6.04 mg.h/dL). The LS mean difference between lixisenatide 10 µg and placebo was -1.52 mmol.h/L; 95% CI: -5.59 to 2.56 mmol.h/L, p=0.4359 (-27.33 mg.h/dL; 95% CI: -100.75 to 46.10 mg.h/dL) (see tables below).

**Pediatric patients - plasma glucose premeal corrected $AUC_{0:30h·4:30h}$ (mmol.h/L) per treatment group and difference of lixisenatide 5 µg and 10 µg to placebo - evaluable PD population**

| Least Square Means (SE)[a] | | | | | |
|---|---|---|---|---|---|
| Treatment group | N | Corrected plasma glucose-$AUC_{0:30-4:30h}$ [mmol.h/L] | Corrected plasma glucose-$AUC_{0:30-4:30h}$ difference to placebo [mmol.h/L] | 95% CI of difference [mmol.h/L] | p-value |
| Placebo | 9 | 9.63 (3.95) | | | |
| Lixisenatide 5 µg | 9 | 5.72 (3.99) | -3.92 (1.97) | (-8.17; 0.34) | 0.0681 |
| Lixisenatide 10 µg | 9 | 8.11 (4.08) | -1.52 (1.89) | (-5.59; 2.56) | 0.4359 |

a SE (standard error)

Pediatric patients - plasma glucose premeal corrected $AUC_{0:30h-4:30h}$ (mg.h/dL) per treatment group and difference of lixisenatide 5 µg and 10 µg to placebo - evaluable PD population

| Least Square Means (SE)[a] |
|---|

(continued)

| Treatment group | N | Corrected plasma glucose-$AUC_{0:30-4:30h}$ [mg.h/dL] | Corrected plasma glucose-$AUC_{0:30-4:30h}$ difference to placebo [mg.h/dL] | 95% CI of difference [mg.h/dL] | p-value |
|---|---|---|---|---|---|
| Placebo | 9 | 173.51 (71.24) | | | |
| Lixisenatide 5 μg | 9 | 102.96 (71.81) | -70.56 (35.46) | (-147.15; 6.04) | 0.0681 |
| Lixisenatide 10 ug | 9 | 146.19 (73.44) | -27.33 (34.00) | (-100.75; 46.10 | 0.4359 |

a SE (standard error)

In contrast to pediatric patients, in the adult evaluable PD population, single doses of lixisenatide 5 and 10 μg significantly reduced PPG assessed as corrected plasma glucose-$AUC_{0:30h:30h}$ compared to placebo. The LS mean difference between lixisenatide 5 μg dose and placebo was -8.57 mmol.h/L; 95% CI: -14.91 to -2.23 mmol.h/L, p=0.0104 (-154.41 mg.h/dL; 95% CI: -268.60 to -40.21 mg.h/dL). The LS mean difference between lixisenatide 10 μg and placebo was -15.48 mmol.h/L; 95% CI: -21.59 to -9.38 mmol.h/L, p<0.0001 (-278.93 mg.h/dL; 95% CI: -388.96 to -168.90 mg.h/dL) (see tables below). The difference between lixisenatide 10 and 5 μg was not statistically significant.

**Adult patients - plasma glucose premeal corrected $AUC_{0:30h-4:30h}$ (mmol.h/L) per treatment group and difference of lixisenatide 5 μg and 10 μg to placebo - evaluable PD population**

| Least Square Means (SE)[a] | | | | | |
|---|---|---|---|---|---|
| Treatment group | N | Corrected plasma glucose-$AUC_{0:30-4:30h}$ [mmol.h/L] | Corrected plasma glucose-$AUC_{0:30-4:30h}$ difference to placebo [mmol.h/L] | 95% CI of difference [mmol.h/L] | p-value |
| Placebo | 12 | 16.60 (2.46) | | | |
| Lixisenatide 5 μg | 12 | 8.03 (2.95) | -8.57 (3.05) | (-14.91 ; -2.23) | 0.0104 |
| Lixisenatide 10 μg | 12 | 1.11 (2.85) | -15.48 (2.93) | (-21.59 ; -9.38) | <0.0001 |

a SE (standard error)

**Adult patients - plasma glucose premeal corrected $AUC_{0:30h-4:30h}$ (mg.h/dL) per treatment group and difference of lixisenatide 5 μg and 10 μg to placebo - evaluable PD population**

| Least Square Means (SE)[a] | | | | | |
|---|---|---|---|---|---|
| Treatment group | N | Corrected plasma glucose-$AUC_{0:30-4:30h}$ [mg.h/dL] | Corrected plasma glucose-$AUC_{0:30-4:30h}$ difference to placebo [mg.h/dL] | 95% CI of difference [mg.h/dL] | p-value |
| Placebo | 12 | 299.01 (44.36) | | | |
| Lixisenatide 5 μg | 12 | 144.60 (53.18) | -154.41 (54.99) | (-268.60; -40.21) | 0.0104 |

(continued)

| | | | | | | |
|---|---|---|---|---|---|---|
| Lixisenatide 10 μg | 12 | 20.08 (51.37) | -278.93 (52.81) | (-388.96 ; -168.90) | <0.0001 |

a SE (standard error)

*Secondary pharmacodynamic endpoints:*

In the pediatric evaluable PD population, the results for plasma glucose $AUC_{0:30h-4:30h}$ were consistent with those for the primary endpoint (corrected plasma glucose-$AUC_{0:30h-4:30h}$). Single dose of lixisenatide 5 μg significantly reduced the maximum PPG excursion compared to placebo: the LS mean difference between lixisenatide 5 μg and placebo was -1.50 mmol/L; 95% CI: -2.94 to -0.07 mmol/L, p=0.0415 (-27.08 mg/dL; 95% CI: -52.95 to -1.22 mg/dL). The difference between lixisenatide 10 μg and placebo was not statistically significant: the LS mean difference was -1.13 mmol/L; 95% CI: -2.50 to 0.25 mmol/L, p=0.1005 (-20.30 mg/dL; 95% CI: -45.09 to 4.50 mg/dL).

In the pediatric evaluable PD population, the $AUC_{0:30h-4:30h}$ for glucagon, insulin, and C-peptide were decreased with both lixisenatide 5 and 10 μg compared to placebo except for insulin that increased with lixisenatide 5 μg; however, the variability was high (see tables below). The differences between lixisenatide 5 or 10 μg and placebo were not statistically significant for any of these endpoints, except the decrease in glucagon with lixisenatide 10 μg. No statistically significant differences between lixisenatide doses were observed for any secondary endpoint in the pediatric evaluable PD population.

In the adult evaluable PD population, the results for plasma glucose $AUC_{0:30h-4:30h}$ were consistent with those for the primary endpoint (corrected plasma glucose-$AUC_{0:30h-4:30h}$). Single doses of lixisenatide 5 and 10 μg significantly reduced the maximum PPG excursion during the postprandial period up to 4 hours after the standardized breakfast, compared to placebo. The LS mean difference between lixisenatide 5 μg and placebo was -2.78 mmol/L; 95% CI: -4.29 to -1.27 mmol/L, p=0.0010 (-50.06 mg/dL; 95% CI: -77.27 to -22.86 mg/dL), and the LS mean difference between lixisenatide 10 μg and placebo was -4.32 mmol/L; 95% CI: -5.77 to -2.87 mmol/L, p<0.0001 (-77.85 mg/dL; 95% CI: -103.95 to -51.76 mg/dL).

In the adult evaluable PD population, the $AUC_{0:30h-4:30h}$ for glucagon, insulin, and C-peptide were decreased with both lixisenatide 5 and 10 μg compared to placebo, and these decreases were statistically significant with lixisenatide 10 μg (see table below). The decreases in $AUC_{0:30h-4:30h}$ for glucagon and C-peptide were not statistically significantly different between lixisenatide doses. The decrease in $AUC_{0:30h-4:30h}$ for insulin with lixisenatide 10 μg compared to lixisenatide 5 μg was statistically significant: the LS mean difference was -378.97 pmol.h/L; 95% CI: -711.56 to -46.38 pmol.h/L, p=0.0277 (-63.16 mcIU.h/mL; 95% CI: -118.59 to -7.73 mcIU.h/mL).

Pediatric patients - $AUC_{0:30h-4:30h}$ for plasma glucose, glucagon, insulin, and C-peptide per treatment group and difference between lixisenatide 5 and 10 μg to placebo (SI units) - evaluable PD population

| Least Square Means (SE)[a] | | | | | | |
|---|---|---|---|---|---|---|
| **Parameter** | **Treatment group** | **N** | **$AUC_{0:30-4:30h}$** | **Difference to placebo** | **95% CI of difference** | **p-value** |
| Plasma glucose (mmol.h/L) | Placebo | 9 | 44.50 (3.91) | | | |
| | Lixisenatide 5 μg | 9 | 40.53 (3.94) | -3.97 (1.93) | (-8.13; 0.19) | 0,0599 |
| | Lixisenatide 10 μg | 9 | 42.94 (4.03) | -1.56 (1.85) | (-5.55; 2.43) | 0.4147 |
| Glucagon (ng.h/L) | Placebo | 9 | 664.83 (19.92) | | | |
| | Lixisenatide 5 μg | 8 | 652.63 (22.22) | -12.20 (21.35) | (-58.05; 33.65) | 0.5769 |
| | Lixisenatide 10 μg | 9 | 621.48 (20.77) | -43.35 (18.30) | (-83.25 ;-3.45) | 0.0356 |
| Insulin (pmol.h/L) | Placebo | 7 | 1843.81 (297.88) | | | |
| | Lixisenatide 5 μg | 8 | 1973.88 (243.52) | 130.07 (372.42) | (-668.69; 928.83) | 0.7321 |
| | Lixisenatide 10 μg | 8 | 1602.80 (239,93) | -241.01 (365.37) | (-1024.64 ; 542.63) | 0.5202 |

(continued)

| | | | | | | |
|---|---|---|---|---|---|---|
| C-peptide (nmol.h/L) | Placebo | 8 | 9.92 (0.56) | | | |
| | Lixisenatide 5 μg | 8 | 9.87 (0.59) | -0.04 (0.80) | (-1.79 ;1.71) | 0.9565 |
| | Lixisenatide 10 μg | 8 | 9.21 (0.58) | -0.70 (0.74 | (-2.35; 0.94) | 0.3631 |

a SE (standard error)

**Pediatric patients - AUC$_{0:30h-4:30h}$ for plasma glucose, glucagon, insulin, and C-peptide per treatment group and difference between lixisenatide 5 and 10 μg to placebo (US units) - evaluable PD population**

| Least Square Means (SE)[a] | | | | | | |
|---|---|---|---|---|---|---|
| Parameter | Treatment group | N | AUC$_{0:30-4:30h}$ | Difference to placebo | 95% CI of difference | p-value |
| Plasma glucose (mg.h/dL) | Placebo | 9 | 801.63 (70.40) | | | |
| | Lixisenatide 5 μg | 9 | 730.11 (70.95) | -71.52 (34.71) | (-146.51; 3.47) | 0.0600 |
| | Lixisenatide 10 μg | 9 | 773.58 (72.53) | -28.04 (33.29) | (-99.92; 43.84) | 0.4147 |
| Glucagon (pg.h/mL) | Placebo | 9 | 664.83 (19.92) | | | |
| | Lixisenatide 5 μg | 8 | 652.63 (22.22) | -12.20 (21.35) | (-58.05; 33.65) | 0.5769 |
| | Lixisenatide 10 μg | 9 | 621.48 (20.77) | 43.35 (18.30) | (-83.25; -3.45) | 0.0356 |
| Insulin (mclU.h/mL) | Placebo | 7 | 307.30 (49.65) | | | |
| | Lixisenatide 5 μg | 8 | 328.98 (40.59) | 21.68 (62.07) | (-111.45; 154.80) | 0.7321 |
| | Lixisenatide 10 μg | 8 | 267.13 (39.99) | -40.17 (60.89) | (-170.77; 90.44) | 0.5202 |
| C-peptide (ng.h/mL) | Placebo | 8 | 29.78 (1.69) | | | |
| | Lixisenatide 5 μg | 8 | 29.65 (1.76) | -0.13 (2.41) | (-5.39; 5.12) | 0.9565 |
| | Lixisenatide 10 μg | 8 | 27.67 (1.76) | -2.11 (2.22) | (-7.05; 2.82) | 0.3631 |

a SE (standard error)

**Adult patients -AUC$_{0:30h-4:30h}$ for plasma glucose, glucagon, insulin, and C-peptide per treatment group and difference between lixisenatide 5 and 10 μg to placebo (SI units) - evaluable PD population**

| Least Square Means (SE)[a] | | | | | | |
|---|---|---|---|---|---|---|
| Parameter | Treatment group | N | AUC$_{0:30-4:30h}$ | Difference to placebo | 95% CI of difference | p-value |
| Plasma glucose (mmol.h/L) | Placebo | 12 | 54.32 (2.46) | | | |
| | Lixisenatide 5 μg | 12 | 45.75 (2.95) | -8.57 (3.05) | (-14.91; -2.23) | 0.0104 |
| | Lixisenatide 10 μg | 12 | 38.83 (2.85) | -15.48 (2.93) | (-21.59; -9.38) | <0.0001 |
| Glucagon (ng.h/L) | Placebo | 12 | 628.98 (26.47) | | | |
| | Lixisenatide 5 μg | 12 | 612.44 (27.90) | -16.54 (18.48) | (-55.53; 22.46) | 0.3834 |

(continued)

| | | | | | | |
|---|---|---|---|---|---|---|
| | Lixisenatide 10 μg | 12 | 575.30 (27.95) | -53.68 (18.59) | (-92.89; -14.46) | 0.0102 |
| Insulin (pmol.h/L) | Placebo | 12 | 1276.36 (85.63) | | | |
| | Lixisenatide 5 μg | 11 | 1181.62 (103.75) | -94.74 (124.99) | (-356.57; 167.09) | 0.4579 |
| | Lixisenatide 10 μg | 12 | 802.65 (104.20) | -473.71 (126.74) | (-738.96; -208.45) | 0.0014 |
| C-peptide (nmol.h/L) | Placebo | 12 | 8.90 (0.48) | | | |
| | Lixisenatide 5 μg | 11 | 8.42 (0.56) | -0.47 (0.64) | (-1.81; 0.87) | 0.4701 |
| | Lixisenatide 10 μg | 12 | 6.81 (0.56) | -2.09 (0.63) | (-3.40; -0.77) | 0.0036 |

a SE (standard error)

**Adult patients - AUC$_{0:30h-4:30h}$ for plasma glucose, glucagon, insulin, and C-peptide per treatment group and difference between lixisenatide 5 and 10 μg to placebo (US units) - evaluable PD population**

| | | | Least Square Means (SE)[a] | | | |
|---|---|---|---|---|---|---|
| Parameter | Treatment group | N | AUC$_{0:30-4:30h}$ | Difference to placebo | 95% CI of difference | p-value |
| Plasma | Placebo | 12 | 978.50 (44.36) | | | |
| glucose (mg.h/dL) | Lixisenatide 5 μg | 12 | 824.10 (53.18) | -154.41 (54.99) | (-268.60 ; | 0.0104 |
| | Lixisenatide 10 μg | 12 | 699.58 (51.37) | -278.93 (52.81) | -40.21) (-388.96 ; -168.90) | <0.0001 |
| Glucagon (pg.h/mL) | Placebo | 12 | 628.98 (26.47) | | | |
| | Lixisenatide 5 μg | 12 | 612.44 (27.90) | -16.54 (18.48) | (-55.53 ; 22.46) | 0.3834 |
| | Lixisenatide 10 μg | 12 | 575.30 (27.95) | -53.68 (18.59) | (-92.89 ; -14.46) | 0.0102 |
| Insulin (mclU.h/mL) | Placebo | 12 | 212.73 (14.27) | | | |
| | Lixisenatide 5 μg | 11 | 196.94 (17.29) | -15.79 (20.83) | (-59.43 ; 27.85) | 0.4579 |
| | Lixisenatide 10 μg | 12 | 133.77 (17.37) | -78.95 (21.12) | (-123.16 ; -34.74) | 0.0014 |
| C-peptide (ng.h/mL) | Placebo | 12 | 26.71 (1.45) | | | |
| | Lixisenatide 5 μg | 11 | 25.30 (1.69) | -1.42 (1.92) | (-5.43 ; 2.60) | 0.4701 |
| | Lixisenatide 10 μg | 12 | 20.45 (1.67) | -6.27 (1.88) | (-10.21 ; -2.32) | 0.0036 |

a SE (standard error)

**Pharmacokinetic results:**

Lixisenatide plasma concentrations were below LLOQ in all samples from 2 pediatric patients treated with lixisenatide 10 μg and 1 adult patient treated with lixisenatide 5 μg. For 1 pediatric and 1 adult patient treated with lixisenatide 5 μg and 1 pediatric patient treated with lixisenatide 10 μg, no more than 3 consecutive samples were above LLOQ and therefore these patients were not evaluable for PK analysis.

(continued)

In the pediatric evaluable PK population, the exposure of lixisenatide was similar for both dose groups. A high variability was observed with lixisenatide 10 $\mu$g. For $C_{max}$, the coefficient of variation (CV%) was 47.7% for lixisenatide 5 $\mu$g and 74.3% for lixisenatide 10 $\mu$g. For $AUC_{last}$, the CV% was 78.2% for lixisenatide 5 $\mu$g and 101.1% for lixisenatide 10 $\mu$g. In the pediatric evaluable PK population, the point estimate of the treatment ratio (lixisenatide 10 $\mu$g versus lixisenatide 5 $\mu$g) for Cmax was 1.04 (90% CI: 0.71 to 1.51) and for $AUC_{last}$ was 0.88 (90% CI: 0.51 to 1.49).

In the pediatric full PK population, the exposure was slightly higher in patients treated with lixisenatide 10 $\mu$g compared to treatment with lixisenatide 5 $\mu$g. A high variability was observed for both dose groups. For $C_{max}$, the coefficient of variation (CV%) was 61.7 for lixisenatide 5 $\mu$g and 72.1 for lixisenatide 10 $\mu$g. For $AUC_{last}$, the CV% was 92.5 for lixisenatide 5 $\mu$g and 97.4 for lixisenatide 10 $\mu$g.

Following single-dose SC administration in adult patients, the exposure of lixisenatide increased with the dose, and was proportional with dose for the evaluable and full PK population.

In pediatric patients, the exposure was similar to that in adults treated with lixisenatide 5 $\mu$g, but lower than in adults treated with lixisenatide 10 $\mu$g.

Pharmacokinetic parameters for lixisenatide in plasma - evaluable PK populations
Plasma Lixisenatide

| **Mean $\pm$ SD** (Geometric Mean) [CV%] | **Paediatric** | | **Adults** | |
|---|---|---|---|---|
| | Lixisenatide 5 $\mu$g | Lixisenatide 10 $\mu$g | Lixisenatide 5 $\mu$g | Lixisenatide 10 $\mu$g |

Pharmacokinetic parameters for lixisenatide in plasma - evaluable PK populations
Plasma Lixisenatide

| **Mean $\pm$ SD** (Geometric Mean) [CV%] | **Paediatric** | | **Adults** | |
|---|---|---|---|---|
| | Lixisenatide 5 $\mu$g | Lixisenatide 10 $\mu$g | Lixisenatide 5 $\mu$g | Lixisenatide 10 $\mu$g |
| N | 8 | 8 | 10 | 10 |
| Cmax (pg/mL) | 29.7 $\pm$ 14.2 (26.3) [47.7] | 34.3 $\pm$ 25.4 (27.2) [74.3] | 26.0 $\pm$ 15.4 (22.8) [59.4] | 56.9 $\pm$ 21.3 (53.3) [37.5] |
| $t_{max}$[a] (h) | 1.25 (0.48 - 3.50) | 0.49 (0.48 - 3.55) | 1.50 (0.42 - 3.50) | 2.50 (0.42 - 3.50) |
| $t_{1/2z}$ (h) | 3.19 $\pm$ 1.12 (3.01) [35.1][b] | 2.52 $\pm$ 0.775 (2.41) [30.8][c] | 3.10 $\pm$ 1.22 (2.89) [39.3] | 2.79 $\pm$ 1.35 (2.59) [48.1] |
| $AUC_{last}$ (pg·h/mL) | 99.4 $\pm$ 77.7 (76.9) [78.2] | 108 $\pm$ 109 (67.4) [101.1] | 101 $\pm$ 58.0 (90.8) [57.3] | 242 $\pm$ 90.0 (228) [37.2] |
| $AUC_{0:30h\text{-}4:30h}$ (pg·h/mL) | 82.5 $\pm$ 54.6 (67.4) [66.2][b] | 88.0 $\pm$ 76.0 (64.3) [86.4][c] | 77.2 $\pm$ 42.4 (70.0) [54.9] | 181 $\pm$ 71.9 (168) [39.6] |

[a] Median (Min - Max)

evaluable paediatric population subjects: 484001004 - 006, 484001008, 484001010, 710002001, 710002005, 710002009

[b] N= 7 for subject 710002005 missing could not be calculated

[c] N= 7 for subject 710002009 missing could not be calculated

evaluable adult population: subjects 826001004, 826001021, 840005006, 840005010-011, 840005014, 840005016 - 017, 840005020-021

Source = PKS Study: PKD11475; Scenario: P-D-A-EV-OD, Version 1, P-D-A-EV-OD-E02, Version 3

| **Point estimates of treatment ratios of lixisenatide 10 $\mu$g versus 5 $\mu$g - evaluable PK population** | | |
|---|---|---|
| Point estimate ratio [90% CI] | Pediatric | Adults |
| N | 8 | 10 |
| Cmax | 1.04 [0.71 -1.51] | 2.34 [1.85 - 2.95] |

(continued)

| | | |
|---|---|---|
| AUC$_{last}$ | 0.88 [0.51 - 1.49] | 2.51 [1.90 - 3.30] |
| AUC$_{0:30-4:30h}$ | 0.93 [0.57 -1.50] | 2.41 [1.88 - 3.08] |

**Safety results:**

No serious AEs were reported during the study, and no patient discontinued the study due to TEAEs. In the pediatric population, 4 patients (1 after injection of placebo, 1 after lixisenatide 5 $\mu$g, and 2 after lixisenatide 10 $\mu$g) experienced 6 TEAEs (5 from the gastrointestinal disorders SOC and 1 from the infections and infestations SOC). Of these patients, 1 experienced vomiting of mild intensity 43 minutes after injection of placebo (5 minutes after the standardized liquid breakfast), and another patient experienced vomiting of mild intensity with concomitant nausea 3 hours and 15 minutes after injection of lixisenatide 5 $\mu$g (2 hours and 31 minutes after the standardized liquid breakfast). One patient experienced diarrhea and concomitant nausea after injection of lixisenatide 10 $\mu$g. The incidence of TEAEs was low in the adult population (1 event of diarrhea in 1 placebo-treated patient). All TEAEs were of mild to moderate intensity. All patients recovered without sequelae with or without corrective treatment.

In the pediatric population, there were few PCSAs for blood pressure with no relationship to the IMP or dose administered. Few patients had PCSAs for ECG parameters (prolonged PR, QRS, and QTc) without relevant differences between lixisenatide and placebo.

There were no PCSAs (during the on-treatment period) for blood pressure or ECG parameters in the adult population. All patients, except 1 adult, were anti-lixisenatide antibody negative at study entry.

**Conclusions:**

After a standardized liquid breakfast in 12 pediatric patients with T2DM aged between 10 and less than 18 years old, with a mean HbA$_{1c}$ of 8.65% and mean body weight of 84.7 kg treated with metformin as a background therapy, a non-significant decrease in plasma glucose (corrected plasma glucose AUC$_{0:30h-4:30h}$ and plasma glucose AUC$_{0:30h-4:30h}$) was observed with single doses of lixisenatide 5 and 10 $\mu$g compared to placebo. In contrast, single doses of lixisenatide 5 and 10 $\mu$g significantly reduced plasma glucose (corrected plasma glucose AUC$_{0:30h-4:30h}$ and plasma glucose AUC$_{0:30h-:30h}$) in 12 adult patients with T2DM compared to placebo. This PPG-lowering effect in adult patients treated with lixisenatide 10 $\mu$g was associated over the same period with statistically significant decreases in concentrations of glucagon, insulin, and C-peptide. These PD effects occurred to a lesser extent with lixisenatide 5 $\mu$g. In pediatric patients, AUC$_{0:30h-4:30h}$ for glucagon and C-peptide were decreased with lixisenatide 5 and 10 $\mu$g compared to placebo, and the effects were more marked with lixisenatide 10 $\mu$g (p=0.04 for glucagon decrease). Of note, a large variability was observed mainly for AUC$_{0:30h-4:30h}$ for insulin, which increased with lixisenatide 5 $\mu$g and decreased with lixisenatide 10 $\mu$g.

Following single subcutaneous administration, lixisenatide exposure was similar for both dose groups in the evaluable pediatric patients, whereas in adult patients, the lixisenatide exposure dose-proportionally increased. In the full pediatric PK population, lixisenatide exposure was slightly higher for the higher dose of 10 $\mu$g. In pediatric patients, the exposure was similar to that in adults for lixisenatide 5 $\mu$g, but lower for lixisenatide 10 $\mu$g.

Single doses of lixisenatide 5 and 10 $\mu$g were safe and well tolerated in pediatric and adult patients.

**Supportive PD and PK data**

| Adult patients - Descriptive statistics on plasma glucose premeal corrected AUC0:30-4:30h (mmol*h/L) per treatment group - Evaluable PD population | | | | |
|---|---|---|---|---|
| Descriptive statistics on Corrected GLU-AUC$_{0:30-4:30h}$ [mmol*h/L] | | | | |
| Treatment group | N | Mean | Median | (min ; max) |
| Placebo | 12 | 17.51 (4.98) | 16.50 | (9.7 ; 25.7) |
| Lixisenatide 5 $\mu$g | 12 | 10.60 (6.83) | 12.08 | (-5.1 ; 18.6) |

(continued)

| Lixisenatide 10 $\mu$g | 12 | 1.89 (8.36) | 1.01 | (-15.9 ; 16.3) |
|---|---|---|---|---|

Pediatric patients - Descriptive statistics Plasma glucose premeal corrected $AUC_{0:30-4:30h}$ (mmol*h/L) per treatment group - Evaluable PD population

| Descriptive statistics on Corrected GLU-$AUC_{0:30-4:30h}$ [mmol*h/L] | | | | |
|---|---|---|---|---|
| Treatment group | N | Mean | Median | (min ; max) |
| Placebo | 9 | 10.10 (9.57) | 7.89 | (-2.1 ; 21.7) |
| Lixisenatide 5 $\mu$g | 9 | 6.24 (8.53) | 3.77 | (-3.2; 19.9) |
| Lixisenatide 10 $\mu$g | 9 | 8.76 (8.12) | 4.63 | (-0.3; 24.1) |

Adult patients - descriptive statistics on $AUC_{0:30-4:30h}$ for plasma glucose, glucagon, insulin, and C-peptide per treatment group - Evaluable PD population

| | | | Descriptive statistics on $AUC_{0:30-4:30h}$ | | |
|---|---|---|---|---|---|
| Parameter | Treatment group | N | Mean (SD) | Median | min-max |
| Plasma glucose (mmol*h/L) | Placebo | 12 | 57.18 (12.06) | 56.63 | (37.4 ; 77.7) |
| | Lixisenatide 5 $\mu$g | 12 | 47.60 (11.02) | 49.08 | (29.9 ; 71.2) |
| | Lixisenatide 10 $\mu$g | 12 | 38.37 (11.37) | 35.10 | (22.5 ; 58.5) |
| Glucagon (ng.h/L) | Placebo | 12 | 647.39 (132.49) | 616.77 | 500.3; 908.0) |
| | Lixisenatide 5 $\mu$g | 12 | 628.56 (191.49) | 591.56 | (442.2; 1163.8) |
| | Lixisenatide 10 $\mu$g | 12 | 564.83 (148.93) | 513.67 | (417.7; 950.6) |
| Insulin (pmol.h/L) | Placebo | 12 | 1488.52 | 1342.19 | (763.6 ; 2859.4) |
| | Lixisenatide 5 $\mu$g | 11 | (512.03) 1314.15 | 1268.09 | (879.7 ; 1841.0) |
| | Lixisenatide 10 $\mu$g | 12 | (306.08) 1015.12 | 1051.94 | (641.6 ; 1317.8) |
| C-peptide | Placebo | 12 | (261.87) 8.73 (1.91) | 8.82 | (6.0 ; 13.3) |
| (nmol.h/L) | Lixisenatide 5 $\mu$g | 11 | 8.08 (1.24) | 8.65 | (5.9 ; 9.7) |
| | Lixisenatide 10 $\mu$g | 12 | 7.08 (1.24) | 7.25 | (4.7 ; 9.1) |

[1] SE (standard error)

Pediatric patients - descriptive statistics on $AUC_{0:30-4:30h}$ for plasma glucose, glucagon, insulin, and C-peptide per treatment group - Evaluable PD population

| Descriptive statistics on $AUC_{0:30-4:30h}$ | | | | | |
|---|---|---|---|---|---|
| Parameter | Treatment group | N | Mean (SD) | Median | min-max |
| Plasma glucose (mmol*h/L) | Placebo | 9 | 45.57 (19.78) | 42.81 | (19.4 ; 75.9) |
| | Lixisenatide 5 $\mu$g | 9 | 39.60 (14.27) | 41.03 | (20.7 ; 55.9) |
| | Lixisenatide 10 $\mu$g | 9 | 44.60 (17.44) | 48.87 | (17.9 ; 64.4) |

(continued)

| | | | | | |
|---|---|---|---|---|---|
| Glucagon (ng.h/L) | Placebo | 9 | 685.37 (130.00) | 695.60 | (456.9 ; 918.5) |
| | Lixisenatide 5 μg | 8 | 616.84 (119.74) | 592.97 | (428.4 ; 763.9) |
| | Lixisenatide 10 μg | 9 | 644.06 (117.88) | 622.34 | (444.1 ; 824.9) |
| Insulin (pmol.h/L) | Placebo | 7 | 2152.06 (942.87) | 2587.48 | (801.6 ; 3335.7) |
| | Lixisenatide 5 μg | 8 | 2208.53 (1566.96) | 1724.15 | (405.7 ; 5330.2) |
| | Lixisenatide 10 μg | 8 | 2143.02 (1393.59) | 1691.69 | (506.2 ; 4693.3) |
| C-peptide (nmol.h/L) | Placebo | 8 | 9.70 (1.75) | 10.14 | (6.6 ; 11.6) |
| | Lixisenatide 5 μg | 8 | 9.68 (2.74) | 9.18 | (5.3 ; 14.5) |
| [1] SE (standard error) | Lixisenatide 10 μg | 8 | 9.55 (3.00) | 9.42 | (5.3 ; 14.3) |

| PK parameter for lixisenatide in plasma (full PK population) | | | | |
|---|---|---|---|---|
| Mean ± SD (Geometric Mean) [CV%] | Adults | | Pediatric | |
| | Lixisenatide 5 μg | Lixisenatide 10 μg | Lixisenatide 5 μg | Lixisenatide 10 μg |
| N | 10[*,#] | 12 | 10[θ,**] | 9[**,***,+] |
| Cmax (pg/mL) | 26.0 ± 15.4 (22.8) [59.4] | 52.8 ± 21.7 (48.8) [41.1] | 25.3 ± 15.6 (20.4) [61.7] | 33.3 ± 24.0 (27.0) [72.1] |
| $t_{maxa}$ (h) | 1.50 (0.42 - 3.50) | 2.50 (0.42 - 4.50) | 1.50 (0.48 - 4.50) | 0.50 (0.48 - 3.55) |
| $AUC_{last}$ (pg·h/mL) | 101 ± 58.0 (90.8) [57.3] | 228 ± 89.0 (213) [39.0] | 83.0 ± 76.8 (57.1) [92.5] | 105 ± 102 (68.9) [97.4] |
| $AUC_{0.5-4.5}$ (pg·h/mL) | 77.2 ± 42.4 (70.0) [54.9] | 169 ± 72.4 (155) [43.0] | 74.7 ± 55.2 (57.9) [73.9][b] | 86.8 ± 71.2 (65.6) [82.1] |

[a] Median (Min - Max)
\* 826001011; \*\* 484001001, \*\*\*710002007 all samples were below LLOQ.
[θ] 840003002, [#] 826001020 ; [+]484001003 no more than 3 consecutive samples were above LLOQ and therefore not evaluable for PK analysis [b] N=8, could not be calculated for patients 710002005, 710002007, therefore not included in the summary statistics Source = PKS Study: PKD11475;: P-D-A-EV-OD Version 1, P-D-A-EV-OD-E02, Version 3

**A randomized, double-blind, placebo-controlled, dose escalation, study on safety, pharmacokinetics and pharmacodynamics of lixisenatide in pediatric patients with type 2 diabetes not adequately controlled with metformin and/or basal insulin**

**Clinical Trial Summary**

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| INVESTIGATORITRIAL LOCATION | Multinational, multicenter |
| STUDY OBJECTIVE(S) | Primary objective: |

(continued)

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| **INVESTIGATORITRIAL LOCATION** | Multinational, multicenter |
| | • To demonstrate safety of 14-day repeated lixisenatide doses of 5 $\mu$g, 10 $\mu$g and 20 $\mu$g as compared to placebo pediatric in patients with type 2 diabetes<br><br>Secondary objectives:<br>• To evaluate plasma concentrations of lixisenatide after repeated doses of 5 $\mu$g, 10 $\mu$g and 20 $\mu$g and pharmacokinetic parameters of repeated lixisenatide doses of 20 $\mu$g in plasma in pediatric patients with type 2 diabetes<br>• To evaluate the change to baseline in post-prandial plasma glucose concentrations during a standardized meal test after repeated doses of lixisenatide 5 $\mu$g, 10 $\mu$g and 20 $\mu$g in comparison to placebo |
| **STUDY DESIGN** | Phase I, multi-center, randomized (3:1), double-blind, placebo-controlled, dose escalation study<br>The study comprises:<br>• An up to 3-week screening period<br>• A 6-week randomized double-blind treatment period with 2 parallel arms (placebo arm / lixisenatide arm) and incremental sequential steps of 2 weeks for the lixisenatide dose escalation (5 $\mu$g, 10 $\mu$g and 20 $\mu$g) or matched placebo<br>• A post-treatment follow-up period of 3 days |
| **STUDY POPULATION Main selection criteria:** | • Male and female patients with documented type 2 diabetes mellitus insufficiently controlled with a metformin dose $\geq$ 1000 mg/day (or maximum tolerated dose according to the investigator's judgment) at a stable regimen for 8 weeks prior to randomization and/or stable basal insulin alone or in combination for 12 weeks prior to randomization<br>• Aged $\geq$ 10 and < 18 years old (at least 4 patients below 16 years old)<br>• HbA1c >6.5% and $\leq$ 11 %<br>• Body mass index (BMI) of >85th percentile for age and gender and BMI $\leq$ 50 kg/m2 ;<br>• Fasting C-peptide at screening > 0.6 ng/mL<br>• Negative test for anti-insulinoma associated protein (IA2) and anti-glutamic acid decarboxylase (GAD) autoantibodies; |
| **Total expected number of patients: Expected number of sites** | A total of 24 completed patients Tbd |

(continued)

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| **INVESTIGATORITRIAL LOCATION** | Multinational, multicenter |
| STUDY TREATMENT(s) Investigational Medicinal Product(s) Formulation: Route(s) of administration: Dose regimen: | • During the first part of the double-blind treatment period with lixisenatide 5 $\mu$g or matching placebo during 14 days: <br><br> Test drug: Lixisenatide 5 $\mu$g <br> Lixisenatide will be supplied as a disposable pre-filled pen, ie a self-injector device (Tactipen®) containing 3 mL of a sterile aqueous solution for subcutaneous (s.c.) injection in a 3-mL volume containing 300 $\mu$g of the active ingredient (i.e., 100 $\mu$g/mL), glycerol, sodium acetate trihydrate, methionine, metacresol, HCL/NaOH, water for injection. <br> Control drug: Lixisenatide matching placebo <br> Lixisenatide matching placebo will be supplied as a sterile 3-mL aqueous solution. <br> Both lixisenatide and the matching placebo are to be injected once daily with a pen self-injector device and the volume to be injected will be 50 $\mu$L. <br> Route(s) of administration: s.c. <br> • During the second part of the double-blind treatment period with lixisenatide 10 $\mu$g or matching placebo during 14 days: Test drug: Lixisenatide 10 $\mu$g <br> Lixisenatide is supplied as green disposable pre-filled pen, ie a self-injector device (Delta 14®) containing 3 mL of a sterile aqueous solution for s.c. injection with 150 $\mu$g of the active ingredient (ie, 50 $\mu$g/mL), glycerol, sodium acetate trihydrate, methionine, metacresol, CL/NaOH, water for injection. The lixisenatide pen-injector dispenses fourteen fixed doses of 200 $\mu$L. <br> Control drug: Lixisenatide matching placebo <br> Lixisenatide matching placebo will be supplied as a sterile 3-mL aqueous solution <br> Both lixisenatide and the matching placebo are to be injected once daily with a pen self-injector device (Delta 14® of green color) and the volume to be injected will be 200 $\mu$L. <br> Route(s) of administration: s.c. |

(continued)

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| **INVESTIGATORITRIAL LOCATION** | Multinational, multicenter |
| | • During the third part of the double-blind treatment period with lixisenatide 20 $\mu$g during 14 days or matching placebo: Test drug: Lixisenatide 20 $\mu$g Lixisenatide is supplied as purple disposable self-injector device (Delta 14®) containing 3 mL of a sterile aqueous solution for s.c. injection with 300 $\mu$g of the active ingredient (ie, 100 $\mu$g/mL), glycerol, sodium acetate trihydrate, methionine, metacresol, HCL/NaOH, water for injection. The lixisenatide pen-injector dispenses fourteen fixed doses of 200 $\mu$L. Control drug: Lixisenatide matching placebo Lixisenatide matching placebo will be supplied as a sterile 3-mL aqueous solution Both lixisenatide and the matching placebo are to be injected once daily with a pen self-injector device (Delta 14® of purple color) and the volume to be injected will be 200 $\mu$L. Route(s) of administration: s.c. Before starting each dose escalation step the patients and/or their parents will be trained on site by the study staff/nurse to use each type of injector pens appropriately before they start the new dose of daily subcutaneous injection of lixisenatide or placebo. Furthermore, if needed, depending on patients maturity, a home nursing service can be proposed during the first 3 injections (or more if needed) to ensure a good compliance. At home, injections should be performed once daily within 1 hour prior to breakfast. At on-site visits, injections should be performed once daily approximately 30 minutes prior to the start of the standardized breakfast. Background antidiabetic therapy will be administered daily about the same clock time as usually done; adjustment of basal insulin dose may be needed with the supervision of the investigator or medical designee. |
| **Non Investigational Medicinal Product(s) (if applicable) Formulation: Route(s) of administration: Dose regimen** | Not applicable |
| **PRIMARY ENDPOINT(S) AND MAIN SECONDARY ENDPOINT(S)** | **Primary endpoint:** <u>Safety</u>: Adverse events (AEs) /Treatment-Emergent Adverse Events (TEAEs) , clinical laboratory (hematology, biochemistry, lipase and amylase, urinalysis) evaluations including vital signs, 12-lead ECG parameters, body temperature and physical examination. **Secondary endpoints** <u>Pharmacokinetics:</u> • PK parameters ($C_{max}$, $t_{max}$, , $AUC_{0-4.5}$)after 14-day repeated dosingat20 $\mu$g on Day 42 |

(continued)

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| INVESTIGATOR/TRIAL LOCATION | Multinational, multicenter |
| | • Lixisenatide plasma concentrations 0, 0.5, 1.5 and 2.5 hours after IMP injection, i.e. T0 before IMP, T0.5, T1.5 and T2.5, after 14-day repeated dosing at 5 $\mu$g, 10 $\mu$g and 20 $\mu$g on Day 14, Day 28 and Day 42;<br><br>Pharmacodynamics:<br>• The change to baseline in plasma glucose $AUC_{0-4.5}$ after 14-day repeated dosing at 20 $\mu$g on Day 42<br>• The change to baseline in postprandial plasma glucose excursion 1H post meal test and 2H post meal test, i.e. difference T1.5-T0 and T2.5-T0 after 14-day repeated dosing at 5 $\mu$g, 10 $\mu$g and 20 $\mu$g on Day 14, 28 and 42,<br><br>Anti-lixisenatide antibodies: assessment before first dosing at Visit 2 (Day-1), and after 14-day repeated dosing at 5 $\mu$g, 10 $\mu$g and 20 $\mu$g.<br>**Other endpoints**<br>Body weight, HbA1c at baseline and after 14-day repeated dosing at 20 $\mu$g. |
| ASSESSMENT SCHEDULE | Screening period: from Week-3 to Week -1<br>At Visit 2 (Day-1): face to face training of patients and/or parent(s) on IMP pen injector, glucose meter use, diary recording use, hypoglycemia awareness and management education; IMP subcutaneous injection training for the home nurse service, if any, in charge of the administration of the appropriate dose and in the respect of given condition (outpatient procedure excepted at on-site visits).<br>Baseline pharmacodynamics assessments with blood sampling 0.5 hours prior to the standard breakfast ingestion (ie, T0). Then blood sampling will be performed at 1, 1.5, 2, 2.5, 3.5 and 4.5 hours after breakfast (ie, T1, T1.5, T2, T2.5, T3.5 and T4.5).<br>Randomized, double-blind placebo-controlled treatment period with dose escalation every 14 days (5 **$\mu$g, 10 $\mu$g** and **20 $\mu$g** or matching placebo)<br>Every 2 weeks, on-site visits: Visit 3 (Day14), Visit 4 (Day28) and Visit 5.(Day42) for safety, pharmacodynamics and pharmacokinetic assessments<br>These on-site visits require patients to be in fasting condition for blood sampling prior to IMP injection (approximately 30 min before the start of the standard breakfast, ie T0). Then, blood sampling will be performed at the following timepoints:<br>• 0 (immediately before IMP injection), 0.5 (PK only) 1.5 and 2.5 hours after IMP injection (ie T0, T0.5, T1.5 and T2.5) at Visit 3 (Day14) and Visit 4 (Day28)<br>• 0, 0.5 (PK only), 1, 1.5, 2, 2.5, 3.5 and 4.5 min (ie T0, T0.5, T1, T1.5, T2, T2.5, T3.5 and T4.5) at Visit 5 (Day 42)<br>Safety: refer to the study flow chart for physical examination, body temperature and vital signs, 12-lead ECG, and laboratory assessments. Throughout the study : adverse events recording |
| STATISTICAL CONSIDERATIONS | All analyses will be interpreted in an exploratory way, no confirmatory analyses will be done.<br>**Safety:**<br>All randomized patients receiving at least one dose of the IMP (regardless of the amount of treatment administered) will be included in the safety population. |

(continued)

| TITLE | Randomized, double-blind, placebo-controlled, dose escalation study on safety, pharmacokinetics and pharmacodynamics of lixisenatide in pediatric patients with type 2 diabetes not adequately controlled with metformin and/or basal insulin |
|---|---|
| INVESTIGATORITRIAL LOCATION | Multinational, multicenter |
| | The safety analysis will be conducted on the safety population based on individual values (clinically significant abnormalities) and descriptive statistics (summary tables and plots if appropriate). Individual values will be flagged for potentially clinically significant abnormalities (PCSAs), TEAEs will be tabulated (counts and percents). Descriptive statistics will be generated by dose level/treatment for selected parameters of interest. <br><br> Vital signs, Laboratory- and ECG parameters and changes compared to baseline (where appropriate) will be analyzed by dose level/treatment using descriptive statistics. Number and percentage of patients with antibody status negative/positive will be summarized by dose level. <br><br> **Pharmacokinetics (PK):** <br> Plasma AVE0010 concentrations will be summarized by antibody status for each dose level using descriptive statistics. PK parameters ($C_{max}$, $t_{max}$, $AUC_{0-4.5}$) will be summarized by antibody status for the 20 $\mu$g dose level using descriptive statistics. <br><br> **Pharmacodynamics (PD):** <br> Descriptive statistics and graphs will be provided on raw data and change from baseline. Analyses will be done by dose level/treatment. <br><br> Relationship between PK and PD will be explored graphically at the 20 $\mu$g dose level. Results from PK modeling and PK/PD analysis will be reported separately. <br><br> **Others analyses:** <br> Descriptive statistics and graphs will be provided on raw data and absolute change from baseline for HbA1c, and body weight. |
| DURATION OF STUDY PERIOD (per patient) | Duration of each part of the study for one patient: <br><br> - Screening: Day-21 to Day-1 (overnight hospital stay from Day-1 to Day 1 or two single visits) <br> - Treatment period: 6 weeks (Day 1 to Day 42) with on-site visit prior to the dose escalation every 2 weeks <br> - Follow-up and end-of-study: Day 45 <br> - Total study duration: up to 10 weeks |

## 1. FLOW CHARTS

### 1.1. GRAPHICAL STUDY DESIGN

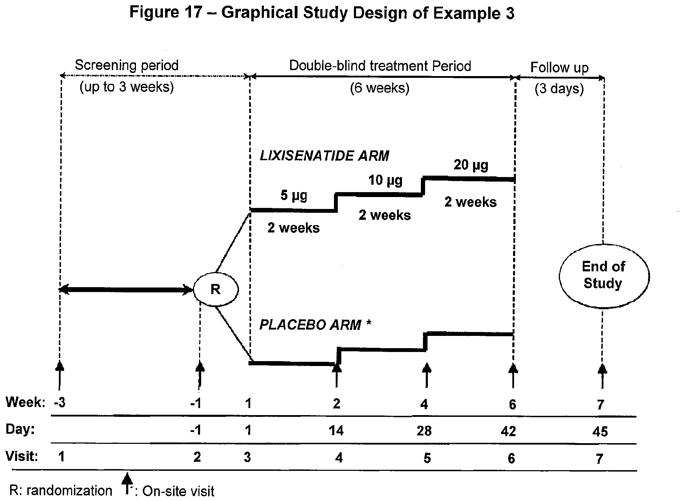

The graphical study design of Example 3 is shown in Figure 17.

### 1.2 STUDY FLOW CHART

EP 3 244 912 B1

| Phase | Screening | | Treatment Period | | | | | | End-of-study (EOS) |
|---|---|---|---|---|---|---|---|---|---|
| Week | W-3 to W-2 | W-1 | W1 | W2 | W3 | W4 | W5 | W6 | W7 |
| Day | D-21 to D-7 | D-1 | D1 | D14 | D15 | D28 | D29 | D42 | D45 |
| Visit window (days) | | -2 | | +3 | | +3 | | +3 | +3 |
| Visit | 1 | 2 | 3 | 4 | | 5 | | 6 | 7 |
| Informed consent | X | | | | | | | | |
| Overnight hospital stay or | | ←--------------------→ | | | | | | | |
| 2 single visits [a] | | X | X | | | | | | |
| Discharge | | | | | | | | | |
| Visit at clinical site [b] | X | | | X | | X | | X | X |
| Medical/surgical history | X | X | | | | | | | |
| Prior/concomitant medications | ←----------- | ---- | --- | --- | ---- | -------- | -------- | - | - | --→ |
| Height | X | | | | | | | | |
| Tanner stage | X | | | | | | | | |
| Autoantibodies test [c] | X | | | | | | | | |
| C-peptide | X | | | | | | | | |
| Urine drug screen, alcohol test [d] | X | X | | | | | | | |
| Inclusion/exclusion criteria | X | X | | | | | | | |
| IRT call [e] | X | X | | X | | X | | X | X |
| Inclusion / Randomization [f] | | X | | | | | | | |
| Standardized meal ingestion in fasting conditions [g] | | X | | X | | X | | X | |
| Study treatment administration | | | | | | | | | |
| Instructions on Pen injector kit / use [h] | | X | | | | | | | |
| IMP dispensation [i] | | X | | X | | X | | | |
| IMP administration [j] | | | |-------------------| | |-------------------| | |------------------| | |
| Other antidiabetic compound [k] | X | X | X | X | X | X | X | X | X |
| Other material dispensed | | | | | | | | | |
| Glucose meter and diary instructions | | X | | | | | | | |
| Glucose meter | | X | | X | | X | | | |
| Diary [l] | | X | X | X | | X | | X | |

81

| Phase | Screening | | Treatment Period | | | | | | End-of-study (EOS) |
|---|---|---|---|---|---|---|---|---|---|
| Week | W-3 to W-2 | W-1 | W1 | W2 | W3 | W4 | W5 | W6 | W7 |
| Day | D-21 to D-7 | D-1 | D1 | D14 | D15 | D28 | D29 | D42 | D45 |
| Visit window (days) | | -2 | | +3 | | +3 | | +3 | +3 |
| Visit | 1 | 2 | 3 | 4 | | 5 | | 6 | 7 |
| Safety | | | | | | | | | |
| Physical examination | X | X | | $X^m$ | | $X^m$ | | $X^m$ | X |
| Body weight | X | X | | $X^m$ | | $X^m$ | | $X^m$ | X |
| Body temperature | X | X | | | | | | | X |
| Blood pressure[n] | X | X | | $X^m$ | | $X^m$ | | $X^m$ | X |
| heart rate [n] | X | X | | | | | | | X |
| 12-lead ECG [o] | X | X | | | | | | | X |
| Serology tests [p] | X | | | | | | | | |
| Hematology, biochemistry, urinalysis [q] | X | | | | | | | | X |
| β-HCG blood test (if applicable) [r] | X | | | | | | | | X |
| Urine pregnancy test (if applicable) [s] | | $X^m$ | | $X^m$ | | $X^m$ | | $X^m$ | |
| Pancreatic enzyme tests [t] | | X | | X | | X | | X | |
| Anti-lixisenatide antibodies | | AB00 | | $AB01^m$ | | $AB02^m$ | | $AB03^m$ | |
| Adverse event / SAE collection | ←---------- | ---- | ---- | ----- | --------- | -------- | --- | -------- | ----- ---→ |

| Phase | Screening | | Treatment Period | | | | | | End-of-study (EOS) |
|---|---|---|---|---|---|---|---|---|---|
| Week | W-3 to W-2 | W-1 | W1 | W2 | W3 | W4 | W5 | W6 | W7 |
| Day | D-21 to D-7 | D-1 | D1 | D14 | D15 | D28 | D29 | D42 | D45 |
| Visit window (days) | 1 | -2 | 3 | +3 | | +3 | | +3 | +3 |
| Visit | 1 | 2 | 3 | 4 | | 5 | | 6 | 7 |
| **Pharmacokinetics** | | | | | | | | | |
| Lixisenatide pharmacokinetic plasma samples $u$ | | | | X | | X | | X | |
| **Pharmacodynamics** | | | | | | | | | |
| Plasma glucose $v$ | | X | | X | | X | | X | |
| HbA1c | X | | | | | | | X | |

a   Either overnight hospital stay from Day -1 to Day 1 at the convenience of patient/parent(s) and according to the possibilities of the investigational site, or 2 single visits
b   Only a single visit scheduled
c   Blood sampling to be done at latest on Day -15 (tbc) for obtaining the results of autoantibody (anti-IA2 and anti-GAD) testing before the randomization
d   Urine drug screen: amphetamines/methamphetamines, barbiturates, benzodiazepines, cannabinoids, cocaine, opiates
e   Call to IRT (interactive response technology) at each visit : At V1, Allocation of the patient's number at screening ; At V2, V3, V4: Allocation of the patient's treatment kit number to be used during 2 weeks from the day after this visit (ie, Day 1, Day 15, Day 29, respectively)- at V6 for the end of treatment and at EOS or for screen failures or drop-out during the treatment period if any
f   Randomization using IRT
g   Ingestion of Liquid Standard meal test provided supervised by the medical staff
h   Demo kit to show to the patient/parents and nurse before starting each treatment period
i   New kit to be dispensed, containing pen injectors for a 2-week treatment period starting the day after this visit
j   IMP subcutaneous injection approximately 30 minutes before breakfast (at Visits V3, V4 injection time and breakfast ingestion accurately reported in eCRF)
k   Metformin administration as usually except at V3, V4 and V5 at lunch time (after last blood sampling); from Day 1 basal insulin always administered once daily about the same clock time as usually done by the patient/nurse either at the same time as IMP injection or in the evening ; adjustment of basal insulin dose if necessary.
l   Diary to be dispensed at V2 and checked at Visits V3, V4, V5 and V6
m   Before study drug administration or before breakfast at V2
n   Vital signs (Heart rate and blood pressure) measured after 10 min in supine resting position
o   12-lead ECG will be recorded after at least 10 min in supine position. Automatic reading will be performed.
p   Hepatitis B antigen and hepatitis C antibodies
q   Hematology: Red Blood Cell Count, Hematocrit, Hemoglobin, White Blood Cell Count with differential (Neutrophils, Lymphocytes, Monocytes, Basophils, Eosinophils), Platelets; Serum Chemistry: Sodium, Potassium, Chloride, Calcium, AST, ALT, alkaline phosphatase, gamma-glutamyl transferase (GGT), total and conjugated bilirubin, Urea, Creatinine, Glucose, Albumin, total Protein, total Cholesterol, Triglycerides, creatine phosphokinase (CPK), amylase, lipase) and at screening only calcitonin ;Urinalysis: proteins, glucose, blood (erythrocytes/leucocytes), ketone bodies, pH
r   In females of reproductive potential (Tanner Stage ≥ 3), serum beta-HCG only at screening
s   In all females urinary pregnancy test
t   Lipase and amylase tests
u   Pharmacokinetic profile ; analysis in Central laboratory
v   Pharmacodynamic profile ; analysis in Central laboratory

## 1.3 PERIOD FLOW CHART

1.3.1 Overnight hospital stay from Day-1 to Day 1

| Phase | End of Screening | | | | | | | | | Start treatment period | | |
|---|---|---|---|---|---|---|---|---|---|---|---|---|
| Week | W-1 | | | | | | | | | W1 | | |
| Day | D-1 | | | | | | | | | D1 | | |
| Visit | V2 | | | | | | | | | | | |
| Time (hour/minute) T (hr) | 0 H T0 | 0.5 H T0.5 | 1 H T1 | 1.5 H T1.5 | 2 H T2 | 2.5 H T2.5 | 3 H T3.5 | 4.5 H T4.5 | 5 H | 0 H T0 | 0.5 H T0.5 | 1 H T1 |
| Indicative clock time a | 8:00 am | 8:30 am | 9:00 am | 9:30 am | 10:00 am | 10:30 am | 11:30 am | 12:30 pm | | 8:00 am | 8:30 am | 9:00 am |
| Overnight hospital stay at clinical site | ← | | | | | | | | | ← | | → |
| or 2 single visits b | ← | | | | | | | → | | | | → |
| Discharge c | | | | | | | | | | | | X |
| Medical/surgical history d | X | | | | | | | | | | | |
| Prior/Concomitant medications | < | | | | | | | → | | | | |
| Urine drug screen, alcohol test e | X | | | | | | | | | | | |
| Inclusion/exclusion criteria | X | | | | | | | | | | | |
| IRT call / Inclusion / Randomization f | X | | | | | | | | | | | |
| Standardized meal test in fasting conditions g | | X | | | | | | | | | | |
| Study treatment administration | | | | | | | | | | | | |
| Instructions on Pen injector kit /use h | | | | | X | | | | | | X | |
| IMP allocation i | | | | | | | | | | | X | |
| IMP administration j | | | | | | | | | | | X | |
| Other antidiabetic compound k | | X | | | X [k] | | | | | | X [k] | |
| Other material dispensed with instructions | | | | | | | | | | | | |
| Glucose meter and diary instructions | | | | | | | | | X | | | |
| Glucose meter | X | | | | | | | | | | | |
| Diary | X | | | | | | | | | | | |
| Safety | | | | | | | | | | | | |
| Physical examination | X | | | | | | | | | | | |
| Body weight | X | | | | | | | | | | | |
| Body temperature | X | | | | | | | | | | | |
| Blood pressure/heart rate l | X | | | | | | | | | | | X |
| Urine pregnancy test (if applicable) m | X | | | | | | | | | | | X |

| Phase | End of Screening | | | | | | | | | Start treatment period | | |
|---|---|---|---|---|---|---|---|---|---|---|---|---|
| Week | W-1 | | | | | | | | | W1 | | |
| Day | D-1 | | | | | | | | | D1 | | |
| Visit | V2 | | | | | | | | | | | |
| Time (hour/minute)<br>T (hr) | 0 H<br>T0 | 0.5 H<br>T0.5 | 1 H<br>T1 | 1.5 H<br>T1.5 | 2 H<br>T2 | 2.5 H<br>T2.5 | 3 H<br>T3.5 | 4.5 H<br>T4.5 | 5 H | 0 H<br>T0 | 0.5 H<br>T0.5 | 1 H<br>T1 |
| Indicative clock time [a] | 8:00 am | 8:30 am | 9:00 am | 9:30 am | 10:00 am | 10:30 am | 11:30 am | 12:30 pm | | 8:00 am | 8:30 am | 9:00 am |
| Pancreatic enzyme tests [n] | X | | | | | | | | | | | |
| Anti-lixisenatide antibodies | AB00 | | | | | | | | | | | |
| Adverse event collection | ←-----------------------------------------------------------------------------------------------------------------------------------------------→ | | | | | | | | | | | |
| Pharmacodynamics | | | | | | | | | | | | |
| Plasma glucose [o] | X | | X | X | X | X | X | X | | | | |

a  Indicative clock times are approximate times used to provide a clear understanding regarding the timing for dosing, procedures and assessments. The first tests and examination can start between 07:30 and 9:00 and the successive timepoints will be adjusted in the respect of the time intervals per protocol

b  Overnight hospital stay from Day -1 to Day 1 at the convenience of patient/parent(s) and according to the possibilities of the investigational site, or 2 single visits.

c  Discharge on Day 1 at least 1 hour after breakfast and after study material and instructions given

d  Update from the last visit

e  Urine drug screen: amphetamines/methamphetamines, barbiturates, benzodiazepines, cannabinoids, cocaine, opiates

f  Randomization and allocation of the patient's treatment kit number using IRT (kit to be used from Day1 during 2 weeks). OR Call to IRT for screen failures, if any

g  Ingestion of Liquid Standard meal test provided supervised by the medical staff

h  Demo kit to show to the patient/parent(s) and the nurse with condition of administration (deep subcutaneous injection at alternating sites and approximately 30 min prior to breakfast)

i  IMP treatment box to be dispensed (kit number delivered by IRT), containing pen injectors for a 2-week treatment period starting on Day 1

j  IMP subcutaneous injection approximately 30 minutes before breakfast (injection time and breakfast ingestion accurately reported in eCRF)

k  On Day -1, metformin administered after the last blood sampling and basal insulin always administered throughout the study once daily about the same clock time as usually done by the patient/parent/nurse either at the same time as IMP injection or in the evening ; on Day 1 : adjustment of basal insulin dose if necessary (by the investigator or designee).

l  Vital signs (Heart rate and blood pressure) measured after 10 min in supine resting position

m  In females urinary pregnancy test

n  Amylase and lipase tests

o  Pharmacodynamic profile (7 blood sampling); analysis in Central laboratory

| Phase | Treatment period | | | | | | |
|---|---|---|---|---|---|---|---|
| Week | W2 / W4 | | | | | | |
| Day | D14 / D28 | | | | | | |
| Visit window | +3 days | | | | | | |
| Visit | V3 / V4 | | | | | | |
| Time (hour/minute) | 0H | 0H30 | 1H | 1H30 | 2H | 2H30 | 3H |
| Time (hr) | T0 | T0.5 | T1 | T1.5 | T2 | T2.5 | |
| Indicative clock time[a] | 8:00 am | 8:30 am | 9:00 am | 9:30 am | 10:00 am | 10:30 am | 11 am |
| Single visit at clinical site [b] | ←----------------------------------------------------------------------→ | | | | | | |
| Discharge [c] | | | | | | | X |
| Prior/Concomitant medications | < | - | - | - | - | - | |
| IRT call [d] | | | | | | | X |
| Standardized meal test in fasting conditions [e] | | X | | | | | |
| Study treatment administration | | | | | | | |
| IMP compliance / inventory | X | | | | | | |
| IMP administration [f] | X | | | | | | |
| IMP allocation [g] | | | | | | | X |
| Other antidiabetic compound [h] | | | | | | | X[h] |
| Other materials checked | | | | | | | |
| Glucose meter | | | | | | | X |
| Diary [i] | X[i] | | | | | | X |
| Safety | | | | | | | |
| Physical examination | X[i] | | | | | | |
| Body weight | X[i] | | | | | | |
| Blood pressure [k] | X[i] | | X | | | | X |
| Urine pregnancy test (if applicable) [l] | X[i] | | | | | | |
| Pancreatic enzyme tests [m] | X[i] | | | | | | |
| Anti-lixisenatide antibodies | X[i] | | | | | | |
| Adverse event collection | < | - | - | - | - | - | |

| Phase | Treatment period | | | | | | |
|---|---|---|---|---|---|---|---|
| Week | W2 / W4 | | | | | | |
| Day | D14 / D28 | | | | | | |
| Visit window | +3 days | | | | | | |
| Visit | V3 / V4 | | | | | | |
| Time (hour/minute)<br>Time (hr) | 0H<br>T0 | 0H30<br>T0.5 | 1H<br>T1 | 1H30<br>T1.5 | 2H<br>T2 | 2H30<br>T2.5 | 3H |
| Indicative clock time[a] | 8:00 am | 8:30 am | 9:00 am | 9:30 am | 10:00 am | 10:30 am | 11 am |
| Pharmacokinetics | | | | | | | |
| Lixisenatide pharmacokinetic plasma samples [n] | P00 | P01 | | P02 | | P03 | |
| Pharmacodynamics | | | | | | | |
| Plasma glucose [o] | X | | | X | | X | |

a   Indicative clock times are approximate times used to provide a clear understanding regarding the timing for dosing, procedures and assessments. The first tests and examination can start between 07:30 and 9:00 and the successive timepoints will be adjusted in the respect of the time intervals per protocol
b   Stay at the clinic all the morning
c.  Discharge approximately 30 minutes after the last sample and after study material and instructions given
d   Call to IRT for visit done (or drop out, if any) and track of drug inventory at each visit; At V3 and V4: Allocation of the patient's treatment kit number using IRT (kit to be used from the day after this visit during 2 weeks).
e   Ingestion of Liquid Standard meal test provided supervised by the medical staff
f   IMP subcutaneous injection approximately 30 minutes before breakfast (injection time and breakfast ingestion accurately reported in eCRF)
g   At V3 and V4: new IMP treatment box to be dispensed, containing pen injectors for a 2-week treatment period starting the day after this visit
h   At V3, and V4: metformin administered after the last blood sampling and basal insulin always administered throughout the study once daily about the same clock time as usually done by the patient/parent/nurse either at the same time as IMP injection or in the evening ; adjustment of basal insulin dose if necessary (by the investigator or designee).
i   Diary to be checked before IMP administration and given back to the patient/parent
j   Before study drug administration
k   Vital signs (Heart rate and blood pressure) measured after 10 min in supine resting position
l   In females, urinary pregnancy test at visit
m   Amylase, lipase tests
n   Pharmacokinetic profile (4 blood sampling); analysis in Central laboratory
o   Pharmacodynamic profile (3 blood sampling); analysis in Central laboratory

**1.3.3 Single visit on Day 42**

| Phase | Treatment period | | | | | | | | |
|---|---|---|---|---|---|---|---|---|---|
| Week | W6 | | | | | | | | |
| Day | D42 | | | | | | | | |
| Visit window | +3 days | | | | | | | | |
| Visit | V5 | | | | | | | | |
| Time (hour/minute) [a] | 0H<br>T0 | 0H30<br>T0.5 | 1H<br>T1 | 1H30<br>T1.5 | 2 H<br>T2 | 2H30<br>T2.5 | 3 H30<br>T3.5 | 4H30<br>T4.5 | 5H |
| Indicative clock time | 8:00 am | 8:30 am | 9:00 am | 9:30 am | 10:00 am | 10:30 am | 11:30 am | 12:30 am | 13:00 |
| Single visit at clinical site [b] | ←----------------------------------------------------------------------------------------------------------------------→ | | | | | | | | |
| Discharge [c] | | | | | | | | | X |
| Prior/Concomitant medications | < | - | - | - | - | - | - - | - | --> |
| IRT call [d] | | | | | | | | | X |
| Standardized meal test in fasting conditions [e] | | X | | | | | | | |
| Study treatment administration | | | | | | | | | |
| IMP compliance / inventory | X | | | | | | | | |
| IMP administration [f] | X | | | | | | | | |
| Other antidiabetic compound [g] | | | | | | | | | X[g] |
| Other materials checked | | | | | | | | | |
| Glucose meter | | | | | | | | | |
| Diary [h] | X[i] | | | | | | | | |
| Safety | | | | | | | | | |
| Physical examination | X[i] | | | | | | | | |
| Body weight | X[i] | | | | | | | | |
| Blood pressure [j] | X[i] | | X | | | | | | X |
| Urine pregnancy test (if applicable) [k] | X[i] | | | | | | | | |
| Pancreatic enzyme tests [l] | X[i] | | | | | | | | |
| Anti-lixisenatide antibodies | X[i] | | | | | | | | |
| Adverse event collection | < | - | - | - | - | - | - | | > |

89

| Phase | Treatment period | | | | | | | | |
|---|---|---|---|---|---|---|---|---|---|
| Week | W6 | | | | | | | | |
| Day | D42 | | | | | | | | |
| Visit window | +3 days | | | | | | | | |
| Visit | V5 | | | | | | | | |
| Time (hour/minute) [a] | 0H<br>T0 | 0H30<br>T0.5 | 1H<br>T1 | 1H30<br>T1.5 | 2 H<br>T2 | 2H30<br>T2.5 | 3 H30<br>T3.5 | 4H30<br>T4.5 | 5H |
| Indicative clock time | 8:00 am | 8:30 am | 9:00 am | 9:30 am | 10:00 am | 10:30 am | 11:30 am | 12:30 am | 13:00 |
| **Pharmacokinetics** | | | | | | | | | |
| Lixisenatide pharmacokinetic plasma samples [m] | P00 | P01 | P02 | P03 | P04 | P05 | P06 | P07 | |
| **Pharmacodynamics** | | | | | | | | | |
| Plasma glucose [n] | X | | X | X | X | X | X | X | |
| HbA1c | X | | | | | | | | |

a Indicative clock times are approximate times used to provide a clear understanding regarding the timing for dosing, procedures and assessments. The first tests and examination can start between 07:30 and 9:00 and the successive timepoints will be adjusted in the respect of the time intervals per protocol

b Stay at the clinic all the morning UP TO 5H00 POST DOSING

c Discharge approximately half hour after the last sample

d Call to IRT for end of treatment (or drop out, if any)

e Ingestion of Liquid Standard meal test provided supervised by the medical staff

f IMP subcutaneous injection approximately 30 minutes before breakfast (injection time and breakfast ingestion accurately reported in eCRF)

g Metformin administered after the last blood sampling and basal insulin always administered throughout the study once daily about the same clock time as usually done by the patient/parent/nurse either at the same time as IMP injection or in the evening ; adjustment of basal insulin dose if necessary (by the investigator or designee).

h Diary to be checked before IMP administration

i Before study drug administration

j Vital signs (Heart rate and blood pressure) measured after 10 min in supine resting position

k In females, urinary pregnancy test at visit

l Amylase, lipase tests

m Pharmacokinetic profile (8 blood sampling); analysis in Central laboratory

n Pharmacodynamic profile (7 blood sampling); analysis in Central laboratory

## 2 INTRODUCTION AND RATIONALE

### 2.1 INTRODUCTION

Lixisenatide is a potent and selective Glucagon-Like peptide-1 (GLP-1) receptor agonist. The GLP-1 receptor is the target for native GLP-1, an endogenous incretin hormone that potentiates glucose-dependent insulin secretion from beta cells and suppresses glucagon secretion from alpha cells in the pancreas. Similar to endogenous GLP-1, the action of lixisenatide is mediated via a specific interaction with GLP-1 receptors, including those on pancreatic alpha and beta cells. After a meal, lixisenatide activates the following individual physiologic responses:

- Enhances insulin secretion by β-cells in a glucose dependent way

- Suppresses glucagon secretion by α-cells

- Delays gastric emptying

Lixisenatide stimulates insulin secretion only when blood glucose is increased, but not at euglycemia, which limits the risk of hypoglycemia. In parallel, glucagon secretion is suppressed. In case of hypoglycemia, the rescue mechanism of glucagon secretion is preserved. Lixisenatide also slows gastric emptying thereby reducing the rate at which meal-derived glucose appears in the circulation. The effect on gastric emptying may contribute to body weight reduction.

Lixisenatide further showed a trend towards insulinotropic activity, including enhancement of insulin biosynthesis and stimulation of beta-cell proliferation in animals, and has been shown to preserve beta cell function and prevent cell death (apoptosis) in isolated human pancreatic islet cells.

In summary, lixisenatide is an exendin analog with strong GLP-1 agonistic activity. The principal therapeutic potential of lixisenatide to lower blood glucose in adult T2DM patients has been established in clinical studies.

Lixisenatide (Lyxumia®) was approved since 2013 in the European Union, Japan, Mexico and other parts of the world for the treatment of adults with T2DM to achieve glycemic control in combination with oral glucose lowering medicinal products and/or basal insulin when these, together with diet and exercise, do not provide adequate glycemic control. The indication may vary slightly across the countries where lixisenatide is approved.

According to the Summary of Product Characteristics, the 10 μg dose is the starting dose intended to improve gastrointestinal tolerability. After 2 weeks at 10 μg QD, the dose should be increased to 20 μg QD from Day 15. The 20 μg dose QD is the fixed maintenance dose.

Up to now, the safety and efficacy of the GLP-1 receptor agonists currently approved in the U.S. have not been established for use in patients less than 18 years of age. Therefore, there is little clinical evidence upon which to base a discussion of anticipated therapeutic similarities or differences between pediatric and adult patients with T2DM administering these agents.

In a single-dose pharmacokinetic study conducted with exenatide in 13 patients with type 2 diabetes and between the age of 12 and 16 years, administration of exenatide (5μg, the initiation dose in adult) resulted in slightly lower mean AUC (16% lower) and Cmax (25% lower) compared to those observed in adults (12). Based on the structural similarities of lixisenatide and exenatide, these results may be taken into consideration when designing the clinical studies proposed with lixisenatide.

A previous PK/PD study (Example 2) in type 2 diabetic children and adolescents (from 10 to 17 years) and in type 2 diabetic adults (as a control group) was a multicenter study evaluating PK, safety/tolerability and PD parameters after single s.c. administration of lixisenatide 5 μg, 10 μg and placebo according to a randomized, double-blind crossover design. All included patients were previously treated with metformin. In 12 pediatric patients, a non-significant decrease in plasma glucose (plasma glucose AUC$_{0:30h-4:30h}$) after a standardized liquid breakfast was observed with single doses of lixisenatide 5 and 10 μg compared to placebo. In contrast, these single doses of lixisenatide significantly reduced plasma glucose compared to placebo in 12 adult patients with T2DM. Lixisenatide exposure was similar for both dose groups in the evaluable pediatric patients, whereas in adult patients, the lixisenatide exposure dose-proportionally increased. In pediatric patients, the exposure was similar to that in adults for lixisenatide 5 μg, but lower for lixisenatide 10 μg. Single doses of lixisenatide 5 and 10 μg were safe and well tolerated in both, pediatric and adult patients in this study of short duration.

In conclusion, Example 2 demonstrated comparable PK and PD profiles in pediatric and adult patients at a dose of 5μg, as well as no unexpected safety results. However, the results observed with a dose of 10μg (initiation dose in adult) are not conclusive. The dose of 20μg (maintenance dose in adult) was not evaluated in this study.

As a consequence, this repeated dose study will therefore be conducted to further evaluate PK, PD and safety at a dose of 5, 10 and 20μg before conducting a large phase 3 study with the expected therapeutic dose.

More detailed information on lixisenatide (AVE0010) is provided in the Investigator's Brochure.

## 2.2 RATIONALE

### 2.2.1 Study Rationale

The aim of the present study is to evaluate safety, pharmacokinetics and pharmacodynamics of repeated subcutaneous QD dose administration of lixisenatide (5 µg, 10 µg and 20 µg) versus placebo in pediatric patients with type 2 diabetes (10-17 years old).

T2DM in children and adolescents has become an increasingly important public health concern throughout the world. T2DM occurs when insulin secretion is inadequate to meet the increased demand posed by insulin resistance, leading to relative insulin deficiency (1) and is frequently associated with other metabolic abnormalities, characteristic of insulin resistance (dyslipidemia, hypertension, polycystic ovary syndrome, fatty liver) (2).

Coinciding with the increasing prevalence of obesity in children, the incidence of T2DM in children and adolescents has markedly increased. Obesity is a major risk factor impacting insulin sensitivity and leading to T2DM in pediatric patients.

The pathophysiology of T2DM in children and adolescents appears to be similar to that in adults. The increase in diabetes in a younger population is likely to be related to the increase in obesity in this population.

One study found an inverse relationship between body mass index and age at diagnosis of T2DM amongst adults, and it is possible that the degree of obesity determines when diabetes will develop. It is then reasonable to assume that such glucose-lowering agents associated with weight reduction will be effective in the pediatric population. However, some factors, such as the number of associated co-morbidities in the different age groups, and differences in the management of children / adolescents as compared to adults, make it difficult to estimate the similarities and differences of treatment effects in T2DM between these two populations.

At present, metformin and insulin are the only drugs with regulatory approval in most countries for the treatment of pediatric diabetes. Because approximately half of youth with T2DM fail to maintain glycemic control when treated with metformin either alone or in conjunction with lifestyle interventions, insulin therapy is often required soon after diagnosis. Thus, there is a need for more treatment options for children and adolescents with T2DM.

### 2.2.2 Design Rationale and risk assessment

This is a multi-centric, randomized, double-blind, placebo-controlled, repeated dose study with lixisenatide dose escalation by 2-week step starting at 5 µg, followed by 10µg and 20µg.

### • Study population

The study population will include male and female patients aged between 10 and 17 years old inclusive.

Use of GLP-1 receptor agonists may be associated with gastrointestinal adverse reactions.

Therefore, pediatric patients with severe gastrointestinal disease associated with prolonged nausea and vomiting, including severe gastroparesis will not be included in this study. To date, there is limited therapeutic experience of lixisenatide in adult patients with moderate renal impairment and no therapeutic experience in patients with severe renal impairment (creatinine clearance less than 30 mL/min) or end-stage renal disease. As a consequence, pediatric patients with severe renal impairment will not be included in the present study.

### • Doses and regimen

In the present study, lixisenatide treatment will be initiated with 5 µg QD during 2 weeks then increased to 10 µg QD for 2 weeks then 20 µg QD for 2 weeks. This stepwise dose increase can prevent or reduce gastro-intestinal adverse events frequently observed with lixisenatide. The dose of 5 µg corresponds to 50 % of the starting dose in adults, and 20 µg QD is the maintenance dose in adults. This study will assess lixisenatide given in combination with metformin and/or basal insulin.

Patients will be included with a stable dose of metformin (unchanged for at least 8 weeks prior to randomization) and the initial metformin dose is to be kept unchanged throughout the study. The metformin morning dose will not be taken before the last blood sample and it may be delayed at lunch time or later.

When lixisenatide is added to existing therapy of basal insulin, a reduction in the dose of the basal insulin may be considered to reduce the risk of hypoglycemia, possibly when starting the dosing with 20 µg, at the discretion of the investigator.

• **Condition of administration**

Lixisenatide should be administered by deep subcutaneous injection, alternating between the left and right anterolateral and left and right posterolateral abdominal wall, thighs or upper arms. Within a given area, location should be changed (rotated) at each time to prevent injection site skin reactions.

Lixisenatide will be subcutaneously administered on site approximately 30 minutes before the start of the standardized breakfast on Days 14, 28 and 42.The other days, it will be administered within 1 hour before breakfast in outpatients.

## 2.2.3 Specific parameters rationale

Hypoglycemia and symptomatic hypoglycemia will be carefully monitored by reporting of adverse events and regular control of glycemia; appropriate device for self-monitoring plasma glucose (monitored by parents) will be provided to participants (Section 4.2.2).

The monitoring of pancreatic enzyme levels will be applied in this study. This is an established practice in clinical trials involving glucagon-like peptide-1 receptor agonists following reports of pancreatitis during T2DM treatment with this therapeutic class (3). Diagnosis of pancreatitis required meeting of two of the following three criteria: amylase/lipase levels three or more times the upper normal limit, characteristic abdominal pain, and/or characteristic findings of acute pancreatitis on computed tomography scan or magnetic resonance imaging.

Anti-lixisenatide antibody formation may occur. Therefore, they will be measured before first dosing at Day- 1 (baseline), and after 14-day repeated dosing at 5 $\mu$g, 10 $\mu$g and 20 $\mu$g. Systemic allergic reactions may occur, as well as other hypersensitivity reactions that have been observed in lixisenatide clinical trials, eg, rash or exanthema, urticaria, angioedema and anaphylactic reactions. Hypersensitivity reactions may occur, with or without the presence of anti-lixisenatide antibodies.

## 2.2.4 Study committees

The sponsor can ask the opinion from independent experts in the field of allergy to review the cases of allergic or allergic-like reactions in a blinded manner with regard to study treatment. Similarly, in cases of pancreatitis, the events can be reviewed by independent gastroenterology experts.

## 3 SELECTION OF PATIENTS

### 3.1 INCLUSION CRITERIA

**Demography**

I01. Male or female patients aged $\geq$ 10 and < 18 years old (at least 4 patients below 16 years old)

I02. Body mass index (BMI) > 85th percentile for age and gender ; BMI $\leq$ 50 kg/m2 ; body weight > 50 kg

**Health status**

I03. Male and female patients with documented type 2 diabetes mellitus insufficiently controlled with a metformin dose $\geq$1000 mg/day (or maximum tolerated dose according to the Investigator's judgment) at a stable regimen for 8 weeks prior to randomization and/or stable basal insulin alone or in combination for 12 weeks prior to randomization

I04. HbAlc > 6.5% and $\leq$ 11% at screening

I05. Fasting C-peptide at screening > 0.6 ng/mL (0.20 nmol/L)

I06. Negative test for anti-insulinoma associated protein (IA2) and anti-glutamic acid decarboxylase (GAD) autoantibodies

I07. Menstruating females (even if irregular) must have a negative pregnancy test for inclusion and agree to repeat pregnancy tests at designated visits throughout the study.

**Regulations**

I08. Provision of Informed Consent form signed by the patient's parent (s)/legal representative. In addition, provision of Assent Form signed by minor patient or Informed Consent Form signed by emancipated or mature minors (defined by local laws)

I 09. Covered by a health insurance system where applicable, and/or in compliance with the recommendations of the national laws in force relating to biomedical research.

I 10. Not under any administrative or legal supervision.

**3.2 EXCLUSION CRITERIA**

**3.2.1 Exclusion criteria related to study methodology**

E 01. If female, ongoing pregnancy (defined as positive serum pregnancy test), breast-feeding

E 02. Sexually active postmenarchal female patient who does not agree to use an adequate and highly effective method of contraception throughout the study duration and according to local regulation (i.e. hormonal contraception, condom, etc.).

E 03. Diabetes other than type 2 diabetes

E 04. History of acute metabolic decompensation such as diabetic ketoacidosis within 3 months

E 05. Fasting plasma glucose > 250 mg/dL (>13.9 mmol/L) at screening

E 06. Hemoglobinopathy or hemolytic anemia

E 07. Recurrence of severe hypoglycemia or hypoglycemic unawareness as judged by the investigator

E 08. Uncontrolled hypertension, treated or untreated above 99th percentile for age and gender in children (see *Appendix A*)

E 09. Any clinically significant abnormality identified on physical examination, laboratory tests, ECG or vital signs at the time of screening that in the judgment of the Investigator or any sub Investigator would make implementation of the protocol or interpretation of the study results difficult or would preclude the safe participation of the patient in this protocol such as active malignant tumor diagnosed hyperthyroidism or uncontrolled hypothyroidism or major systemic diseases etc. (euthyroid patients on replacement therapy will be included if the dosage of thyroxin is stable for at least three months prior to screening Visit)

E 10. Receipt of blood or plasma products within 3 months prior to the time of screening

E 11. Patient/Parent(s) considered by the investigator or any sub investigator as inappropriate for this study for any reason (eg, impossibility to meet specific protocol requirements, such as scheduled visits, administer s.c. IMP QD self-injection or refusal of any assistance of home nurse service for the s.c. IMP injections, etc)

E 12. Use of other oral or injectable antidiabetic or hypoglycemic agents other than metformin and basal insulin (eg, alpha glucosidase inhibitor, GLP-1 receptor agonist, DPP-IV inhibitors, short-acting insulin etc.) within 1 months prior to the time of screening

E 13. Use of systemic glucocorticoids (excluding topical application or inhaled forms) for one week or more within 3 months prior to the time of screening

E 14. Patient having received or receiving psychotropic medication

E 15. Patient receiving treatment with weight reduction medications (including anti-obesity treatment)

E 16. Likelihood of requiring treatment during the screening phase and treatment phase with drugs not permitted by the clinical study protocol

E 17. Use of any investigational drug within 3 months prior to screening

### 3.2.2 Exclusion criteria related to the current knowledge of lixisenatide

E 18. Clinically relevant history of gastrointestinal disease associated with prolonged nausea and vomiting, including, but not limited to gastroparesis and gastroesophageal reflux disease requiring medical treatment, within 6 months prior to the time of screening

E 19. Any previous treatment with lixisenatide

E 20. Allergic reaction to any GLP-1receptor agonist in the or to metacresol

E 21. History of unexplained pancreatitis, chronic pancreatitis, pancreatectomy, stomach/gastric surgery, inflammatory bowel disease

E 22. Personal or family history of medullary thyroid cancer (MTC) or genetic conditions that predispose to MTC (eg, multiple endocrine neoplasia syndromes)

E 23. Known history of drug or alcohol abuse within 6 months prior to the time of screening

E 24. Laboratory findings at the time of screening:

- Elevations in blood tests of renal (serum creatinine >1.0 mg/dL) and/or liver (ALT, AST and/or bilirubin) >2 times the upper limit of normal (ULN) for age.

- Hemoglobin <11 g/dL and/or neutrophils <1500/mm$^3$ and/or platelets <100 000/mm$^3$

- Calcitonin $\geq$20 pg/mL

- Amylase and/or lipase above 3 times the upper limit

- Positive result on any of the following tests: hepatitis B surface (HBs Ag) antigen, anti-hepatitis C virus (anti-HCV) antibodies

E 25. Positive alcohol breath test

E 26. Positive result on urine drug screen (amphetamines/methamphetamines, barbiturates, benzodiazapines, cannabinoids, cocaine, opiates)

E 27. Severe renal impairment defined with creatinine clearance < 30 mL/min/1.73m$^2$ using the revised Schwartz Formula (4)

$$GFR = \frac{0.413 * \text{Ht}}{\text{Cr}_{serum}}$$

CrCl (mL/min/1.73 m$^2$) - Ht: Height in cm - Cr$_{serum}$ (mg/dL)

## 4 ASSESSMENT OF INVESTIGATIONAL MEDICINAL PRODUCT

### 4.1 PHARMACODYNAMICS

#### 4.1.1 Pharmacodynamic parameters

- Plasma glucose

  - the change to baseline in plasma glucose AUC$_{0-4.5 \text{ after}}$ 14-day repeated dosing at 20 μg on Day 42 (GLU-AUC$_{0-4.5}$). GLU- AUC$_{0-4.5}$ is defined as the area under the plasma glucose concentration time profile from time of the IMP injection until 4:30 hours later (T4.5). AUC will be calculated using the trapezoidal rule.

  - the change to baseline in postprandial plasma glucose excursion 1 hour postprandial and 2 hours postprandial after 14-day repeated dosing at 5 μg, 10 μg and 20 μg on Day 14, 28 and 42:

    o postprandial plasma glucose excursion 1 hour post prandial (1H-PPG) will be calculated as the difference between the plasma glucose value 1 hour post meal test (T1.5) and the plasma glucose value before time of injection (T0): 1H-PPG = PG-T1.5 - PG-T0

    ∘ postprandial plasma glucose excursion 2 hours post prandial (2H-PPG) will be calculated as the difference between the plasma glucose value 2 hours post meal test (T2.5) and the plasma glucose value before time of injection (T0): 2H-PPG = PG-T2.5 - PG-T0

- HbA1c

  - Change from baseline to Week 6

- Body weight

  - Change from baseline to Week 6

#### 4.1.2 Assessment methods

##### 4.1.2.1 Plasma glucose

Plasma glucose assessments are planned on Day -1 (V2) (Baseline), Day 14 (V3), Day 28 (V4) and Day 42 (V5). Blood samples will be taken as indicated below in Table 1

**Table 1 - Blood sampling for plasma glucose**

| Time (hour/min) | 0 H | 1H | 1H30 | 2H | 2H30 | 3H30 | 4H30 |
|---|---|---|---|---|---|---|---|
| T (h) | T0 [a] | T1 | T1.5 | T2 | T2.5 | T3.5 | T4.5 |
| Visit/Day: | | | | | | | |
| V2/Day-1 | X | X | X | X | X | X | X |
| V3/D14 | X | | X | | X | | |
| V4/D28 | X | | X | | X | | |

(continued)

| Time (hour/min) | 0 H | 1H | 1H30 | 2H | 2H30 | 3H30 | 4H30 |
|---|---|---|---|---|---|---|---|
| T (h) | T0 [a] | T1 | T1.5 | T2 | T2.5 | T3.5 | T4.5 |
| V5/D42 | X | X | X | X | X | X | X |

a 30 min before to the standardized breakfast ingestion and prior to IMP administration at V3/D14, V4/D28 and V5/D42

The first blood sampling (T0) for plasma glucose will be withdrawn in fasting condition (i.e., patients will be fasted for approximately 10 hours overnight), 30 min prior to the standardized breakfast and prior to dosing on Days 14, 28 and 42.

Samples for plasma glucose will be analyzed in a Central laboratory. Detailed information on sample drawing, management and bioanalytical methods for plasma glucose will be provided in the laboratory manual.

### 4.1.2.2 HbA1c

HbA1c will be measured by a central laboratory certified level I "National Glycohemoglobin Standardization Program" (NGSP) central laboratory.

HbA1c will be measured at screening (V1) and Day42 (V5).

Detailed information on sample drawing, management and bioanalytical methods for HbA1c will be provided in the laboratory manual.

### 4.1.2.3 Body weight

Body weight should be obtained with the patient wearing undergarments or very light clothing and no shoes, and with an empty bladder. The same scale should be used throughout the study, and calibrated on a regular basis as recommended by the manufacturer.

The use of balance scales is recommended; if digital scales are used, testing with standard weights is of particular importance. The floor surface on which the scale rests must be hard and should not be carpeted or covered with other soft material. The scale should be balanced with both weights at zero and the balance bar aligned. The patient should stand in the center of the platform as standing off-center may affect measurement. The weights are moved until the beam balances (the arrows are aligned). The weight is read and recorded in the e-CRF and Source Data. Self-reported weights are not acceptable; patients must not read the scales themselves.

Body weight will be measured at screening, on Day -1, Day14, Day 28, Day 42 and at the end of the study visit.

### 4.1.2.4 Patient diary

Information recorded into diary will document the compliance of IMP (lixisenatide/placebo) treatment as well as the safety and tolerability and these recordings will be carefully reviewed also at each on-site visit.

All patients will receive 1 diary, at visit V2 and they will bring it back to the center at each following visit during the treatment. Patients/parents will be instructed how to fill in it every day.

The diary includes sections for recording:

- Time and dose of IMP injections (during the treatment period),

- Any change in metformin dose or missing dose(s) and time if any,

- Any change in basal insulin daily dose and time or missing dose(s)if any,

- Any change or new concomitant medication,

- Adverse events, including signs and symptoms suggesting occurrence of hypoglycemia (possibly documented with measurement of "self-monitored plasma glucose" or plasma glucose monitored by others) and local injection site reactions, if any.

All patients will receive 1 diary, at visit V2 and they will bring it back to the center at each following visit during the treatment. Patients/parents will be instructed how to fill in it every day.

**4.1.3 Assessment schedule**

The assessment timing can be found in the period flow chart (Section 1.3).

**Table 2** - **Number of samples**

|  | Plasma glucose | HbA1c |
|---|---|---|
| By patient | (7x2)+(3x2) a | 2 |
| Total by patient | 20 | 2 |
| Total for study (n patients) | 20 * 24=480 | 2 * 24=48 |
| a 7 timepoints at Day-1 (V2) and Day 42 (V5) - 3 timepoints at days 14 and 28 (V3 and V4), | | |

**4.2 SAFETY**

Assessment schedule should be adapted to the compound specificities and the objectives of the study. Suggested list below:

**4.2.1 Baseline demographic characteristics:**

Baseline demographic characteristics will consist of:

1. Age (years)

2. Height (cm)

3. Body weight / Body mass index

4. Race / Ethnicity

5. Gender

6. Diabetes history including:

- Date of the diagnosis of diabetes;

- Start date, daily dose and regimen of administration of the background treatment at screening: metformin, basal insulin if any

7. Tanner staging (*Appendix B*)

**4.2.2 Safety assessment at baseline and during the study**

The tolerability investigations at baseline and during the study will consist of:

1. Physical examination (includes at a minimum: heart and respiratory auscultation; peripheral arterial pulse; pupil, knee, Achilles, and plantar reflexes; peripheral lymph nodes and abdomen examination), Body temperature (°C), Vital signs (heart rate, systolic and diastolic blood pressure measured after 10 minutes in supine resting position).

2. Body weight (kg).

3. Laboratory tests (in fasting conditions for blood samples):

• Hematology: red blood cell count, hematocrit, hemoglobin, white blood cell count with differential count (neutrophils, eosinophils, basophils, monocytes, and lymphocytes), platelets.

• Biochemistry:

- Plasma/serum electrolytes: sodium, potassium, chloride, calcium;

- Liver function: AST, ALT, alkaline phosphatase, gamma-glutamyl transferase, total and conjugated bilirubin;

- Pancreatic enzymes: amylase, lipase

- Renal function: urea, creatinine;

- Metabolism: glucose, albumin, total proteins, total cholesterol, triglycerides;

- Potential muscle toxicity: creatine phosphokinase.

- Calcitonin (thyroid c-cell tumor marker) at screening only

4. Serology tests: hepatitis B antigen, hepatitis C antibodies

5. Urinalysis: proteins, glucose, erythrocytes, leucocytes, ketone bodies, and pH. (To be adapted according to investigator site dipsticks)

- Qualitative: A dipstick is to be performed on a freshly voided specimen for qualitative detection using a reagent strip.

- Quantitative: A quantitative measurement for glucose, protein, erythrocytes, and leucocytes count will be required in the event that the urine sample test is positive for any of the above parameters by urine dipstick (eg, to confirm any positive dipstick parameter by a quantitative measurement).

6. Urine drug screen: amphetamines/methamphetamines, barbiturates, benzodiazepines, cannabinoids, cocaine, and opiates.

7. Alcohol breath test.

8. If female, beta-HCG plasma test.

9. Anti-lixisenatide antibodies

10. Adverse events, spontaneously reported by the patient or observed by the Investigator, will be monitored;

11. Standard 12-lead ECGs (safety ECGs) are recorded after at least 10 minutes in supine position using an (type of recorder and company to be added) electrocardiographic device. The electrodes will be positioned at the same place for each ECG recording throughout the study (attachment sites of the leads will be marked with an indelible pen). In case of triplicate (ie, baseline in TDU), 3 ECGs will be recorded within 5 minutes with at least 1 minute between 2 replicates.
Each ECG consists of a 10-second recording of the 12 leads simultaneously, leading to:

- A single 12-lead ECG (25 mm/s, 10mm/mV) printout with heart rate, PR, QRS, QT, QTc automatic correction evaluation (by the ECG device), including date, time, initials, and number of the patient, signature of the research physician, and at least 3 complexes for each lead. The Investigator's medical opinion and automatic values will be recorded in the e-CRF. This printout will be retained at the site.

- A digital storage that enables eventual further reading by an ECG central laboratory: each digital file will be identified by theoretical time (day and time DxxTxxHxx), real date and real time (recorder time), Sponsor study code, patient number (ie, 3 digits), initials (ie, 3 characters), and site and country numbers, if relevant. The digital recording, data storage, and transmission (whenever requested) need to comply with all applicable regulatory requirements (ie, FDA 21 CFR, part 11).

12. Self-monitored plasma glucose measurement

All the patients will be supplied with a plasma glucose meter, the corresponding supplies (lancets, test strips, etc.) and with diaries at visit V2 (week -1) in order to perform self-measurement of plasma glucose (or by others) and its recording. The glucose meters should be calibrated according to instructions given in the package leaflet and the study site should also check the glucose meters regularly using the provided control solutions for data validity. At visit V2 (week -1) patients and their "referent parent(s)" will be trained to accurately measure plasma glucose values with the glucose meter. The patients will be instructed to bring their glucose meters with them to each on-site visit.

It is the investigator's responsibility to explain the need to measure glucose at the times indicated below. Training will be repeated as often as necessary at the study visits and the study site staff reviews the patient's diary at each visit. Plasma glucose values will be measured by the patient/parent using the sponsor-provided blood glucose meter and recorded in the patient diary.

The patient will be instructed to perform SMPG measurements:

- Fasting value at least 3 times a week or more for patients treated with basal insulin, as medically indicated

- And for all patients treated with or without basal insulin, whenever a measurement is considered helpful, e.g. whenever the patients feel hypoglycemic symptoms, plasma glucose should be measured by the patient (or others, if applicable), if possible. Patients should be instructed to measure plasma glucose levels prior to the administration of glucose or carbohydrate intake whenever hypoglycemia is suspected (see XX of the protocol) unless safety considerations necessitate immediate glucose/carbohydrate countermeasure prior to confirmation. The values will be entered in the patient's individual diary and transcribed into the e-CRF.

### 4.3 ANTI-LIXISENATIDE ANTIBODIES

#### 4.3.1 Sampling times

Plasma samples from all patients will be collected to determine anti-lixisenatide antibodies on Day -1 /Day 14 / Day 28 /Day 42 before the study drug administration . Procedures for collection, storage, and shipment will be provided in a separate manual.

#### 4.3.2 Number of samples

**Table 3 - Number of plasma samples for anti-lixisenatide antibodies**

|  | Anti-lixisenatide antibodies |
|---|---|
| Total by patient | 4 |
| Total for patients (n=24) | 96 |

#### 4.3.3 Sample handling procedure for anti-lixisenatide antibodies

**Table 4 - Bioanalytical method**

| Analyte | Anti- Lixisenatide antibodies |
|---|---|
| Matrix | Plasma |
| Analytical Technique | BIAcore |
| Lower Limit of Quantification | cut-off |
| Assay Range | not relevant |
| Assay Volume | 100 $\mu$L |
| Site of Bioanalysis | Dept. of Disposition, Safety and Animal Research (DSAR), sanofi aventis, Frankfurt |
| Method Reference | RPSMPK-DOH0754-BM1-EN-E01 |

## 4.4 PHARMACOKINETICS

### 4.4.1 Sampling times

The sampling times for blood collection can be found in Table 5 and in the period flow chart (Section 1.3).

**Table 5 - Blood sampling for lixisenatide plasma concentrations**

| Time (hour/min) | 0 H | 0H30 | 1 H | 1H30 | 2 H | 2H30 | 3H30 | 4H |
|---|---|---|---|---|---|---|---|---|
| T (h) | T0 [a] | T0.5 [b] | T1 | T1.5 | T2 | T2.5 | T3.5 | T4.5 |
| Visit/Day: | | | | | | | | |
| V3/D14 | P00 | P01 | | P02 | | P03 | | |
| V4/D28 | P00 | P01 | | P02 | | P03 | | |
| V5/D42 | P00 | P01 | P02 | P03 | P04 | P05 | P06 | P07 |

a Prior to IMP administration
b Just prior to the standardized breakfast ingestion

### 4.4.2 Number of pharmacokinetic samples

**Table 6 - Number of plasma samples**

| | lixisenatide |
|---|---|
| By patient Total for study (n patients) | (4 x2)+8 =16 16 * 24=384 |

a 4 timepoints at Day14 (V3) and Day 28 (V4) - 8 timepoints on Day 42 (V5)

### 4.4.3 Sample handling procedure

Special procedures for collection, storage, and shipment should be provided in the laboratory manual.

**Table 7 - Summary of handling procedures**

| | |
|---|---|
| **Blood sample volume** | 2 mL |
| **Anticoagulant** | K3 EDTA |
| **Handling procedures** | "See Appendix B of the protocol |
| **Plasma aliquot split** | 2 tubes with one containing at least 0.5mL |
| **Plasma storage conditions** | -20°C |
| **Plasma shipment conditions** | On dry ice |

### 4.4.4 Bioanalytical methods

Lixisenatide plasma concentrations were determined using a validated double antibody sandwich enzyme linked immunosorbent assay method with an LLOQ (lower limit of quantification) of 5.5 pg/mL and an assay volume of 120 $\mu$L.

**Table 8 - Summary of bioanalytical method**

| | |
|---|---|
| **Analyte** | lixisenatide |
| **Matrix** | Plasma |
| **Analytical technique** | Double-antibody sandwich ELISA |
| **Lower limit of quantification** | 5.5 pg/mL |
| **Assay volume** | 120 $\mu$L |
| **Site of bioanalysis** | Covance laboratories Inc, Chantilly, France |
| **Method reference** | VA 20151-1130 / DOH1317 |

### 4.4.5 Pharmacokinetic parameters

The following pharmacokinetic parameters will be calculated, using noncompartmental methods from plasma concentrations obtained after repeated dose administration. The parameters will include, but may not be limited to the following.

**Table 9 - List of pharmacokinetic parameters and definitions**

| Parameters | Drug/Analyte | Matrix | Definition/Calculation |
|---|---|---|---|
| Cmax | lixisenatide | Plasma | Maximum plasma concentration observed during the respective treatment period, |
| tmax | lixisenatide | Plasma | Time to reach Cmax |
| $AUC_{0-4.50}$ | lixisenatide | Plasma | Area under the plasma concentration versus time curve calculated using the trapezoidal method from time zero (lixisenatide scale) to time 4.30 hours post dose |

### 4.5 SAMPLED BLOOD VOLUME

Sample blood volume should be presented in a table.

**Table 10 - Sampled blood volume per patient**

| Type | Volume per sample | Sample number | Total |
|---|---|---|---|
| Serology tests | 2.5 mL | 1 | 2.5 mL |
| Auto anti-bodies | 3.5 mL | 1 | 3.5 mL |
| Calcitonin | 2.0 mL | 1 | 2.0 mL |
| $\beta$-HCG (if applicable) | 1.1 mL | 1 | 1.1 mL |
| Hematology | 2.0 mL | 2 | 4.0 mL |
| Biochemistry | 2.5 mL | 2 | 5.0 mL |
| Amylase, lipase only | 2.5 mL | 4 | 10.0 mL |
| HbA1c | 2.0 mL | 2 | 4.0 mL |
| Plasma glucose | 1.2 mL | 20 | 24 mL |
| Pharmacokinetics Lixisenatide | 2 mL | 16 | 32 mL |
| Anti-lixisenatide antibodies | 1 mL | 4 | 4 mL |
| **Total if male** | | | 91 mL |
| **Total if female** | | | 92.1 mL |

The approximate total sampled blood volume in children is 91 and 92.1 mL for male and female patients, respectively (approximate due to discarded blood when catheter is set up at each period). The amount of blood volume per visit will not exceed 32 mL (the highest at Visit 5).

Additional samples may be needed if any laboratory result is outside of the normal range or for safety purposes.

### 4.6 FUTURE USE OF SAMPLES

Not applicable.

### 5 BIBLIOGRAPHIC REFERENCES

1. Druet C, Tubiana-Rufi N, Chevenne D, Rigal O, Polak M, Levy-Marchal C. Characterization of insulin secretion and resistance in type 2 diabetes of adolescents. J Clin Endocrinol Metab 2006: 91: 401-4.

2. Miller J, Silverstein JH, Rosenbloom AL. Type 2 diabetes in the child and adolescent. In: Lifshitz F (ed) Pediartric Endocrinology: fifth edition, volume 1. New York, Marcel Dekker 2007: pp 169-88.

3. Olansky L.: Do incretin-based therapies cause acute pancreatitis? J Diabetes Technol 2010; 4:2228-9

4. Schwartz GJ, Muñoz A, Schneider MF, Mak RH, Kaskel F, Warady BA, et al. New Equations to Estimate GFR in Children with CKD. J Am Soc Nephrol. 2009 Mar;20(3):629-37

**Appendix A Blood pressure levels by gender, age and height**

## Blood Pressure Levels for Boys by Age and Height Percentile

| Age (Year) | BP Percentile ↓ | Systolic BP (mmHg) | | | | | | | Diastolic BP (mmHg) | | | | | | |
|---|---|---|---|---|---|---|---|---|---|---|---|---|---|---|---|
| | | ← Percentile of Height → | | | | | | | ← Percentile of Height → | | | | | | |
| | | 5th | 10th | 25th | 50th | 75th | 90th | 95th | 5th | 10th | 25th | 50th | 75th | 90th | 95th |
| 10 | 50th | 97 | 98 | 100 | 102 | 103 | 105 | 106 | 58 | 59 | 60 | 61 | 61 | 62 | 63 |
| | 90th | 111 | 112 | 114 | 115 | 117 | 119 | 119 | 73 | 73 | 74 | 75 | 76 | 77 | 78 |
| | 95th | 115 | 116 | 117 | 119 | 121 | 122 | 123 | 77 | 78 | 79 | 80 | 81 | 81 | 82 |
| | 99th | 122 | 123 | 125 | 127 | 128 | 130 | 130 | 85 | 88 | 86 | 88 | 88 | 89 | 90 |
| 11 | 50th | 99 | 100 | 102 | 104 | 105 | 107 | 107 | 59 | 59 | 60 | 61 | 62 | 63 | 63 |
| | 90th | 113 | 114 | 115 | 117 | 118 | 120 | 121 | 74 | 74 | 75 | 76 | 77 | 78 | 78 |
| | 95th | 117 | 118 | 119 | 121 | 123 | 124 | 125 | 78 | 78 | 79 | 80 | 81 | 82 | 82 |
| | 99th | 124 | 125 | 127 | 129 | 130 | 132 | 132 | 86 | 86 | 87 | 88 | 89 | 90 | 90 |
| 12 | 50th | 101 | 102 | 104 | 106 | 108 | 109 | 110 | 59 | 60 | 61 | 62 | 83 | 63 | 64 |
| | 90th | 115 | 116 | 118 | 120 | 121 | 123 | 123 | 74 | 75 | 75 | 76 | 77 | 78 | 79 |
| | 95th | 119 | 120 | 122 | 123 | 125 | 127 | 127 | 78 | 79 | 80 | 81 | 82 | 82 | 83 |
| | 99th | 129 | 127 | 129 | 131 | 133 | 134 | 135 | 86 | 87 | 88 | 89 | 90 | 90 | 91 |
| 13 | 50th | 104 | 105 | 106 | 108 | 110 | 111 | 112 | 60 | 60 | 61 | 62 | 63 | 64 | 64 |
| | 90th | 117 | 118 | 120 | 122 | 124 | 125 | 126 | 75 | 75 | 78 | 77 | 78 | 79 | 79 |
| | 95th | 121 | 122 | 124 | 126 | 128 | 129 | 130 | 79 | 79 | 80 | 81 | 82 | 83 | 83 |
| | 99th | 128 | 130 | 131 | 133 | 135 | 136 | 137 | 87 | 87 | 88 | 89 | 90 | 91 | 91 |
| 14 | 50th | 106 | 107 | 109 | 111 | 113 | 114 | 115 | 80 | 61 | 62 | 63 | 64 | 65 | 65 |
| | 90th | 120 | 121 | 123 | 125 | 126 | 128 | 128 | 75 | 76 | 77 | 78 | 79 | 79 | 80 |
| | 95th | 124 | 125 | 127 | 128 | 130 | 132 | 132 | 80 | 80 | 81 | 82 | 83 | 84 | 84 |
| | 90th | 131 | 132 | 134 | 136 | 138 | 139 | 140 | 87 | 88 | 89 | 90 | 91 | 92 | 92 |
| 15 | 50th | 109 | 110 | 112 | 113 | 115 | 117 | 117 | 61 | 62 | 63 | 64 | 65 | 66 | 60 |
| | 90th | 122 | 124 | 125 | 127 | 129 | 130 | 131 | 76 | 77 | 78 | 79 | 80 | 80 | 81 |
| | 95th | 126 | 127 | 129 | 131 | 133 | 134 | 135 | 81 | 81 | 82 | 83 | 84 | 85 | 85 |
| | 99th | 134 | 135 | 136 | 138 | 140 | 142 | 142 | 88 | 89 | 90 | 91 | 92 | 93 | 93 |

| Age (Year) | BP Percentile ↓ | Systolic BP (mmHg) | | | | | | | Diastolic BP (mmHg) | | | | | | |
|---|---|---|---|---|---|---|---|---|---|---|---|---|---|---|---|
| | | ← Percentile of Height → | | | | | | | ← Percentile of Height → | | | | | | |
| | | 5th | 10th | 25th | 50th | 75th | 90th | 95th | 5th | 10th | 25th | 50th | 75th | 90th | 95th |
| 16 | 50th | 111 | 112 | 114 | 116 | 118 | 119 | 120 | 63 | 63 | 64 | 65 | 66 | 67 | 67 |
| | 90th | 125 | 128 | 128 | 130 | 131 | 133 | 134 | 78 | 78 | 79 | 80 | 81 | 82 | 82 |
| | 95th | 129 | 130 | 132 | 134 | 135 | 137 | 137 | 82 | 83 | 83 | 84 | 85 | 86 | 87 |
| | 99th | 136 | 137 | 139 | 141 | 143 | 144 | 145 | 90 | 90 | 91 | 92 | 93 | 94 | 94 |
| 17 | 50th | 114 | 115 | 116 | 118 | 120 | 121 | 122 | 65 | 66 | 66 | 67 | 68 | 69 | 70 |
| | 90th | 127 | 128 | 130 | 132 | 134 | 135 | 136 | 80 | 80 | 81 | 82 | 83 | 84 | 84 |
| | 95th | 131 | 132 | 134 | 136 | 138 | 139 | 140 | 84 | 85 | 86 | 87 | 87 | 88 | 89 |
| | 99th | 139 | 140 | 141 | 143 | 145 | 146 | 147 | 92 | 93 | 93 | 94 | 95 | 96 | 97 |

BP, blood pressure * The 90th percentile is 1.28 SD, 95th percentile is 1.645 SD, and the 99th percentile is 2.326 SD over the mean.

**Blood Pressure Levels for Girls by Age and Height Percentile**

| Age (Year) | BP Percentile | Systolic BP (mmHg) ← Percentile of Height → | | | | | | | Diastolic BP (mmHg) ← Percentile of Height → | | | | | | |
|---|---|---|---|---|---|---|---|---|---|---|---|---|---|---|---|
| | | 5th | 10th | 25th | 50th | 75th | 90th | 95th | 5th | 10th | 25th | 50th | 75th | 90th | 95th |
| 10 | 50th | 98 | 99 | 100 | 102 | 103 | 104 | 105 | 59 | 59 | 59 | 60 | 61 | 62 | 62 |
| | 90th | 112 | 112 | 114 | 115 | 116 | 118 | 118 | 73 | 73 | 73 | 74 | 75 | 76 | 76 |
| | 95th | 116 | 116 | 117 | 119 | 120 | 121 | 122 | 77 | 77 | 77 | 78 | 79 | 80 | 80 |
| | 99th | 123 | 123 | 125 | 126 | 127 | 129 | 129 | 84 | 84 | 85 | 86 | 86 | 87 | 88 |
| 11 | 50th | 100 | 101 | 102 | 103 | 105 | 106 | 107 | 60 | 60 | 60 | 61 | 62 | 63 | 63 |
| | 90th | 114 | 114 | 116 | 117 | 118 | 119 | 120 | 74 | 74 | 74 | 75 | 76 | 77 | 77 |
| | 95th | 11B | 118 | 119 | 121 | 122 | 123 | 124 | 78 | 78 | 78 | 79 | 80 | 81 | 81 |
| | 99th | 125 | 125 | 126 | 128 | 129 | 130 | 131 | 85 | 85 | 86 | 87 | 87 | 88 | 89 |
| 12 | 50th | 102 | 103 | 104 | 105 | 107 | 108 | 109 | 61 | 61 | 61 | 62 | 63 | 64 | 64 |
| | 90th | 116 | 116 | 117 | 119 | 120 | 121 | 122 | 75 | 75 | 75 | 76 | 77 | 78 | 78 |
| | 95th | 119 | 120 | 121 | 123 | 124 | 125 | 126 | 79 | 79 | 79 | 80 | 81 | 82 | 82 |
| | 99th | 127 | 127 | 128 | 130 | 131 | 132 | 133 | 86 | 86 | 87 | 88 | 88 | 89 | 90 |
| 13 | 50th | 104 | 105 | 106 | 107 | 109 | 110 | 110 | 62 | 62 | 62 | 63 | 64 | 65 | 65 |
| | 90th | 117 | 118 | 119 | 121 | 122 | 123 | 124 | 76 | 76 | 76 | 77 | 78 | 79 | 79 |
| | 95th | 121 | 122 | 123 | 124 | 126 | 127 | 128 | 80 | 80 | 80 | 81 | 82 | 83 | 83 |
| | 99th | 128 | 129 | 130 | 132 | 133 | 134 | 135 | 87 | 87 | 88 | 89 | 89 | 90 | 91 |
| 14 | 50th | 106 | 106 | 107 | 109 | 110 | 111 | 112 | 63 | 63 | 63 | 64 | 65 | 66 | 66 |
| | 90th | 119 | 120 | 121 | 122 | 124 | 125 | 125 | 77 | 77 | 77 | 78 | 79 | 80 | 80 |
| | 95th | 123 | 123 | 125 | 126 | 127 | 129 | 129 | 81 | 81 | 81 | 82 | 83 | 84 | 84 |
| | 99th | 130 | 131 | 132 | 133 | 135 | 136 | 136 | 88 | 88 | 89 | 90 | 90 | 91 | 92 |
| 15 | 50th | 107 | 108 | 109 | 110 | 111 | 113 | 113 | 64 | 64 | 64 | 65 | 66 | 67 | 67 |
| | 90th | 120 | 121 | 122 | 123 | 125 | 126 | 127 | 78 | 78 | 78 | 79 | 80 | 81 | 81 |
| | 95th | 124 | 125 | 126 | 127 | 129 | 130 | 131 | 82 | 82 | 82 | 83 | 84 | 85 | 85 |
| | 99th | 131 | 132 | 133 | 134 | 136 | 137 | 138 | 89 | 89 | 90 | 91 | 91 | 92 | 93 |
| 16 | 50th | 108 | 108 | 110 | 111 | 112 | 114 | 114 | 64 | 64 | 65 | 66 | 66 | 67 | 68 |
| | 90th | 121 | 122 | 123 | 124 | 126 | 127 | 128 | 78 | 78 | 79 | 80 | 81 | 81 | 82 |
| | 95th | 125 | 126 | 127 | 128 | 130 | 131 | 132 | 82 | 82 | 83 | 84 | 85 | 85 | 86 |
| | 99th | 132 | 133 | 134 | 135 | 137 | 138 | 139 | 90 | 90 | 90 | 91 | 92 | 93 | 93 |

| Age (Year) | BP Percentile ↓ | Systolic BP (mmHg) | | | | | | | Diastolic BP (mmHg) | | | | | | |
|---|---|---|---|---|---|---|---|---|---|---|---|---|---|---|---|
| | | ← Percentile of Height → | | | | | | | ← Percentile of Height → | | | | | | |
| | | 5th | 10th | 25th | 50th | 75th | 90th | 95th | 5th | 10th | 25th | 50th | 75th | 90th | 95th |
| 17 | 50th | 108 | 109 | 110 | 111 | 113 | 114 | 115 | 64 | 65 | 65 | 66 | 67 | 67 | 68 |
| | 90th | 122 | 122 | 123 | 125 | 126 | 127 | 128 | 78 | 79 | 79 | 80 | 81 | 81 | 82 |
| | 95th | 125 | 126 | 127 | 129 | 130 | 131 | 132 | 82 | 83 | 83 | 84 | 85 | 85 | 86 |
| | 99th | 133 | 133 | 134 | 136 | 137 | 138 | 139 | 90 | 90 | 91 | 91 | 92 | 93 | 93 |

BP, blood pressure
* The 90th percentile is 1.28 SD, 95th percentile is 1.645 SD, and the 99th percentile is 2.326 SD over the mean.

**Appendix B Tanner stage**

**Tanner puberty stage classification**

**Classification of sex maturity stages in girls**

| Stage | Pubic hair | Stage | Breasts |
|---|---|---|---|
| P1 | Preadolescent | B1 | Preadolescent |
| P2 | Sparse, lightly pigmented, straight, medial border of labia | B2 | Breast and papilla elevated as small mound; areolar diameter increased |
| P3 | Darker, beginning to curl, increased amount | B3 | Breast and areola enlarged, no contour separation |
| P4 | Coarse, curly, abundant but amount less than in adult | B4 | Areola and papilla form secondary mound |
| P5 | Adult feminine triangle, spread to medial surface of thighs | B5 | Mature; nipple projects, areola part of general breast contour |

**Classification of sex maturity stages in boys**

| Stage | Pubic hair | Stage | Testes |
|---|---|---|---|
| P1 | None | T1 | Preadolescent |
| P2 | Scanty, long, slightly pigmented | T2 | Enlarged scrotum, pink texture altered |
| P3 | Darker, starts to curl, small amount | T3 | Larger |
| P4 | Resembles adult type, but less in quantity; coarse, curly | T4 | Larger, scrotum dark |
| P5 | Adult distribution, spread to medial surface of thighs | T5 | Adult size |

**Claims**

1. Lixisenatide or/and a pharmaceutically acceptable salt thereof, for use in a method of treatment of pediatric patients suffering from type 2 diabetes mellitus.

2. Lixisenatide or/and a pharmaceutically acceptable salt thereof, for use according to claim 1, wherein lixisenatide or/and the pharmaceutically acceptable salt thereof is administered as an add-on therapy to metformin or/and a pharmaceutically acceptable salt thereof.

3. Lixisenatide or/and a pharmaceutically acceptable salt thereof, for use according to claim 2, wherein metformin or/and the pharmaceutically acceptable salt thereof is prepared for oral administration.

4. Lixisenatide or/and a pharmaceutically acceptable salt thereof, for use according to any one of the preceding claims, wherein the patient in need of the pediatric treatment has an age of at least 10 years.

5. Lixisenatide or/and a pharmaceutically acceptable salt thereof, for use according to any one of the preceding claims, wherein the patient in need of the pediatric treatment has an age of less than 18 years.

6. Lixisenatide or/and a pharmaceutically acceptable salt thereof, for use according to any one of the preceding claims, wherein the type 2 diabetes mellitus has been diagnosed at least three months.

7. Lixisenatide or/and a pharmaceutically acceptable salt thereof, for use according to any one of the preceding claims, wherein the type 2 diabetes mellitus is not adequately controlled by metformin monotherapy, by basal insulin monotherapy or by a combination of metformin and a basal insulin.

8. Lixisenatide or/and a pharmaceutically acceptable salt thereof, for use according to any one of the preceding claims, wherein the patient in need of the pediatric treatment is obese.

9. Lixisenatide or/and a pharmaceutically acceptable salt thereof, for use according to any one of the preceding claims, wherein the patient in need of the pediatric treatment has a body mass index of at least 30 kg/m$^2$ or at least 31 kg/m$^2$.

10. Lixisenatide or/and a pharmaceutically acceptable salt thereof, for use according to any one of the preceding claims, wherein lixisenatide is administered about 30 min before breakfast.

11. Lixisenatide or/and a pharmaceutically acceptable salt thereof, for use according to any one of the preceding claims, wherein at the onset of treatment with lixisenatide or/and the pharmaceutically acceptable salt thereof, the patient has a fasting plasma glucose concentration of at least 8 mmol/L or at least 8.5 mmol/L.

12. Lixisenatide or/and a pharmaceutically acceptable salt thereof, for use according to any one of the preceding claims, wherein at the onset of treatment with lixisenatide or/and the pharmaceutically acceptable salt thereof, the patient has a 2 hours postprandial plasma glucose concentration of at least 11.1 mmol/L or at least 12 mmol/L.

13. Lixisenatide or/and a pharmaceutically acceptable salt thereof, for use according to any one of the preceding claims, wherein at the onset of treatment with lixisenatide or/and the pharmaceutically acceptable salt thereof, the patient has a glucose excursion of at least 3 mmol/L, wherein the glucose excursion is the difference of the 2 hours postprandial plasma glucose concentration and plasma glucose concentration 30 minutes prior to a meal test.

14. Lixisenatide or/and a pharmaceutically acceptable salt thereof, for use according to any one of the preceding claims, wherein at the onset of treatment with lixisenatide or/and the pharmaceutically acceptable salt thereof, the patient has a HbA1c value of at least about 7 %, at least about 7.5 %, at least about 8 %, at least about 8.5 %, at least about 8.65 %, or at least about 9%.

15. Lixisenatide or/and a pharmaceutically acceptable salt thereof, for use according to any one of the preceding claims, wherein lixisenatide or/and the pharmaceutically acceptable salt thereof is administered by one injection per day.

**Patentansprüche**

1. Lixisenatid oder/und ein pharmazeutisch akzeptables Salz davon zur Verwendung bei einem Verfahren zur Behandlung von pädiatrischen Patienten, die an Diabetes mellitus Typ 2 leiden.

2. Lixisenatid oder/und ein pharmazeutisch akzeptables Salz davon zur Verwendung nach Anspruch 1, wobei Lixisenatid oder/und das pharmazeutisch akzeptable Salz davon als Zusatztherapie zu Metformin oder/und einem pharmazeutisch akzeptablen Salz davon verabreicht wird.

3. Lixisenatid oder/und ein pharmazeutisch akzeptables Salz davon zur Verwendung nach Anspruch 2, wobei Metformin oder/und das pharmazeutisch akzeptable Salz davon zur oralen Verabreichung hergestellt wird.

4. Lixisenatid oder/und ein pharmazeutisch akzeptables Salz davon zur Verwendung nach einem der vorhergehenden Ansprüche, wobei der Patient, welcher der pädiatrischen Behandlung bedarf, ein Alter von mindestens 10 Jahren hat.

5. Lixisenatid oder/und ein pharmazeutisch akzeptables Salz davon zur Verwendung nach einem der vorhergehenden Ansprüche, wobei der Patient, welcher der pädiatrischen Behandlung bedarf, ein Alter von weniger als 18 Jahren hat.

6. Lixisenatid oder/und ein pharmazeutisch akzeptables Salz davon zur Verwendung nach einem der vorhergehenden Ansprüche, wobei der Diabetes mellitus Typ 2 seit mindestens drei Monaten diagnostiziert ist.

7. Lixisenatid oder/und ein pharmazeutisch akzeptables Salz davon zur Verwendung nach einem der vorhergehenden Ansprüche, wobei der Diabetes mellitus Typ 2 durch eine Metformin-Monotherapie, durch eine Basalinsulin-Monotherapie oder durch eine Kombination von Metformin und einem Basalinsulin nicht ausreichend kontrolliert wird.

8. Lixisenatid oder/und ein pharmazeutisch akzeptables Salz davon zur Verwendung nach einem der vorhergehenden Ansprüche, wobei der Patient, welcher der pädiatrischen Behandlung bedarf, fettleibig ist.

9. Lixisenatid oder/und ein pharmazeutisch akzeptables Salz davon zur Verwendung nach einem der vorhergehenden Ansprüche, wobei der Patient, welcher der pädiatrischen Behandlung bedarf, einen Körpermassenindex von mindestens 30 kg/m$^2$ oder mindestens 31 kg/m$^2$ aufweist.

10. Lixisenatid oder/und ein pharmazeutisch akzeptables Salz davon zur Verwendung nach einem der vorhergehenden Ansprüche, wobei Lixisenatid etwa 30 Minuten vor dem Frühstück verabreicht wird.

11. Lixisenatid oder/und ein pharmazeutisch akzeptables Salz davon zur Verwendung nach einem der vorhergehenden Ansprüche, wobei der Patient zu Beginn der Behandlung mit Lixisenatid oder/und dem pharmazeutisch akzeptablen Salz davon eine Nüchtern-Plasmaglukosekonzentration von mindestens 8 mmol/l oder mindestens 8,5 mmol/l aufweist.

12. Lixisenatid oder/und ein pharmazeutisch akzeptables Salz davon zur Verwendung nach einem der vorhergehenden Ansprüche, wobei der Patient zu Beginn der Behandlung mit Lixisenatid oder/und dem pharmazeutisch akzeptablen Salz davon eine Plasmaglukosekonzentration 2 Stunden postprandial von mindestens 11,1 mmol/l oder mindestens 12 mmol/l aufweist.

13. Lixisenatid oder/und ein pharmazeutisch akzeptables Salz davon zur Verwendung nach einem der vorhergehenden Ansprüche, wobei der Patient zu Beginn der Behandlung mit Lixisenatid oder/und dem pharmazeutisch akzeptablen Salz davon eine Glukoseexkursion von mindestens 3 mmol/l aufweist, wobei die Glukoseexkursion die Differenz zwischen der Plasmaglukosekonzentration 2 Stunden postprandial und der Plasmaglukosekonzentration 30 Minuten vor einem Mahlzeitentest ist.

14. Lixisenatid oder/und ein pharmazeutisch akzeptables Salz davon zur Verwendung nach einem der vorhergehenden Ansprüche, wobei der Patient zu Beginn der Behandlung mit Lixisenatid oder/und dem pharmazeutisch akzeptablen Salz davon einen HbA1c-Wert von mindestens etwa 7 %, mindestens etwa 7,5 %, mindestens etwa 8 %, mindestens etwa 8,5 %, mindestens etwa 8,65 % oder mindestens etwa 9 % aufweist.

15. Lixisenatid oder/und ein pharmazeutisch akzeptables Salz davon zur Verwendung nach einem der vorhergehenden Ansprüche, wobei Lixisenatid oder/und das pharmazeutisch akzeptable Salz davon durch eine Injektion pro Tag verabreicht wird.

**Revendications**

1. Lixisénatide ou/et sel pharmaceutiquement acceptable de celui-ci, pour utilisation dans un procédé de traitement de patients pédiatriques souffrant de diabète sucré de type 2.

2. Lixisénatide ou/et sel pharmaceutiquement acceptable de celui-ci, pour utilisation selon la revendication 1, où le lixisénatide ou/et le sel pharmaceutiquement acceptable de celui-ci est administré en tant que traitement adjuvant pour la metformine ou/et un sel pharmaceutiquement acceptable de celle-ci.

3. Lixisénatide ou/et sel pharmaceutiquement acceptable de celui-ci, pour utilisation selon la revendication 2, où la metformine ou/et le sel pharmaceutiquement acceptable de celle-ci est préparé pour une administration orale.

4. Lixisénatide ou/et sel pharmaceutiquement acceptable de celui-ci, pour utilisation selon l'une quelconque des revendications précédentes, où le patient ayant besoin du traitement pédiatrique est âgé d'au moins 10 ans.

5. Lixisénatide ou/et sel pharmaceutiquement acceptable de celui-ci, pour utilisation selon l'une quelconque des revendications précédentes, où le patient ayant besoin du traitement pédiatrique est âgé de moins de 18 ans.

6. Lixisénatide ou/et sel pharmaceutiquement acceptable de celui-ci, pour utilisation selon l'une quelconque des revendications précédentes, où le diabète sucré de type 2 a été diagnostiqué depuis au moins trois mois.

**7.** Lixisénatide ou/et sel pharmaceutiquement acceptable de celui-ci, pour utilisation selon l'une quelconque des revendications précédentes, où le diabète sucré de type 2 n'est pas adéquatement contrôlé par la metformine en monothérapie, par l'insuline basale en monothérapie ou par une combinaison de metformine et d'une insuline basale.

**8.** Lixisénatide ou/et sel pharmaceutiquement acceptable de celui-ci, pour utilisation selon l'une quelconque des revendications précédentes, où le patient ayant besoin du traitement pédiatrique est obèse.

**9.** Lixisénatide ou/et sel pharmaceutiquement acceptable de celui-ci, pour utilisation selon l'une quelconque des revendications précédentes, où le patient ayant besoin du traitement pédiatrique a un indice de masse corporelle d'au moins 30 kg/m$^2$ ou d'au moins 31 kg/m$^2$.

**10.** Lixisénatide ou/et sel pharmaceutiquement acceptable de celui-ci, pour utilisation selon l'une quelconque des revendications précédentes, où le lixisénatide est administré environ 30 min avant le petit-déjeuner.

**11.** Lixisénatide ou/et sel pharmaceutiquement acceptable de celui-ci, pour utilisation selon l'une quelconque des revendications précédentes, où, au début du traitement avec le lixisénatide ou/et le sel pharmaceutiquement acceptable de celui-ci, le patient présente une concentration plasmatique de glucose à jeun d'au moins 8 mmol/l ou d'au moins 8,5 mmol/l.

**12.** Lixisénatide ou/et sel pharmaceutiquement acceptable de celui-ci, pour utilisation selon l'une quelconque des revendications précédentes, où, au début du traitement avec le lixisénatide ou/et le sel pharmaceutiquement acceptable de celui-ci, le patient présente une concentration plasmatique de glucose postprandiale à 2 heures d'au moins 11,1 mmol/l ou d'au moins 12 mmol/l.

**13.** Lixisénatide ou/et sel pharmaceutiquement acceptable de celui-ci, pour utilisation selon l'une quelconque des revendications précédentes, où, au début du traitement avec le lixisénatide ou/et le sel pharmaceutiquement acceptable de celui-ci, le patient présente un écart de glucose d'au moins 3 mmol/l, où l'écart de glucose est la différence de la concentration plasmatique de glucose postprandiale à 2 heures et la concentration plasmatique de glucose 30 minutes avant un repas test.

**14.** Lixisénatide ou/et sel pharmaceutiquement acceptable de celui-ci, pour utilisation selon l'une quelconque des revendications précédentes, où, au début du traitement avec le lixisénatide ou/et le sel pharmaceutiquement acceptable de celui-ci, le patient a une valeur HbAlc d'au moins environ 7 %, au moins environ 7,5 %, au moins environ 8 %, au moins environ 8,5 %, au moins environ 8,65 % ou au moins environ 9 %.

**15.** Lixisénatide ou/et sel pharmaceutiquement acceptable de celui-ci, pour utilisation selon l'une quelconque des revendications précédentes, où le lixisénatide ou/et le sel pharmaceutiquement acceptable de celui-ci est administré par une injection par jour.

## Figure 1 – Graphical Study Design of Example 1

| Screening | TP 1 Lixisenatide or Placebo | Washout Period 1 to 7 days | TP 2 Lixisenatide or Placebo | Washout Period 1 to 7 days | TP 3 Lixisenatide or Placebo | Washout Period 1 to 7 days | EOS D2 to D7 after TP3 |

D-30 to D-2*
1. Inclusion
2. Informed Consent

D1
1. Admission
2. Inclusion check
3. Randomization
4. PD/PK Blood sampling
5. Study drug injection
6. Liquid meal test
7. Discharge

D1
1. Admission
2. Inclusion check
3. PD/PK Blood sampling
4. Study drug injection
5. Liquid meal test
6. Discharge

D1
1. Admission
2. Inclusion check
3. PD/PK Blood sampling
4. Study drug injection
5. Liquid meal test
6. Discharge

1. Urinalysis Blood laboratory examination

## Figure 2 – 3 cartridges for injections

Figure 3 – Body mass index (BMI) for age percentiles by gender: Boys, 2 to 20 years

Body mass Index-for-age percentiles:
Boys, 2 to 20 years

Age (years)

**Figure 4 – Body mass index (BMI) for age percentiles by gender: Girls, 2 to 20 years**

Body mass index-for-age percentiles: Girls, 2 to 20 years

Figure 5 – Mean ± SEM plasma glucose per treatment group in adult patients - evaluable PD population

**Figure 6 – Mean ± SEM plasma glucose per treatment group in pediatric patients – evaluable PD population**

Figure 7 – Median plasma glucose (mmol/L) per treatment group in adult patients -
evaluable PD population

Raw data - Median

## Figure 8 – Median plasma glucose (mmol/L) per treatment group in pediatric patients - evaluable PD population

Raw data - Median

Figure 9 – Median glucagon (ng/L) per treatment group in adult patients - evaluable PD population

Raw data - Median

| Actual Treatment——————— Placebo — — — — —Lixisenatide 5 μg—— — — —Lixisenatide 10μg |
| 1 | 2 | 3 |

Figure 10 – Median glucagon (ng/L) per treatment group in pediatric patients - evaluable PD population

Raw data - Median

Figure 11 – Median plasma insulin (pmol/L) per treatment group in adult patients -
evaluable PD population

**Raw data - Median**

| Actual Treatment ————— Placebo — — — — —Lixisenatide 5 µg —— — — —Lixisenatide 10µg |

1                          2                          3

**Figure 12 – Median plasma insulin (pmol/L) per treatment group in pediatric patients - evaluable PD population**

Raw data - Median

**Figure 13 – Median C-peptide (nmol/L) per treatment group in adult patients - evaluable PD population**

Raw data - Median

Figure 14 – Median C-peptide (nmol/L) per treatment group in pediatric patients -
evaluable PD population

Raw data - Median

Figure 15 – Mean (+ SD) lixisenatide plasma concentrations by treatment (full PK population, linear scale)

Figure 16 – Mean (+SD) lixisenatide plasma concentrations by treatment (evaluable PK population, linear scale)

## Figure 17 – Graphical Study Design of Example 3

Screening period (up to 3 weeks) | Double-blind treatment Period (6 weeks) | Follow up (3 days)

LIXISENATIDE ARM

20 µg

10 µg

5 µg

2 weeks

2 weeks

2 weeks

R

PLACEBO ARM *

End of Study

Week: -3     -1   1     2     4     6     7

Day:     -1   1     14     28     42     45

Visit: 1     2   3     4     5     6     7

R: randomization   ↑: On-site visit

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0104156 A

- EP 15151488

### Non-patent literature cited in the description

- The Diabetes Control and Complications Trial Research Group. *N. Engl. J. Med.,* 1993, vol. 329, 977-986
- Type 2 Diabetes in Children and Adolescents. Clinical Practice Guidelines. Canadian Diabetes Association Clinical Practice Guidelines Expert Committee, 2008, S162-S167
- Diabetes Care. American Diabetes Association, 2000, vol. 23, 381-389
- **DOMBROWSKY ; BARRETT.** Type II Diabetes Mellitus in Children: Analysis of Prevalence Based on the Pediatric Heath Information System (PHIS) Database. *American College of Clinical Pharmacology Annual Meeting,* 22 September 2013
- *CHEMICAL ABSTRACTS,* 320367-13-3
- Definition, Diagnosis and Classification of Diabetes Mellitus and its Complications. *Diagnosis and Classification of Diabetes Mellitus,* 1999
- Global IDF/ISPAD Guideline for Diabetes. Childhood and Adolescence. International Diabetes Federation
- **CRAIG.** *Pediatric Diabetes,* 2014, vol. 15 (20), 4-17
- **CRAPO et al.** *Diabetes,* 1977, vol. 26 (12), 1178-1183
- Investigator's Brochure Lixisenatide. 01 April 2011
- National diabetes fact sheet United States, 2003: general information. Centers for Disease Control and Prevention, 06 June 2008
- Clinical Practice Guidelines for the Prevention and Management of Diabetes in Canada. Canadian Journal of Diabetes. Canadian Diabetes Association, 2008, S161-S167
- **PINHAS-HAMIEL O. ; ZEITLER P.** Clinical presentation and treatment of type 2 diabetes in children. *Pediatric Diabetes,* 2007, vol. 8 (9), 16-27
- Type 2 diabetes in children and adolescents. Diabetes Care. American Diabetes Association, 2000, vol. 23, 381-389

- **JONES KL ; ARSLANIAN S ; PETEROKOVA VA ; PARK J-S ; TOMLINSON MJ.** Effect of metformin in pediatric patients with type 2 diabetes: a randomized controlled trial. *Diabetes Care,* 2002, vol. 25, 89-94
- **TANNER JM ; DAVIES, PS.** Clinical longitudinal standards for height and height velocity for North American children. *J Pediatr,* 1985, vol. 107 (3), 317-329
- **TANNER JM ; WHITEHOUSE RH ; TAKAISHI M.** Standards from birth to maturity for height, weight, height velocity, and weight velocity: British children, 1965. II. *Arch Dis Child.,* 1966, vol. 41 (220), 613-635
- **WOLEVER TMS ; JENKINS D. JA ; OCANA A.M ; RAO VA ; COLLIER G.C.** Second-meal effect: low-glycemic -index foods eaten at dinner improve subsequent breakfast glycemic response. *Am J CLin Nutr,* 1988, vol. 48, 1041-7
- **NILSSON A ; OSTMAN E ; PRESTON T ; BJÖRCK.** Effects of Gi vs content of cereal fibre of the evening meal on glucose tolerance at a subsequent standardized breakfast. *Eur. J Clin Nutr.,* 2008, vol. 62, 712-720
- **DRUET C ; TUBIANA-RUFI N ; CHEVENNE D ; RIGAL O ; POLAK M ; LEVY-MARCHAL C.** Characterization of insulin secretion and resistance in type 2 diabetes of adolescents. *J Clin Endocrinol Metab,* 2006, vol. 91, 401-4
- Type 2 diabetes in the child and adolescent. **MILLER J ; SILVERSTEIN JH ; ROSENBLOOM AL.** Pediartric Endocrinology. Marcel Dekker, 2007, vol. 1, 169-88
- **OLANSKY L.** Do incretin-based therapies cause acute pancreatitis?. *J Diabetes Technol,* 2010, vol. 4, 2228-9
- **SCHWARTZ GJ ; MUÑOZ A ; SCHNEIDER MF ; MAK RH ; KASKEL F ; WARADY BA et al.** New Equations to Estimate GFR in Children with CKD. *J Am Soc Nephrol.,* March 2009, vol. 20 (3), 629-37

SEQUENCE LISTING

<110> Sanofi-Aventis Deutschland GmbH

<120> Treatment of pediatric type 2 diabetes mellitus patients

<130> 58590P EP

<140> EP 15151488.2
<141> 2015-01-16

<160> 2

<170> PatentIn version 3.5

<210> 1
<211> 44
<212> PRT
<213> Artificial

<220>
<223> desPro36-Exendin-4(1-39)-Lys6-NH2

<400> 1

```
His Gly Glu Gly Thr Phe Thr Ser Asp Leu Ser Lys Gln Met Glu Glu
1               5               10                  15

Glu Ala Val Arg Leu Phe Ile Glu Trp Leu Lys Asn Gly Gly Pro Ser
            20                  25                  30

Ser Gly Ala Pro Pro Ser Lys Lys Lys Lys Lys Lys
        35                  40
```

<210> 2
<211> 39
<212> PRT
<213> Heloderma suspectum

<220>
<223> Exendin-4-NH2

<400> 2

```
His Gly Glu Gly Thr Phe Thr Ser Asp Leu Ser Lys Gln Met Glu Glu
1               5               10                  15

Glu Ala Val Arg Leu Phe Ile Glu Trp Leu Lys Asn Gly Gly Pro Ser
            20                  25                  30

Ser Gly Ala Pro Pro Pro Ser
        35
```